# EUROPEAN PATENT SPECIFICATION

(11) **EP 2 165 716 B1**
(45) Date of publication and mention of the grant of the patent: **12.11.2014**
(21) Application number: 08765502.3
(22) Date of filing: 05.06.2008
(51) Int. Cl.: A61K 45/00, A61K 38/00, A61P 19/02, A61P 19/08, A61P 19/10, C07K 14/705, C12Q 1/02, G01N 33/15, G01N 33/50

(54) **NOVEL BONE MASS INCREASING AGENT**
NEUE MITTEL ZUR ERHÖHUNG DER KNOCHENMASSE
NOUVEL AGENT D'AUGMENTATION DE LA MASSE OSSEUSE

(30) Priority: 05.06.2007 JP 2007149799; 04.12.2007 JP 2007313822; 10.03.2008 JP 2008060145; 20.05.2008 JP 2008131572
(43) Date of publication of application: 24.03.2010
(73) Proprietor: Oriental Yeast Co., Ltd., Tokyo 174-8505 (JP)
(72) Inventor: YASUDA, Hisataka, Nagahama-shi Shiga 526-0804 (JP); FURUYA, Yuriko, Nagahama-shi Shiga 526-0804 (JP); TAGUCHI, Yusuke, Nagahama-shi Shiga 526-0804 (JP)
(74) Representative: Denison, Christopher Marcus
(86) International application number: PCT/JP2008/060731
(87) International publication number: WO 2008/150025

(56) References cited:
- WO-A1-01/08677
- WO-A2-01/83525
- JP-A- 2003 512 011
- JP-A- 2003 533 187
- KOSTENUIK ET AL: "Osteoprotegerin and RANKL regulate bone resorption, density, geometry and strength" CURRENT OPINION IN PHARMACOLOGY, ELSEVIER SCIENCE PUBLISHERS, NL LNKD- DOI:10.1016/J.COPH.2005.06.005, vol. 5, no. 6, 1 December 2005 (2005-12-01), pages 618-625, XP005134774 ISSN: 1471-4892
- MCINTYRE J A ET AL: "AMG-162. Treatment of osteoporosis bone cancer therapy treatment of rheumatoid arthritis human anti-RANKL monoclonal antibody" DRUGS OF THE FUTURE, vol. 30, no. 3, March 2005 (2005-03), pages 237-239, XP002603447 ISSN: 0377-8282
- TAKASAKI WATARU ET AL: "Structure-based design and characterization of exocyclic peptidomimetics that inhibit TNF-alpha binding to its receptor" NATURE BIOTECHNOLOGY, vol. 15, no. 12, 1997, pages 1266-1270, XP002603448 ISSN: 1087-0156
- HIGUCHI C. ET AL.: 'Hone Keisei Inshi (BMP)' JAPANESE JOURNAL OF CLINICAL MEDICINE vol. 62, no. SUPPL. 2, 2004, pages 52 - 56
- HODA N.: 'OPG' JOURNAL OF OSTEOPOROTIC MEDICINE vol. 2, no. 3, 2003, pages 213 - 218
- HODA N.: 'OPG Seizai. Ko-RANKL Kotai' JAPANESE JOURNAL OF CLINICAL MEDICINE vol. 63, no. 9, 2005, pages 1647 - 1653
- AOKI K. ET AL.: 'A TNF receptor loop peptide mimic blocks RANK ligand-induced signaling, bone resorption, and bone loss' THE JOURNAL OF CLINICAL INVESTIGATION vol. 116, no. 6, 2006, pages 1525 - 1534, XP008126832
- CHAVASSIEUX P ET AL: "Insights into material and structural basis of bone fragility from diseases associated with fractures: How determinants of the biomechanical properties of bone are compromised by disease", ENDOCRINE REVIEWS, vol. 28, no. 2, April 2007 (2007-04), pages 151-164, ISSN: 0163-769X

### SEQUENCE LISTING

<110> ORIENTAL YEAST Co., Ltd.
<120> A novel agent for increasing bone mass
<130> PH-3583-PCT
<150> JP2007-149799
   <151> 2007-06-05
<150> JP2007-313822
   <151> 2007-12-04
<150> JP2008-060145
   <151> 2008-03-10
<150> JP2008-131572
   <151> 2008-05-20
<160> 55
<170> PatentIn version 3.4
<210> 1
   <211> 2201
   <212> DNA
   <213> Homo sapiens
<220>
   <221> CDS
   <222> (129).. (1082)
<400> 1
   ggccaaagcc gggctccaag tcggcgcccc acgtcgaggc tccgccgcag cctccggagt      60
   tggccgcaga caagaagggg agggagcggg agagggagga gagctccgaa gcgagagggc     120
   cgagcgcc atg cgc cgc gcc agc aga gac tac acc aag tac ctg cgt ggc      170
            Met Arg Arg Ala Ser Arg Asp Tyr Thr Lys Tyr Leu Arg Gly
            1               5                   10
   tcg gag gag atg ggc ggc ggc ccc gga gcc ccg cac gag ggc ccc ctg 218
   Ser Glu Glu Met Gly Gly Gly Pro Gly Ala Pro His Glu Gly Pro Leu
   15 20 25 30
   cac gcc ccg ccg ccg cct gcg ccg cac cag ccc ccc gcc gcc tcc cgc 266
   His Ala Pro Pro Pro Pro Ala Pro His Gln Pro Pro Ala Ala Ser Arg
   35 40 45
   tcc atg ttc gtg gcc ctc ctg ggg ctg ggg ctg ggc cag gtt gtc tgc 314
   Ser Met Phe Val Ala Leu Leu Gly Leu Gly Leu Gly Gln Val Val Cys
   50 55 60
   agc gtc gcc ctg ttc ttc tat ttc aga gcg cag atg gat cct aat aga 362
   Ser Val Ala Leu Phe Phe Tyr Phe Arg Ala Gln Met Asp Pro Asn Arg
   65 70 75
   ata tca gaa gat ggc act cac tgc att tat aga att ttg aga ctc cat 410
   Ile Ser Glu Asp Gly Thr His Cys Ile Tyr Arg Ile Leu Arg Leu His
   80 85 90
   gaa aat gca gat ttt caa gac aca act ctg gag agt caa gat aca aaa 458
   Glu Asn Ala Asp Phe Gln Asp Thr Thr Leu Glu Ser Gln Asp Thr Lys
   95 100 105 110
   tta ata cct gat tca tgt agg aga att aaa cag gcc ttt caa gga gct 506
   Leu Ile Pro Asp Ser Cys Arg Arg Ile Lys Gln Ala Phe Gln Gly Ala
   115 120 125
   gtg caa aag gaa tta caa cat atc gtt gga tca cag cac atc aga gca 554
   Val Gln Lys Glu Leu Gln His Ile Val Gly Ser Gln His Ile Arg Ala
   130 135 140
   gag aaa gcg atg gtg gat ggc tca tgg tta gat ctg gcc aag agg agc 602
   Glu Lys Ala Met Val Asp Gly Ser Trp Leu Asp Leu Ala Lys Arg Ser
   145 150 155
   aag ctt gaa gct cag cct ttt gct cat ctc act att aat gcc acc gac 650
   Lys Leu Glu Ala Gln Pro Phe Ala His Leu Thr Ile Asn Ala Thr Asp
   160 165 170
   atc cca tct ggt tcc cat aaa gtg agt ctg tcc tct tgg tac cat gat 698
   Ile Pro Ser Gly Ser His Lys Val Ser Leu Ser Ser Trp Tyr His Asp
   175 180 185 190
   cgg ggt tgg gcc aag atc tcc aac atg act ttt agc aat gga aaa cta 746
   Arg Gly Trp Ala Lys Ile Ser Asn Met Thr Phe Ser Asn Gly Lys Leu
   195 200 205
   ata gtt aat cag gat ggc ttt tat tac ctg tat gcc aac att tgc ttt 794
   Ile Val Asn Gln Asp Gly Phe Tyr Tyr Leu Tyr Ala Asn Ile Cys Phe
   210 215 220
   cga cat cat gaa act tca gga gac cta gct aca gag tat ctt caa cta 842
   Arg His His Glu Thr Ser Gly Asp Leu Ala Thr Glu Tyr Leu Gln Leu
   225 230 235
   atg gtg tac gtc act aaa acc agc atc aaa atc cca agt tct cat acc 890
   Met Val Tyr Val Thr Lys Thr Ser Ile Lys Ile Pro Ser Ser His Thr
   240 245 250
   ctg atg aaa gga gga agc acc aag tat tgg tca ggg aat tct gaa ttc 938
   Leu Met Lys Gly Gly Ser Thr Lys Tyr Trp Ser Gly Asn Ser Glu Phe
   255 260 265 270
   cat ttt tat tcc ata aac gtt ggt gga ttt ttt aag tta cgg tct gga 986
   His Phe Tyr Ser Ile Asn Val Gly Gly Phe Phe Lys Leu Arg Ser Gly
   275 280 285
   gag gaa atc agc atc gag gtc tcc aac ccc tcc tta ctg gat ccg gat 1034
   Glu Glu Ile Ser Ile Glu Val Ser Asn Pro Ser Leu Leu Asp Pro Asp
   290 295 300
   cag gat gca aca tac ttt ggg gct ttt aaa gtt cga gat ata gat tga 1082
   Gln Asp Ala Thr Tyr Phe Gly Ala Phe Lys Val Arg Asp Ile Asp
   305 310 315
   gccccagttt ttggagtgtt atgtatttcc tggatgtttg gaaacatttt ttaaaacaag 1142
   ccaagaaaga tgtatatagg tgtgtgagac tactaagagg catggcccca acggtacacg 1202
   actcagtatc catgctcttg accttgtaga gaacacgcgt atttacctgc cagtgggaga 1262
   tgttagactc atggtgtgtt acacaatggt ttttaaattt tgtaatgaat tcctagaatt 1322
   aaaccagatt ggagcaatta cgggttgacc ttatgagaaa ctgcatgtgg gctatgggag 1382
   gggttggtcc ctggtcatgt gccccttcgc agctgaagtg gagagggtgt catctagcgc 1442
   aattgaagga tcatctgaag gggcaaattc ttttgaattg ttacatcatg ctggaacctg 1502
   caaaaaatac tttttctaat gaggagagaa aatatatgta tttttatata atatctaaag 1562
   ttatatttca gatgtaatgt tttctttgca aagtattgta aattatattt gtgctatagt 1622
   atttgattca aaatatttaa aaatgtcttg ctgttgacat atttaatgtt ttaaatgtac 1682
   agacatattt aactggtgca ctttgtaaat tccctgggga aaacttgcag ctaaggaggg 1742
   gaaaaaaatg ttgtttccta atatcaaatg cagtatattt cttcgttctt tttaagttaa 1802
   tagatttttt cagacttgtc aagcctgtgc aaaaaaatta aaatggatgc cttgaataat 1862
   aagcaggatg ttggccacca ggtgcctttc aaatttagaa actaattgac tttagaaagc 1922
   tgacattgcc aaaaaggata cataatgggc cactgaaatt tgtcaagagt agttatataa 1982
   ttgttgaaca ggtgtttttc cacaagtgcc gcaaattgta cctttttttt tttttcaaaa 2042
   tagaaaagtt attagtggtt tatcagcaaa aaagtccaat tttaatttag taaatgttat 2102
   tttatactgt acaataaaaa cattgccttt gaatgttaat tttttggtac aaaaataaat 2162
   ttatatgaaa aaaaaaaaaa aaaaaaaaaa aaaaaaaaa 2201
<210> 2
   <211> 317
   <212> PRT
   <213> Homo sapiens
<400> 2
   Met Arg Arg Ala Ser Arg Asp Tyr Thr Lys Tyr Leu Arg Gly Ser Glu
   1 5 10 15
   Glu Met Gly Gly Gly Pro Gly Ala Pro His Glu Gly Pro Leu His Ala
   20 25 30
   Pro Pro Pro Pro Ala Pro His Gln Pro Pro Ala Ala Ser Arg Ser Met
   35 40 45
   Phe Val Ala Leu Leu Gly Leu Gly Leu Gly Gln Val Val Cys Ser Val
   50 55 60
   Ala Leu Phe Phe Tyr Phe Arg Ala Gln Met Asp Pro Asn Arg Ile Ser
   65 70 75 80
   Glu Asp Gly Thr His Cys Ile Tyr Arg Ile Leu Arg Leu His Glu Asn
   85 90 95
   Ala Asp Phe Gln Asp Thr Thr Leu Glu Ser Gln Asp Thr Lys Leu Ile
   100 105 110
   Pro Asp Ser Cys Arg Arg Ile Lys Gln Ala Phe Gln Gly Ala Val Gln
   115 120 125
   Lys Glu Leu Gln His Ile Val Gly Ser Gln His Ile Arg Ala Glu Lys
   130 135 140
   Ala Met Val Asp Gly Ser Trp Leu Asp Leu Ala Lys Arg Ser Lys Leu
   145 150 155 160
   Glu Ala Gln Pro Phe Ala His Leu Thr Ile Asn Ala Thr Asp Ile Pro
   165 170 175
   Ser Gly Ser His Lys Val Ser Leu Ser Ser Trp Tyr His Asp Arg Gly
   180 185 190
   Trp Ala Lys Ile Ser Asn Met Thr Phe Ser Asn Gly Lys Leu Ile Val
   195 200 205
   Asn Gln Asp Gly Phe Tyr Tyr Leu Tyr Ala Asn Ile Cys Phe Arg His
   210 215 220
   His Glu Thr Ser Gly Asp Leu Ala Thr Glu Tyr Leu Gln Leu Met Val
   225 230 235 240
   Tyr Val Thr Lys Thr Ser Ile Lys Ile Pro Ser Ser His Thr Leu Met
   245 250 255
   Lys Gly Gly Ser Thr Lys Tyr Trp Ser Gly Asn Ser Glu Phe His Phe
   260 265 270
   Tyr Ser Ile Asn Val Gly Gly Phe Phe Lys Leu Arg Ser Gly Glu Glu
   275 280 285
   Ile Ser Ile Glu Val Ser Asn Pro Ser Leu Leu Asp Pro Asp Gln Asp
   290 295 300
   Ala Thr Tyr Phe Gly Ala Phe Lys Val Arg Asp Ile Asp
   305 310 315
<210> 3
   <211> 3133
   <212> DNA
   <213> Homo sapiens
<220>
   <221> CDS
   <222> (39).. (1889)
<400> 3
ccgctgaggc cgcggcgccc gccagcctgt cccgcgcc atg gcc ccg cgc gcc cgg      56
   cgg cgc cgc ccg ctg ttc gcg ctg ctg ctg ctc tgc gcg ctg ctc gcc       104
   cgg ctg cag gtg gct ttg cag atc gct cct cca tgt acc agt gag aag       152
   cat tat gag cat ctg gga cgg tgc tgt aac aaa tgt gaa cca gga aag       200
   tac atg tct tct aaa tgc act act acc tct gac agt gta tgt ctg ccc       248
   tgt ggc ccg gat gaa tac ttg gat agc tgg aat gaa gaa gat aaa tgc       296
   ttg ctg cat aaa gtt tgt gat aca ggc aag gcc ctg gtg gcc gtg gtc       344
   gcc ggc aac agc acg acc ccc cgg cgc tgc gcg tgc acg gct ggg tac       392
   cac tgg agc cag gac tgc gag tgc tgc cgc cgc aac acc gag tgc gcg       440
   ccg ggc ctg ggc gcc cag cac ccg ttg cag ctc aac aag gac aca gtg       488
   tgc aaa cct tgc ctt gca ggc tac ttc tct gat gcc ttt tcc tcc acg 536
   gac aaa tgc aga ccc tgg acc aac tgt acc ttc ctt gga aag aga gta 584
   gaa cat cat ggg aca gag aaa tcc gat gcg gtt tgc agt tct tct ctg 632
   cca gct aga aaa cca cca aat gaa ccc cat gtt tac ttg ccc ggt tta 680
   ata att ctg ctt ctc ttc gcg tct gtg gcc ctg gtg gct gcc atc atc 728
   ttt ggc gtt tgc tat agg aaa aaa ggg aaa gca ctc aca gct aat ttg 776
   tgg cac tgg atc aat gag gct tgt ggc cgc cta agt gga gat aag gag 824
   tcc tca ggt gac agt tgt gtc agt aca cac acg gca aac ttt ggt cag 872
   cag gga gca tgt gaa ggt gtc tta ctg ctg act ctg gag gag aag aca 920
   ttt cca gaa gat atg tgc tac cca gat caa ggt ggt gtc tgt cag ggc 968
   aca tgt gta gga ggt ggt ccc tac gca caa ggc gaa gat gcc agg atg 1016
   ctc tca ttg gtc agc aag acc gag ata gag gaa gac agc ttc aga cag 1064
   atg ccc aca gaa gat gaa tac atg gac agg ccc tcc cag ccc aca gac 1112
   cag tta ctg ttc ctc act gag cct gga agc aaa tcc aca cct cct ttc 1160
   tct gaa ccc ctg gag gtg ggg gag aat gac agt tta agc cag tgc ttc 1208
   acg ggg aca cag agc aca gtg ggt tca gaa agc tgc aac tgc act gag 1256
   ccc ctg tgc agg act gat tgg act ccc atg tcc tct gaa aac tac ttg 1304
   caa aaa gag gtg gac agt ggc cat tgc ccg cac tgg gca gcc agc ccc 1352
   agc ccc aac tgg gca gat gtc tgc aca ggc tgc cgg aac cct cct ggg 1400
   gag gac tgt gaa ccc ctc gtg ggt tcc cca aaa cgt gga ccc ttg ccc 1448
   cag tgc gcc tat ggc atg ggc ctt ccc cct gaa gaa gaa gcc agc agg 1496
   acg gag gcc aga gac cag ccc gag gat ggg gct gat ggg agg ctc cca 1544
   agc tca gcg agg gca ggt gcc ggg tct gga agc tcc cct ggt ggc cag 1592
   tcc cct gca tct gga aat gtg act gga aac agt aac tcc acg ttc atc 1640
   tcc agc ggg cag gtg atg aac ttc aag ggc gac atc atc gtg gtc tac 1688
   gtc agc cag acc tcg cag gag ggc gcg gcg gcg gct gcg gag ccc atg 1736
   ggc cgc ccg gtg cag gag gag acc ctg gcg cgc cga gac tcc ttc gcg 1784
   ggg aac ggc ccg cgc ttc ccg gac ccg tgc ggc ggc ccc gag ggg ctg 1832
   cgg gag ccg gag aag gcc tcg agg ccg gtg cag gag caa ggc ggg gcc 1880
   aag gct tga gcgcccccca tggctgggag cccgaagctc ggagccaggg 1929
   ctcgcgaggg cagcaccgca gcctctgccc cagccccggc cacccaggga tcgatcggta 1989
   cagtcgagga agaccacccg gcattctctg cccactttgc cttccaggaa atgggctttt 2049
   caggaagtga attgatgagg actgtcccca tgcccacgga tgctcagcag cccgccgcac 2109
   tggggcagat gtctcccctg ccactcctca aactcgcagc agtaatttgt ggcactatga 2169
   cagctatttt tatgactatc ctgttctgtg gggggggggg tctgttttcc ccccatattt 2229
   gtattccttt tcataacttt tcttgatatc tttcctccct cttttttaat gtaaaggttt 2289
   tctcaaaaat tctcctaaag gtgagggtct ctttcttttc tcttttcctt ttttttttct 2349
   ttttttggca acctggctct ggcccaggct agagtgcagt ggtgcgatta tagcccggtg 2409
   cagcctctaa ctcctgggct caagcaatcc aagtgatcct cccacctcaa ccttcggagt 2469
   agctgggatc acagctgcag gccacgccca gcttcctccc cccgactccc cccccagaga 2529
   cacggtccca ccatgttacc cagcctggtc tcaaactccc cagctaaagc agtcctccag 2589
   cctcggcctc ccaaagtact gggattacag gcgtgagccc ccacgctggc ctgctttacg 2649
   tattttcttt tgtgcccctg ctcacagtgt tttagagatg gctttcccag tgtgtgttca 2709
   ttgtaaacac ttttgggaaa gggctaaaca tgtgaggcct ggagatagtt gctaagttgc 2769
   taggaacatg tggtgggact ttcatattct gaaaaatgtt ctatattctc atttttctaa 2829
   aagaaagaaa aaaggaaacc cgatttattt ctcctgaatc tttttaagtt tgtgtcgttc 2889
   cttaagcaga actaagctca gtatgtgacc ttacccgcta ggtggttaat ttatccatgc 2949
   tggcagaggc actcaggtac ttggtaagca aatttctaaa actccaagtt gctgcagctt 3009
   ggcattcttc ttattctaga ggtctctctg gaaaagatgg agaaaatgaa caggacatgg 3069
   ggctcctgga aagaaagggc ccgggaagtt caaggaagaa taaagttgaa attttaaaaa 3129
   aaaa 3133
<210> 4
   <211> 616
   <212> PRT
   <213> Homo sapiens
<400> 4
<210> 5
   <211> 2291
   <212> DNA
   <213> Homo sapiens
<220>
   <221> CDS
   <222> (252) .. (1457)
<400> 5
   ctttccgccc cagccctgaa agcgttaacc ctggagcttt ctgcacaccc cccgaccgct    60
   cccgcccaag cttcctaaaa aagaaaggtg caaagtttgg tccaggatag aaaaatgact   120
   gatcaaaggc aggcgatact tcctgttgcc gggacgctat atataacgtg atgagcgcac   180
   gggctgcgga gacgcaccgg agcgctcgcc cagccgccgc ctccaagccc ctgaggtttc   240
   cggggaccac a atg aac aag ttg ctg tgc tgc gcg ctc gtg ttt ctg gac    290
                Met Asn Lys Leu Leu Cys Cys Ala Leu Val Phe Leu Asp
                1               5                   10
   atc tcc att aag tgg acc acc cag gaa acg ttt cct cca aag tac ctt     338
   Ile Ser Ile Lys Trp Thr Thr Gln Glu Thr Phe Pro Pro Lys Tyr Leu
       15                  20                  25
   cat tat gac gaa gaa acc tct cat cag ctg ttg tgt gac aaa tgt cct     386
   His Tyr Asp Glu Glu Thr Ser His Gln Leu Leu Cys Asp Lys Cys Pro
   30                  35                  40                  45
   cct ggt acc tac cta aaa caa cac tgt aca gca aag tgg aag acc gtg     434
   Pro Gly Thr Tyr Leu Lys Gln His Cys Thr Ala Lys Trp Lys Thr Val
                   50                  55                  60
   tgc gcc cct tgc cct gac cac tac tac aca gac agc tgg cac acc agt     482
   Cys Ala Pro Cys Pro Asp His Tyr Tyr Thr Asp Ser Trp His Thr Ser
               65                  70                  75
   gac gag tgt cta tac tgc agc ccc gtg tgc aag gag ctg cag tac gtc     530
   Asp Glu Cys Leu Tyr Cys Ser Pro Val Cys Lys Glu Leu Gln Tyr Val
           80                  85                  90
   aag cag gag tgc aat cgc acc cac aac cgc gtg tgc gaa tgc aag gaa     578
   Lys Gln Glu Cys Asn Arg Thr His Asn Arg Val Cys Glu Cys Lys Glu
       95                  100                 105
   ggg cgc tac ctt gag ata gag ttc tgc ttg aaa cat agg agc tgc cct     626
   Gly Arg Tyr Leu Glu Ile Glu Phe Cys Leu Lys His Arg Ser Cys Pro
   110                 115                 120                 125
   cct gga ttt gga gtg gtg caa gct gga acc cca gag cga aat aca gtt 674
   Pro Gly Phe Gly Val Val Gln Ala Gly Thr Pro Glu Arg Asn Thr Val
   130 135 140
   tgc aaa aga tgt cca gat ggg ttc ttc tca aat gag acg tca tct aaa 722
   Cys Lys Arg Cys Pro Asp Gly Phe Phe Ser Asn Glu Thr Ser Ser Lys
   145 150 155
   gca ccc tgt aga aaa cac aca aat tgc agt gtc ttt ggt ctc ctg cta 770
   Ala Pro Cys Arg Lys His Thr Asn Cys Ser Val Phe Gly Leu Leu Leu
   160 165 170
   act cag aaa gga aat gca aca cac gac aac ata tgt tcc gga aac agt 818
   Thr Gln Lys Gly Asn Ala Thr His Asp Asn Ile Cys Ser Gly Asn Ser
   175 180 185
   gaa tca act caa aaa tgt gga ata gat gtt acc ctg tgt gag gag gca 866
   Glu Ser Thr Gln Lys Cys Gly Ile Asp Val Thr Leu Cys Glu Glu Ala
   190 195 200 205
   ttc ttc agg ttt gct gtt cct aca aag ttt acg cct aac tgg ctt agt 914
   Phe Phe Arg Phe Ala Val Pro Thr Lys Phe Thr Pro Asn Trp Leu Ser
   210 215 220
   gtc ttg gta gac aat ttg cct ggc acc aaa gta aac gca gag agt gta 962
   Val Leu Val Asp Asn Leu Pro Gly Thr Lys Val Asn Ala Glu Ser Val
   225 230 235
   gag agg ata aaa cgg caa cac agc tca caa gaa cag act ttc cag ctg 1010
   Glu Arg Ile Lys Arg Gln His Ser Ser Gln Glu Gln Thr Phe Gln Leu
   240 245 250
   ctg aag tta tgg aaa cat caa aac aaa gac caa gat ata gtc aag aag 1058
   Leu Lys Leu Trp Lys His Gln Asn Lys Asp Gln Asp Ile Val Lys Lys
   255 260 265
   atc atc caa gat att gac ctc tgt gaa aac agc gtg cag cgg cac att 1106
   Ile Ile Gln Asp Ile Asp Leu Cys Glu Asn Ser Val Gln Arg His Ile
   270 275 280 285
   gga cat gct aac ctc acc ttc gag cag ctt cgt agc ttg atg gaa agc 1154
   Gly His Ala Asn Leu Thr Phe Glu Gln Leu Arg Ser Leu Met Glu Ser
   290 295 300
   tta ccg gga aag aaa gtg gga gca gaa gac att gaa aaa aca ata aag 1202
   Leu Pro Gly Lys Lys Val Gly Ala Glu Asp Ile Glu Lys Thr Ile Lys
   305 310 315
   gca tgc aaa ccc agt gac cag atc ctg aag ctg ctc agt ttg tgg cga 1250
   Ala Cys Lys Pro Ser Asp Gln Ile Leu Lys Leu Leu Ser Leu Trp Arg
   320 325 330
   ata aaa aat ggc gac caa gac acc ttg aag ggc cta atg cac gca cta 1298
   Ile Lys Asn Gly Asp Gln Asp Thr Leu Lys Gly Leu Met His Ala Leu
   335 340 345
   aag cac tca aag acg tac cac ttt ccc aaa act gtc act cag agt cta 1346
   Lys His Ser Lys Thr Tyr His Phe Pro Lys Thr Val Thr Gln Ser Leu
   350 355 360 365
   aag aag acc atc agg ttc ctt cac agc ttc aca atg tac aaa ttg tat 1394
   Lys Lys Thr Ile Arg Phe Leu His Ser Phe Thr Met Tyr Lys Leu Tyr
   370 375 380
   cag aag tta ttt tta gaa atg ata ggt aac cag gtc caa tca gta aaa 1442
   Gln Lys Leu Phe Leu Glu Met Ile Gly Asn Gln Val Gln Ser Val Lys
   385 390 395
   ata agc tgc tta taa ctggaaatgg ccattgagct gtttcctcac aattggcgag 1497
   Ile Ser Cys Leu
   400
   atcccatgga tgagtaaact gtttctcagg cacttgaggc tttcagtgat atctttctca 1557
   ttaccagtga ctaattttgc cacagggtac taaaagaaac tatgatgtgg agaaaggact 1617
   aacatctcct ccaataaacc ccaaatggtt aatccaactg tcagatctgg atcgttatct 1677
   actgactata ttttccctta ttactgcttg cagtaattca actggaaatt aaaaaaaaaa 1737
   aactagactc cattgtgcct tactaaatat gggaatgtct aacttaaata gctttgagat 1797
   ttcagctatg ctagaggctt ttattagaaa gccatatttt tttctgtaaa agttactaat 1857
   atatctgtaa cactattaca gtattgctat ttatattcat tcagatataa gatttgtaca 1917
   tattatcatc ctataaagaa acggtatgac ttaattttag aaagaaaatt atattctgtt 1977
   tattatgaca aatgaaagag aaaatatata tttttaatgg aaagtttgta gcatttttct 2037
   aataggtact gccatatttt tctgtgtgga gtatttttat aattttatct gtataagctg 2097
   taatatcatt ttatagaaaa tgcattattt agtcaattgt ttaatgttgg aaaacatatg 2157
   aaatataaat tatctgaata ttagatgctc tgagaaattg aatgtacctt atttaaaaga 2217
   ttttatggtt ttataactat ataaatgaca ttattaaagt tttcaaatta ttttttaaaa 2277
   aaaaaaaaaa aaaa 2291
<210> 6
   <211> 401
   <212> PRT
   <213> Homo sapiens
<400> 6
   Met Asn Lys Leu Leu Cys Cys Ala Leu Val Phe Leu Asp Ile Ser Ile
   1 5 10 15
   Lys Trp Thr Thr Gln Glu Thr Phe Pro Pro Lys Tyr Leu His Tyr Asp
   20 25 30
   Glu Glu Thr Ser His Gln Leu Leu Cys Asp Lys Cys Pro Pro Gly Thr
   35 40 45
   Tyr Leu Lys Gln His Cys Thr Ala Lys Trp Lys Thr Val Cys Ala Pro
   50 55 60
   Cys Pro Asp His Tyr Tyr Thr Asp Ser Trp His Thr Ser Asp Glu Cys
   65 70 75 80
   Leu Tyr Cys Ser Pro Val Cys Lys Glu Leu Gln Tyr Val Lys Gln Glu
   85 90 95
   Cys Asn Arg Thr His Asn Arg Val Cys Glu Cys Lys Glu Gly Arg Tyr
   100 105 110
   Leu Glu Ile Glu Phe Cys Leu Lys His Arg Ser Cys Pro Pro Gly Phe
   115 120 125
   Gly Val Val Gln Ala Gly Thr Pro Glu Arg Asn Thr Val Cys Lys Arg
   130 135 140
   Cys Pro Asp Gly Phe Phe Ser Asn Glu Thr Ser Ser Lys Ala Pro Cys
   145 150 155 160
   Arg Lys His Thr Asn Cys Ser Val Phe Gly Leu Leu Leu Thr Gln Lys
   165 170 175
   Gly Asn Ala Thr His Asp Asn Ile Cys Ser Gly Asn Ser Glu Ser Thr
   180 185 190
   Gln Lys Cys Gly Ile Asp Val Thr Leu Cys Glu Glu Ala Phe Phe Arg
   195 200 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ile Asp Leu Cys Glu Asn Ser Val Gln Arg His Ile Gly His Ala
   275 280 285
   Asn Leu Thr Phe Glu Gln Leu Arg Ser Leu Met Glu Ser Leu Pro Gly
   290 295 300
   Lys Lys Val Gly Ala Glu Asp Ile Glu Lys Thr Ile Lys Ala Cys Lys
   305 310 315 320
   Pro Ser Asp Gln Ile Leu Lys Leu Leu Ser Leu Trp Arg Ile Lys Asn
   325 330 335
   Gly Asp Gln Asp Thr Leu Lys Gly Leu Met His Ala Leu Lys His Ser
   340 345 350
   Lys Thr Tyr His Phe Pro Lys Thr Val Thr Gln Ser Leu Lys Lys Thr
   355 360 365
   Ile Arg Phe Leu His Ser Phe Thr Met Tyr Lys Leu Tyr Gln Lys Leu
   370 375 380
   Phe Leu Glu Met Ile Gly Asn Gln Val Gln Ser Val Lys Ile Ser Cys
   385 390 395 400
   Leu
<210> 7
   <211> 9
   <212> PRT
   <213> Artificial
<220>
   <223> Synthetic
<220>
   <223> 2nd Cys is linked to 8th Cys through disulfide bond
<400> 7
   Tyr Cys Trp Ser Gln Tyr Leu Cys Tyr
   1 5
<210> 8
   <211> 20
   <212> DNA
   <213> Artificial
<220>
   <223> primer
<400> 8
   gggggtggcc ggaaatacat 20
<210> 9
   <211> 24
   <212> DNA
   <213> Artificial
<220>
   <223> primer
<400> 9
   gggggccaga ccaaagatag agtt 24
<210> 10
   <211> 20
   <212> DNA
   <213> Artificial
<220>
   <223> primer
<400> 10
   attccagttc gagtatggcg 20
<210> 11
   <211> 25
   <212> DNA
   <213> Artificial
<220>
   <223> primer
<400> 11
   ttttgtattc aatcactgtc ttgcc 25
<210> 12
   <211> 26
   <212> DNA
   <213> Artificial
<220>
   <223> primer
<400> 12
   tgaaggtcgg agtcaacgga tttggt 26
<210> 13
   <211> 24
   <212> DNA
   <213> Artificial
<220>
   <223> primer
<400> 13 24
   catgtgggcc atgaggtcca ccac 24
<210> 14
   <211> 1236
   <212> DNA
   <213> Artificial
<220>
   <223> GST-RANKL (aa140-317)
<400> 14
   atgtccccta tactaggtta ttggaaaatt aagggccttg tgcaacccac tcgacttctt 60
   ttggaatatc ttgaagaaaa atatgaagag catttgtatg agcgcgatga aggtgataaa 120
   tggcgaaaca aaaagtttga attgggtttg gagtttccca atcttcctta ttatattgat 180
   ggtgatgtta aattaacaca gtctatggcc atcatacgtt atatagctga caagcacaac 240
   atgttgggtg gttgtccaaa agagcgtgca gagatttcaa tgcttgaagg agcggttttg 300
   gatattagat acggtgtttc gagaattgca tatagtaaag actttgaaac tctcaaagtt 360
   gattttctta gcaagctacc tgaaatgctg aaaatgttcg aagatcgttt atgtcataaa 420
   acatatttaa atggtgatca tgtaacccat cctgacttca tgttgtatga cgctcttgat 480
   gttgttttat acatggaccc aatgtgcctg gatgcgttcc caaaattagt ttgttttaaa 540
   aaacgtattg aagctatccc acaaattgat aagtacttga aatccagcaa gtatatagca 600
   tggcctttgc agggctggca agccacgttt ggtggtggcg accatcctcc aaaatcggat 660
   ctggttccgc gtggatcccc aggaattccc gggtcgacta tcagagcaga gaaagcgatg 720
   gtggatggct catggttaga tctggccaag aggagcaagc ttgaagctca gccttttgct 780
   catctcacta ttaatgccac cgacatccca tctggttccc ataaagtgag tctgtcctct 840
   tggtaccatg atcggggttg ggccaagatc tccaacatga cttttagcaa tggaaaacta 900
   atagttaatc aggatggctt ttattacctg tatgccaaca tttgctttcg acatcatgaa 960
   acttcaggag acctagctac agagtatctt caactaatgg tgtacgtcac taaaaccagc 1020
   atcaaaatcc caagttctca taccctgatg aaaggaggaa gcaccaagta ttggtcaggg 1080
   aattctgaat tccattttta ttccataaac gttggtggat tttttaagtt acggtctgga 1140
   gaggaaatca gcatcgaggt ctccaacccc tccttactgg atccggatca ggatgcaaca 1200
   tactttgggg cttttaaagt tcgagatata gattga 1236
<210> 15
   <211> 429
   <212> PRT
   <213> Artificial
<220>
   <223> GST-RANKL (aa140-317)
<400> 15
   Met Ser Pro Ile Leu Gly Tyr Trp Lys Ile Lys Gly Leu Val Gln Pro
   1 5 10 15
   Thr Arg Leu Leu Leu Glu Tyr Leu Glu Glu Lys Tyr Glu Glu His Leu
   20 25 30
   Tyr Glu Arg Asp Glu Gly Asp Lys Trp Arg Asn Lys Lys Phe Glu Leu
   35 40 45
   Gly Leu Glu Phe Pro Asn Leu Pro Tyr Tyr Ile Asp Gly Asp Val Lys
   50 55 60
   Leu Thr Gln Ser Met Ala Ile Ile Arg Tyr Ile Ala Asp Lys His Asn
   65 70 75 80
   Met Leu Gly Gly Cys Pro Lys Glu Arg Ala Glu Ile Ser Met Leu Glu
   85 90 95
   Gly Ala Val Leu Asp Ile Arg Tyr Gly Val Ser Arg Ile Ala Tyr Ser
   100 105 110
   Lys Asp Phe Glu Thr Leu Lys Val Asp Phe Leu Ser Lys Leu Pro Glu
   115 120 125
   Met Leu Lys Met Phe Glu Asp Arg Leu Cys His Lys Thr Tyr Leu Asn
   130 135 140
   Gly Asp His Val Thr His Pro Asp Phe Met Leu Tyr Asp Ala Leu Asp
   145 150 155 160
   Val Val Leu Tyr Met Asp Pro Met Cys Leu Asp Ala Phe Pro Lys Leu
   165 170 175
   Val Cys Phe Lys Lys Arg Ile Glu Ala Ile Pro Gln Ile Asp Lys Tyr
   180 185 190
   Leu Lys Ser Ser Lys Tyr Ile Ala Trp Pro Leu Gln Gly Trp Gln Ala
   195 200 205
   Thr Phe Gly Gly Gly Asp His Pro Pro Lys Ser Asp Leu Val Pro Arg
   210 215 220
   Gly Ser Pro Gly Ile Pro Gly Ser Thr Arg Ala Ala Ala Ser Leu Val
   225 230 235 240
   Pro Arg Gly Ser Pro Gly Ile Pro Gly Ser Thr Ile Arg Ala Glu Lys
   245 250 255
   Ala Met Val Asp Gly Ser Trp Leu Asp Leu Ala Lys Arg Ser Lys Leu
   260 265 270
   Glu Ala Gln Pro Phe Ala His Leu Thr Ile Asn Ala Thr Asp Ile Pro
   275 280 285
   Ser Gly Ser His Lys Val Ser Leu Ser Ser Trp Tyr His Asp Arg Gly
   290 295 300
   Trp Ala Lys Ile Ser Asn Met Thr Phe Ser Asn Gly Lys Leu Ile Val
   305 310 315 320
   Asn Gln Asp Gly Phe Tyr Tyr Leu Tyr Ala Asn Ile Cys Phe Arg His
   325 330 335
   His Glu Thr Ser Gly Asp Leu Ala Thr Glu Tyr Leu Gln Leu Met Val
   340 345 350
   Tyr Val Thr Lys Thr Ser Ile Lys Ile Pro Ser Ser His Thr Leu Met
   355 360 365
   Lys Gly Gly Ser Thr Lys Tyr Trp Ser Gly Asn Ser Glu Phe His Phe
   370 375 380
   Tyr Ser Ile Asn Val Gly Gly Phe Phe Lys Leu Arg Ser Gly Glu Glu
   385 390 395 400
   Ile Ser Ile Glu Val Ser Asn Pro Ser Leu Leu Asp Pro Asp Gln Asp
   405 410 415
   Ala Thr Tyr Phe Gly Ala Phe Lys Val Arg Asp Ile Asp
   420 425
<210> 16
   <211> 9
   <212> PRT
   <213> Artificial
<220>
   <223> Synthetic
<220>
   <223> 2nd Cys is linked to 8th Cys through disulfide bond
<400> 16
   Tyr Cys Trp Ser Gln Asn Leu Cys Tyr
   1 5
<210> 17
   <211> 24
   <212> DNA
   <213> Artificial
<220>
   <223> Primer
<400> 17
   ggcaagcctg aggcccagcc attt 24
<210> 18
   <211> 24
   <212> DNA
   <213> Artificial
<220>
   <223> Primer
<400> 18 24
   gtctcagtct atgtcctgaa cttt 24
<210> 19
   <211> 20
   <212> DNA
   <213> Artificial
<220>
   <223> Primer
<400> 19 20
   caccatggag aaggccgggg 20
<210> 20
   <211> 20
   <212> DNA
   <213> Artificial
<220>
   <223> Primer
<400> 20 20
   gacggacaca ttgggggtag 20
<210> 21
   <211> 20
   <212> DNA
   <213> Artificial
<220>
   <223> Primer
<400> 21
   ccaagcaggc tctgcatgaa 20
<210> 22
   <211> 20
   <212> DNA
   <213> Artificial
<220>
   <223> Primer
<400> 22
   gccagaccaa agatggagtt 20
<210> 23
   <211> 21
   <212> DNA
   <213> Artificial
<220>
   <223> Primer
<400> 23
   tctgacaaag ccttcatgtc 21
<210> 24
   <211> 22
   <212> DNA
   <213> Artificial
<220>
   <223> Primer
<400> 24
   aaatagtgat accatagatg g 22
<210> 25
   <211> 18
   <212> DNA
   <213> Artificial
<220>
   <223> Primer
<400> 25 18
   cctggtaaag atggtgcc 18
<210> 26
   <211> 22
   <212> DNA
   <213> Artificial
<220>
   <223> Primer
<400> 26 22
   caccaggttc acctttcgca cc 22
<210> 27
   <211> 20
   <212> DNA
   <213> Artificial
<220>
   <223> primer
<400> 27 20
   atgagggatc tttaccggct 20
<210> 28
   <211> 20
   <212> DNA
   <213> Artificial
<220>
   <223> primer
<400> 28
   tttatacggt ggaagccctg 20
<210> 29
   <211> 20
   <212> DNA
   <213> Artificial
<220>
   <223> primer
<400> 29
   agtgtgcact gccaaagatg 20
<210> 30
   <211> 20
   <212> DNA
   <213> Artificial
<220>
   <223> primer
<400> 30
   ggccaaatgt gtcttccagt 20
<210> 31
   <211> 20
   <212> DNA
   <213> Artificial
<220>
   <223> primer
<400> 31
   aagcggctct actgcaagaa 20
<210> 32
   <211> 20
   <212> DNA
   <213> Artificial
<220>
   <223> primer
<400> 32
   tcgtttcagt gccacatacc 20
<210> 33
   <211> 25
   <212> DNA
   <213> Artificial
<220>
   <223> primer
<400> 33
   ctttggcctg gccagggata tcaac 25
<210> 34
   <211> 24
   <212> DNA
   <213> Artificial
<220>
   <223> primer
<400> 34
   ccaactgctt gaatgtgggt ctct 24
<210> 35
   <211> 21
   <212> DNA
   <213> Artificial
<220>
   <223> primer
<400> 35
   cccgctgttc ggtttgcata c 21
<210> 36
   <211> 20
   <212> DNA
   <213> Artificial
<220>
   <223> primer
<400> 36
   acggttggca cggcttgaag 20
<210> 37
   <211> 23
   <212> DNA
   <213> Artificial
<220>
   <223> primer
<400> 37
   agcgtaactg gacatcacag cag 23
<210> 38
   <211> 24
   <212> DNA
   <213> Artificial
<220>
   <223> primer
<400> 38
   cggtgtcaca gtgctttctt gttg 24
<210> 39
   <211> 25
   <212> DNA
   <213> Artificial
<220>
   <223> primer
<400> 39 25
   gtctggtctt ttgggatcct actct 25
<210> 40
   <211> 23
   <212> DNA
   <213> Artificial
<220>
   <223> primer
<400> 40 23
   ctcctcatct acctgctggt act 23
<210> 41
   <211> 24
   <212> DNA
   <213> Artificial
<220>
   <223> primer
<400> 41 24
   ctgattcggt acctagagcc agat 24
<210> 42
   <211> 23
   <212> DNA
   <213> Artificial
<220>
   <223> primer
<400> 42
   ctgtcctctt tagctcttcc cgt 23
<210> 43
   <211> 44
   <212> DNA
   <213> Artificial
<220>
   <223> primer
<400> 43
   ctactgctgg agccagtacc tgtgctacgg tggaggtggt agcg 44
<210> 44
   <211> 48
   <212> DNA
   <213> Artificial
<220>
   <223> primer
<400> 44
   gatccgctac cacctccacc gtagcacagg tactggctcc agcagtag 48
<210> 45
   <211> 44
   <212> DNA
   <213> Artificial
<220>
   <223> primer
<400> 45
   ctactgcgct gcagctgcag cttgctacgg tggaggtggt agcg 44
<210> 46
   <211> 48
   <212> DNA
   <213> Artificial
<220>
   <223> primer
<400> 46
   gatccgctac cacctccacc gtagcaagct gcagctgcag cgcagtag 48
<210> 47
   <211> 9
   <212> PRT
   <213> Artificial
<220>
   <223> synthetic
<400> 47
   Tyr Cys Ala Ala Ala Ala Ala Cys Tyr
   1 5
<210> 48
   <211> 51
   <212> DNA
   <213> Artificial
<220>
   <223> primer
<400> 48
   gatccggtgg aggtggtagc tactgctgga gccagtacct gtgctactga g 51
<210> 49
   <211> 51
   <212> DNA
   <213> Artificial
<220>
   <223> primer
<400> 49 51
   aattctcagt agcacaggta ctggctccag cagtagctac cacctccacc g 51
<210> 50
   <211> 795
   <212> DNA
   <213> Artificial
<220>
   <223> synthetic
<220>
   <221> CDS
   <222> (1).. (795)
<400> 50
   atg tac agg atg caa ctc ctg tct tgc att gca cta agt ctt gca ctt      48
   Met Tyr Arg Met Gln Leu Leu Ser Cys Ile Ala Leu Ser Leu Ala Leu
   1               5                   10                  15
   gtc acg aat tcg atc tac tgc tgg agc cag tac ctg tgc tac ggt gga      96
   Val Thr Asn Ser Ile Tyr Cys Trp Ser Gln Tyr Leu Cys Tyr Gly Gly
               20                  25                  30
   ggt ggt agc gga tct gac aaa act cac aca tgc cca ccg tgc cca gca      144
   Gly Gly Ser Gly Ser Asp Lys Thr His Thr Cys Pro Pro Cys Pro Ala
           35                  40                  45
   cct gaa ctc ctg ggg gga ccg tca gtc ttc ctc ttc ccc cca aaa ccc      192
   Pro Glu Leu Leu Gly Gly Pro Ser Val Phe Leu Phe Pro Pro Lys Pro
       50                  55                  60
   aag gac acc ctc atg atc tcc cgg acc cct gag gtc aca tgc gtg gtg      240
   Lys Asp Thr Leu Met Ile Ser Arg Thr Pro Glu Val Thr Cys Val Val
   65                  70                  75                  80
   gtg gac gtg agc cac gaa gac cct gag gtc aag ttc aac tgg tac gtg      288
   Val Asp Val Ser His Glu Asp Pro Glu Val Lys Phe Asn Trp Tyr Val
                   85                  90                  95
   gac ggc gtg gag gtg cat aat gcc aag aca aag ccg cgg gag gag cag 336
   Asp Gly Val Glu Val His Asn Ala Lys Thr Lys Pro Arg Glu Glu Gln
   100 105 110
   tac aac agc acg tac cgt gtg gtc agc gtc ctc acc gtc ctg cac cag 384
   Tyr Asn Ser Thr Tyr Arg Val Val Ser Val Leu Thr Val Leu His Gln
   115 120 125
   gac tgg ctg aat ggc aag gag tac aag tgc aag gtc tcc aac aaa gcc 432
   Asp Trp Leu Asn Gly Lys Glu Tyr Lys Cys Lys Val Ser Asn Lys Ala
   130 135 140
   ctc cca gcc ccc atc gag aaa acc atc tcc aaa gcc aaa ggg cag ccc 480
   Leu Pro Ala Pro Ile Glu Lys Thr Ile Ser Lys Ala Lys Gly Gln Pro
   145 150 155 160
   cga gaa cca cag gtg tac acc ctg ccc cca tcc cgg gag gag atg acc 528
   Arg Glu Pro Gln Val Tyr Thr Leu Pro Pro Ser Arg Glu Glu Met Thr
   165 170 175
   aag aac cag gtc agc ctg acc tgc ctg gtc aaa ggc ttc tat ccc agc 576
   Lys Asn Gln Val Ser Leu Thr Cys Leu Val Lys Gly Phe Tyr Pro Ser
   180 185 190
   gac atc gcc gtg gag tgg gag agc aat ggg cag ccg gag aac aac tac 624
   Asp Ile Ala Val Glu Trp Glu Ser Asn Gly Gln Pro Glu Asn Asn Tyr
   195 200 205
   aag acc acg cct ccc gtg ctg gac tcc gac ggc tcc ttc ttc ctc tac 672
   Lys Thr Thr Pro Pro Val Leu Asp Ser Asp Gly Ser Phe Phe Leu Tyr
   210 215 220
   agc aag ctc acc gtg gac aag agc agg tgg cag cag ggg aac gtc ttc 720
   Ser Lys Leu Thr Val Asp Lys Ser Arg Trp Gln Gln Gly Asn Val Phe
   225 230 235 240
   tca tgc tcc gtg atg cat gag ggt ctg cac aac cac tac acg cag aag 768
   Ser Cys Ser Val Met His Glu Gly Leu His Asn His Tyr Thr Gln Lys
   245 250 255
   agc ctc tcc ctg tct ccg ggt aaa tga 795
   Ser Leu Ser Leu Ser Pro Gly Lys
   260
<210> 51
   <211> 264
   <212> PRT
   <213> Artificial
<220>
   <223> Synthetic Construct
<400> 51
   Met Tyr Arg Met Gln Leu Leu Ser Cys Ile Ala Leu Ser Leu Ala Leu
   1 5 10 15
   Val Thr Asn Ser Ile Tyr Cys Trp Ser Gln Tyr Leu Cys Tyr Gly Gly
   20 25 30
   Gly Gly Ser Gly Ser Asp Lys Thr His Thr Cys Pro Pro Cys Pro Ala
   35 40 45
   Pro Glu Leu Leu Gly Gly Pro Ser Val Phe Leu Phe Pro Pro Lys Pro
   50 55 60
   Lys Asp Thr Leu Met Ile Ser Arg Thr Pro Glu Val Thr Cys Val Val
   65 70 75 80
   Val Asp Val Ser His Glu Asp Pro Glu Val Lys Phe Asn Trp Tyr Val
   85 90 95
   Asp Gly Val Glu Val His Asn Ala Lys Thr Lys Pro Arg Glu Glu Gln
   100 105 110
   Tyr Asn Ser Thr Tyr Arg Val Val Ser Val Leu Thr Val Leu His Gln
   115 120 125
   Asp Trp Leu Asn Gly Lys Glu Tyr Lys Cys Lys Val Ser Asn Lys Ala
   130 135 140
   Leu Pro Ala Pro Ile Glu Lys Thr Ile Ser Lys Ala Lys Gly Gln Pro
   145 150 155 160
   Arg Glu Pro Gln Val Tyr Thr Leu Pro Pro Ser Arg Glu Glu Met Thr
   165 170 175
   Lys Asn Gln Val Ser Leu Thr Cys Leu Val Lys Gly Phe Tyr Pro Ser
   180 185 190
   Asp Ile Ala Val Glu Trp Glu Ser Asn Gly Gln Pro Glu Asn Asn Tyr
   195 200 205
   Lys Thr Thr Pro Pro Val Leu Asp Ser Asp Gly Ser Phe Phe Leu Tyr
   210 215 220
   Ser Lys Leu Thr Val Asp Lys Ser Arg Trp Gln Gln Gly Asn Val Phe
   225 230 235 240
   Ser Cys Ser Val Met His Glu Gly Leu His Asn His Tyr Thr Gln Lys
   245 250 255
   Ser Leu Ser Leu Ser Pro Gly Lys
   260
<210> 52
   <211> 795
   <212> DNA
   <213> Artificial
<220>
   <223> synthetic
<220>
   <221> CDS
   <222> (1) .. (795)
<400> 52
   atg tac agg atg caa ctc ctg tct tgc att gca cta agt ctt gca ctt      48
   Met Tyr Arg Met Gln Leu Leu Ser Cys Ile Ala Leu Ser Leu Ala Leu
   1               5                   10                  15
   gtc acg aat tcg atc tac tgc gct gca gct gca gct tgc tac ggt gga      96
   Val Thr Asn Ser Ile Tyr Cys Ala Ala Ala Ala Ala Cys Tyr Gly Gly
               20                  25                  30
   ggt ggt agc gga tct gac aaa act cac aca tgc cca ccg tgc cca gca      144
   Gly Gly Ser Gly Ser Asp Lys Thr His Thr Cys Pro Pro Cys Pro Ala
           35                  40                  45
   cct gaa ctc ctg ggg gga ccg tca gtc ttc ctc ttc ccc cca aaa ccc      192
   Pro Glu Leu Leu Gly Gly Pro Ser Val Phe Leu Phe Pro Pro Lys Pro
       50                  55                  60
   aag gac acc ctc atg atc tcc cgg acc cct gag gtc aca tgc gtg gtg      240
   Lys Asp Thr Leu Met Ile Ser Arg Thr Pro Glu Val Thr Cys Val Val
   65                  70                  75                  80
   gtg gac gtg agc cac gaa gac cct gag gtc aag ttc aac tgg tac gtg      288
   Val Asp Val Ser His Glu Asp Pro Glu Val Lys Phe Asn Trp Tyr Val
                   85                  90                  95
   gac ggc gtg gag gtg cat aat gcc aag aca aag ccg cgg gag gag cag      336
   Asp Gly Val Glu Val His Asn Ala Lys Thr Lys Pro Arg Glu Glu Gln
               100                 105                 110
   tac aac agc acg tac cgt gtg gtc agc gtc ctc acc gtc ctg cac cag      384
   Tyr Asn Ser Thr Tyr Arg Val Val Ser Val Leu Thr Val Leu His Gln
   115 120 125
   gac tgg ctg aat ggc aag gag tac aag tgc aag gtc tcc aac aaa gcc 432
   Asp Trp Leu Asn Gly Lys Glu Tyr Lys Cys Lys Val Ser Asn Lys Ala
   130 135 140
   ctc cca gcc ccc atc gag aaa acc atc tcc aaa gcc aaa ggg cag ccc 480
   Leu Pro Ala Pro Ile Glu Lys Thr Ile Ser Lys Ala Lys Gly Gln Pro
   145 150 155 160
   cga gaa cca cag gtg tac acc ctg ccc cca tcc cgg gag gag atg acc 528
   Arg Glu Pro Gln Val Tyr Thr Leu Pro Pro Ser Arg Glu Glu Met Thr
   165 170 175
   aag aac cag gtc agc ctg acc tgc ctg gtc aaa ggc ttc tat ccc agc 576
   Lys Asn Gln Val Ser Leu Thr Cys Leu Val Lys Gly Phe Tyr Pro Ser
   180 185 190
   gac atc gcc gtg gag tgg gag agc aat ggg cag ccg gag aac aac tac 624
   Asp Ile Ala Val Glu Trp Glu Ser Asn Gly Gln Pro Glu Asn Asn Tyr
   195 200 205
   aag acc acg cct ccc gtg ctg gac tcc gac ggc tcc ttc ttc ctc tac 672
   Lys Thr Thr Pro Pro Val Leu Asp Ser Asp Gly Ser Phe Phe Leu Tyr
   210 215 220
   agc aag ctc acc gtg gac aag agc agg tgg cag cag ggg aac gtc ttc 720
   Ser Lys Leu Thr Val Asp Lys Ser Arg Trp Gln Gln Gly Asn Val Phe
   225 230 235 240
   tca tgc tcc gtg atg cat gag ggt ctg cac aac cac tac acg cag aag 768
   Ser Cys Ser Val Met His Glu Gly Leu His Asn His Tyr Thr Gln Lys
   245 250 255
   agc ctc tcc ctg tct ccg ggt aaa tga 795
   Ser Leu Ser Leu Ser Pro Gly Lys
   260
<210> 53
   <211> 264
   <212> PRT
   <213> Artificial
<220>
   <223> Synthetic Construct
<400> 53
   Met Tyr Arg Met Gln Leu Leu Ser Cys Ile Ala Leu Ser Leu Ala Leu
   1               5                   10                  15
   Val Thr Asn Ser Ile Tyr Cys Ala Ala Ala Ala Ala Cys Tyr Gly Gly
               20                  25                  30
   Gly Gly Ser Gly Ser Asp Lys Thr His Thr Cys Pro Pro Cys Pro Ala
           35                  40                  45
   Pro Glu Leu Leu Gly Gly Pro Ser Val Phe Leu Phe Pro Pro Lys Pro
       50                  55                  60
   Lys Asp Thr Leu Met Ile Ser Arg Thr Pro Glu Val Thr Cys Val Val
   65                  70                  75                  80
   Val Asp Val Ser His Glu Asp Pro Glu Val Lys Phe Asn Trp Tyr Val
                   85                  90                  95
   Asp Gly Val Glu Val His Asn Ala Lys Thr Lys Pro Arg Glu Glu Gln
               100                 105                 110
   Tyr Asn Ser Thr Tyr Arg Val Val Ser Val Leu Thr Val Leu His Gln
           115                 120                 125
   Asp Trp Leu Asn Gly Lys Glu Tyr Lys Cys Lys Val Ser Asn Lys Ala
       130                 135                 140
   Leu Pro Ala Pro Ile Glu Lys Thr Ile Ser Lys Ala Lys Gly Gln Pro
   145 150 155 160
   Arg Glu Pro Gln Val Tyr Thr Leu Pro Pro Ser Arg Glu Glu Met Thr
   165 170 175
   Lys Asn Gln Val Ser Leu Thr Cys Leu Val Lys Gly Phe Tyr Pro Ser
   180 185 190
   Asp Ile Ala Val Glu Trp Glu Ser Asn Gly Gln Pro Glu Asn Asn Tyr
   195 200 205
   Lys Thr Thr Pro Pro Val Leu Asp Ser Asp Gly Ser Phe Phe Leu Tyr
   210 215 220
   Ser Lys Leu Thr Val Asp Lys Ser Arg Trp Gln Gln Gly Asn Val Phe
   225 230 235 240
   Ser Cys Ser Val Met His Glu Gly Leu His Asn His Tyr Thr Gln Lys
   245 250 255
   Ser Leu Ser Leu Ser Pro Gly Lys
   260
<210> 54
   <211> 723
   <212> DNA
   <213> Artificial
<220>
   <223> synthetic
<220>
   <221> CDS
   <222> (1)..(723)
<400> 54
   atg tcc cct ata cta ggt tat tgg aaa att aag ggc ctt gtg caa ccc      48
   Met Ser Pro Ile Leu Gly Tyr Trp Lys Ile Lys Gly Leu Val Gln Pro
   1               5                   10                  15
   act cga ctt ctt ttg gaa tat ctt gaa gaa aaa tat gaa gag cat ttg      96
   Thr Arg Leu Leu Leu Glu Tyr Leu Glu Glu Lys Tyr Glu Glu His Leu
               20                  25                  30
   tat gag cgc gat gaa ggt gat aaa tgg cga aac aaa aag ttt gaa ttg     144
   Tyr Glu Arg Asp Glu Gly Asp Lys Trp Arg Asn Lys Lys Phe Glu Leu
           35                  40                  45
   ggt ttg gag ttt ccc aat ctt cct tat tat att gat ggt gat gtt aaa     192
   Gly Leu Glu Phe Pro Asn Leu Pro Tyr Tyr Ile Asp Gly Asp Val Lys
       50                  55                  60
   tta aca cag tct atg gcc atc ata cgt tat ata gct gac aag cac aac     240
   Leu Thr Gln Ser Met Ala Ile Ile Arg Tyr Ile Ala Asp Lys His Asn
   65                  70                  75                  80
   atg ttg ggt ggt tgt cca aaa gag cgt gca gag att tca atg ctt gaa     288
   Met Leu Gly Gly Cys Pro Lys Glu Arg Ala Glu Ile Ser Met Leu Glu
                   85                  90                  95
   gga gcg gtt ttg gat att aga tac ggt gtt tcg aga att gca tat agt     336
   Gly Ala Val Leu Asp Ile Arg Tyr Gly Val Ser Arg Ile Ala Tyr Ser
               100                 105                 110
   aaa gac ttt gaa act ctc aaa gtt gat ttt ctt agc aag cta cct gaa     384
   Lys Asp Phe Glu Thr Leu Lys Val Asp Phe Leu Ser Lys Leu Pro Glu
           115                 120                 125
   atg ctg aaa atg ttc gaa gat cgt tta tgt cat aaa aca tat tta aat     432
   Met Leu Lys Met Phe Glu Asp Arg Leu Cys His Lys Thr Tyr Leu Asn
       130                 135                 140
   ggt gat cat gta acc cat cct gac ttc atg ttg tat gac gct ctt gat     480
   Gly Asp His Val Thr His Pro Asp Phe Met Leu Tyr Asp Ala Leu Asp
   145 150 155 160
   gtt gtt tta tac atg gac cca atg tgc ctg gat gcg ttc cca aaa tta 528
   Val Val Leu Tyr Met Asp Pro Met Cys Leu Asp Ala Phe Pro Lys Leu
   165 170 175
   gtt tgt ttt aaa aaa cgt att gaa gct atc cca caa att gat aag tac 576
   Val Cys Phe Lys Lys Arg Ile Glu Ala Ile Pro Gln Ile Asp Lys Tyr
   180 185 190
   ttg aaa tcc agc aag tat ata gca tgg cct ttg cag ggc tgg caa gcc 624
   Leu Lys Ser Ser Lys Tyr Ile Ala Trp Pro Leu Gln Gly Trp Gln Ala
   195 200 205
   acg ttt ggt ggt ggc gac cat cct cca aaa tcg gat ctg gtt ccg cgt 672
   Thr Phe Gly Gly Gly Asp His Pro Pro Lys Ser Asp Leu Val Pro Arg
   210 215 220
   gga tcc ggt gga ggt ggt agc tac tgc tgg agc cag tac ctg tgc tac 720
   Gly Ser Gly Gly Gly Gly Ser Tyr Cys Trp Ser Gln Tyr Leu Cys Tyr
   225 230 235 240
   tga 723
<210> 55
   <211> 240
   <212> PRT
   <213> Artificial
<220>
   <223> Synthetic Construct
<400> 55
   Met Ser Pro Ile Leu Gly Tyr Trp Lys Ile Lys Gly Leu Val Gln Pro
   1 5 10 15
   Thr Arg Leu Leu Leu Glu Tyr Leu Glu Glu Lys Tyr Glu Glu His Leu
   20 25 30
   Tyr Glu Arg Asp Glu Gly Asp Lys Trp Arg Asn Lys Lys Phe Glu Leu
   35 40 45
   Gly Leu Glu Phe Pro Asn Leu Pro Tyr Tyr Ile Asp Gly Asp Val Lys
   50 55 60
   Leu Thr Gln Ser Met Ala Ile Ile Arg Tyr Ile Ala Asp Lys His Asn
   65 70 75 80
   Met Leu Gly Gly Cys Pro Lys Glu Arg Ala Glu Ile Ser Met Leu Glu
   85 90 95
   Gly Ala Val Leu Asp Ile Arg Tyr Gly Val Ser Arg Ile Ala Tyr Ser
   100 105 110
   Lys Asp Phe Glu Thr Leu Lys Val Asp Phe Leu Ser Lys Leu Pro Glu
   115 120 125
   Met Leu Lys Met Phe Glu Asp Arg Leu Cys His Lys Thr Tyr Leu Asn
   130 135 140
   Gly Asp His Val Thr His Pro Asp Phe Met Leu Tyr Asp Ala Leu Asp
   145 150 155 160
   Val Val Leu Tyr Met Asp Pro Met Cys Leu Asp Ala Phe Pro Lys Leu
   165 170 175
   Val Cys Phe Lys Lys Arg Ile Glu Ala Ile Pro Gln Ile Asp Lys Tyr
   180 185 190
   Leu Lys Ser Ser Lys Tyr Ile Ala Trp Pro Leu Gln Gly Trp Gln Ala
   195 200 205
   Thr Phe Gly Gly Gly Asp His Pro Pro Lys Ser Asp Leu Val Pro Arg
   210 215 220
   Gly Ser Gly Gly Gly Gly Ser Tyr Cys Trp Ser Gln Tyr Leu Cys Tyr
   225 230 235 240

## Description

### Technical Field

The present invention relates to means for enhancing osteogenesis by administering an effective dose of a molecule that can act on osteoblasts or cells capable of differentiating into osteoblasts, such as osteoblast precursor cells, mesenchymal stem cells, stromal cells, and myoblasts, so as to enhance the differentiation and maturation of such cells.

A pharmaceutical composition that stimulates osteogenesis, is also disclosed.

A method of screening for a substance that acts on RANKL for signal transmission, a substance obtained by such screening method, and a pharmaceutical composition comprising the obtained substance is also disclosed.

### Background Art

Bones are active organs that continuously undergo bone remodeling via repetition of formation and resorption/destruction in bone morphogenesis and maintenance of the serum calcium concentration. In general, osteogenesis caused by osteoblasts and bone resorption caused by osteoclasts are in equilibrium. The bone mass can be maintained at a constant level by the mechanism of mutual response between such cells (see Non-Patent Document 1). When the equilibrium state is disturbed by menopause, aging, inflammation, or the like, a metabolic bone disease involving destruction due to osteoporosis or rheumatoid arthritis is induced. The development of such metabolic bone disease is a serious problem in the current aging society. Therefore, molecular-level elucidation of the pathogenic mechanism of such disease and the development of effective therapeutic agents are urgent tasks.

It is assumed that there are 10,000,000 or more potential osteoporosis patients in Japan. Examples of osteopenia such as osteoporosis include juvenile osteoporosis, dysosteogenesis, hypercalcemia, hyperparathyroidism, osteomalacia, osteohalisteresis, osteolytic bone diseases, osteonecrosis, the Paget's disease, rheumatoid arthritis, bone mass reduction due to osteoarthritis, inflammatory arthritis, osteomyelitis, glucocorticoid treatment, metastatic bone diseases, periodontal bone loss, bone loss due to cancer, bone loss due to aging, and other osteopenia-related diseases.

Hitherto, bone resorption inhibitors that inhibit the process of bone resorption rather than enhance osteogenesis have been used as therapeutic agents for bone metabolism diseases exhibiting bone loss, such as osteoporosis. Examples of agents capable of inhibiting bone resorption that are used or suggested for treatment of osteoporosis include estrogen, a selective estrogen receptor modulator (SERM), ipriflavone, vitamin K2, calcium, calcitriol, calcitonin (see Non-Patent Document 2), and a bisphosphonate such as alendronate (see Non-Patent Document 3). However, therapeutic methods using the above agents are not always satisfactory in terms of therapeutic effects and results. Therefore, the further development of safe and effective novel therapeutic agents has been awaited. In particular, there is a concern that bone resorption inhibitors, which are represented by the above bisphosphonate, might cause excessive bone resorption inhibition, which would result in adverse effects in vivo. In addition, the risk of developing the iatrogenic marble bone disease (osteopetrosis) has been suggested (see Non-Patent Document 4). Therefore, it is necessary to use the above agents carefully, especially in the cases of young patients. It has been reported that the healing of bone fractures might be delayed, particularly in cases in which bisphosphonate causes a large decrease in the bone metabolic turnover rate (see Non-Patent Document 5).

Meanwhile, parathyroid hormone (PTH), which can serve as a bone anabolic factor, is under clinical development in Japan. In addition to that, BMP2, BMP7, IGF1, FGF2, and the like are known to have bone anabolic activities. However, the number of cases in which such agents is practically applied for use as bone anabolic factors is limited. For example, such cases have been reported in the U.S. and other countries, but the cases merely involve the clinical use of PTH for osteoporosis, BMP2 and BMP7 for spondylolisthesis and the like, and IGF1 for children with short stature due to severe primary IGF1 deficiency. As described above, there have been very few cases of the applied use of bone anabolic factors. This is because the mechanisms of differentiation and maturation of osteoblasts that cause osteogenesis have not been elucidated.

Osteoclasts, which control osteolysis, are large multinucleated cells derived from hematopoietic cells that differentiate into monocytes/macrophages. Differentiation and maturation of osteoclast precursor cells into osteoclasts are controlled by osteoblasts/stromal cells on the bone surface (see Non-Patent Document 1). An osteoclast differentiation factor (RANKL; receptor activator of NF-κB ligand) is a membrane-bound protein belonging to the family of tumor necrosis factors (TNFs) induced by bone resorption factors onto osteoblasts/stromal cells, and RANKL is essential for osteoclast differentiation/maturation (see Non-Patent Documents 6 and 7) As a result of studies focusing on RANKL/RANK/OPG, including RANK (receptor activator of NF-κB) serving as an RANKL receptor and OPG (osteoprotegerin) serving as a decoy receptor, elucidation of the osteoclast differentiation and maturation control mechanism has been done at the biological level. In addition, involvement of the three factors in bone metabolism diseases is being clarified (see Non-Patent Document 8).

Bone formation and bone resorption are in equilibrium, which is maintained by an existing mechanism that finely controls bone formation to an extent corresponding to the degree of bone resorption. Such conjugation of bone resorption and formation is called "coupling" (see Non-Patent Document 9). RANKL, which is an osteoclast differentiation factor, is produced on osteoblasts in response to stimulation by bone resorption factors. RANKL binds to RANK serving as an RANKL receptor, which is located on osteoclast precursor cells and osteoclasts, resulting in transmission of differentiation and activation signals. It has been reported that an artificial peptide having a conformation similar to that of the binding region of TNF was used for inhibition of signal transmission from RANKL to RANK based on the above mechanism (see Non-Patent Documents 10 to 12).

Meanwhile, the mechanism of transmission of bone formation signals to osteoblasts in response to transmission of bone resorption signals has not been elucidated.

WO 01/08677 A1 discloses methods of inhibiting osteoclast activity or osteoclastogenesis in order to treat diseases characterized by bone loss. A peptide denoted WP9QY, which is identical to SEQ ID NO:16, is disclosed to inhibit osteoclastogenesis.

Kostenuik PJ (2005) Current Opinion in Pharmacology 5:618-625 discloses that osteoprotegerin (OPG) and receptor activator of nuclear factor-κB ligand (RANKL) are dominant regulators of bone resorption. RANKL stimulates osteoclast formation, function and survival, and each of these effects is inhibited by OPG. OPG and denosumab (AMG 162), and anti-RANKL monoclonal antibody suppress bone resorption and increase the density, area and strength of both cancellous and cortical bone.

McIntyre JA and Martin L (2005) Drugs of the Future 30(3):237-239 discloses that AMG-162 is in phase III development for the treatment of osteoporosis in postmenopausal women, as well as phase II testing for its potential in metastatic bone disease and rheumatoid arthritis.

WO 01/83525 A2 relates to fusions of Fc domains with biologically active peptides. It discloses certain TNF-antagonist peptide sequences that are identical to SEQ ID NOs:7 and 16 and their use for the treatment of numerous diseases.
Non-Patent Document 1: Suda et al., Endocr Rev, 13: 66, 1992
Non-Patent Document 2: Sambook et al., N Engl J Med 328: 1747, 1993
Non-Patent Document 3: Luckman et al., J Bone Miner Res 13: 581, 1998
Non-Patent Document 4: Whyte et al., N Engl J Med 349: 457, 2003
Non-Patent Document 5: Odvina et al., J Clin Endocrinol Metab 90: 1294, 2005
Non-Patent Document 6: Yasuda et al., Proc Natl Acad Sci USA 95: 3597, 1998
Non-Patent Document 7: Lacey et al., Cell 93: 165, 1998
Non-Patent Document 8: Suda et al., Endocr Rev, 20: 345, 1999
Non-Patent Document 9: Martin et al., Trends Mol Med, 11: 76, 2005
Non-Patent Document 10: Aoki et al., J Clin Invest 116: 1525, 2006
Non-Patent Document 11: Takasaki et al., Nat Biotec, 15: 1266, 1997
Non-Patent Document 12: Cheng et al., J Biol Chem, 279; 8269, 2004

### Disclosure of the Invention

It is an object of the present invention to provide means for enhancing osteogenesis via administration of an effective dose of an RANKL-binding molecule that enhances differentiation, maturation, or calcification of osteoblasts or cells capable of differentiating into osteoblasts.

The present inventors have found that transmission of reverse signals from RANK, which is an RANKL receptor, to RANKL, which is an RANK ligand, takes place, in addition to transmission of forward signals from RANKL to RANK. Also, the present inventors have found that the bidirectional signals transmitted between RANKL and RANK control coupling of bone resorption and formation. It is thought that reverse signals from membrane-bound RANK located on osteoclasts to membrane-bound RANKL located on osteoblasts control the coupling of bone resorption and bone formation in physiological bone metabolism. The use of such reverse signals allows the development of an agent that can increase bone mass. Specifically, enhancement of osteoblast differentiation and maturation is caused by reverse signals that are transmitted when RANKL-binding molecules such as membrane-bound RANK, an RANK analog peptide, an anti-RANKL antibody, soluble RANK, OPG, and variants and analogs thereof bind to membrane-bound RANKL, resulting in an increase in bone mass.

As described above, the present inventors have found that differentiation, maturation, and calcification of osteoblasts are induced *in vitro* by allowing a variety of proteins, peptides, and the like, which can be used as molecules that act on RANKL, to act on RANKL located on osteoblasts or cells capable of differentiating into osteoblasts. Further, the present inventors have found that a variety of proteins, peptides, and the like, which can be used as molecules that act on RANKL, can cause an increase in the bone density and the like in a mouse when administered *in vivo* to the mouse and thus can be used for treatment and prevention of bone metabolism diseases associated with osteopenia. This has led to the completion of the present invention.

Specifically, the present invention provides a compound for use in the treatment or prevention of bone metabolic diseases associated with osteopenia by inducing osteogenesis, as defined in the claims.

### Brief Description of the Drawings

Fig. 1 is a graph of increases in the ALP activity in human mesenchymal stem cells treated with peptide D.
Fig. 2 is a staining image showing increases in the ALP activity in human mesenchymal stem cells treated with peptide D.
Fig. 3 is an image showing a calcification of human mesenchymal stem cells treated with peptide D.
Fig. 4 is a graph of increases in the ALP activity in MC3T3-E1 cells (of a mouse osteoblast precursor cell line) treated with peptide D.
Fig. 5 is an image showing a calcification of MC3T3-E1 cells treated with peptide D.
Fig. 6 is a graph of increases in the ALP activity in mouse osteoblasts treated with peptide D.
Fig. 7 is a graph of increases in the ALP activity in mouse osteoblasts treated with an anti-RANKL polyclonal antibody.
Fig. 8 is a graph of increases in the ALP activity in mouse osteoblasts treated with an anti-RANKL polyclonal antibody and anti-RANKL monoclonal antibodies.
Fig. 9 is a graph of increases in the ALP activity in human osteoblasts treated with peptide D, an anti-RANKL polyclonal antibody, and anti-RANKL monoclonal antibodies.
Fig. 10 is a graph of increases in the ALP activity in human osteoblasts treated with peptide D, OPGFc, RANKFc, and anti-RANKL monoclonal antibodies.
Fig. 11 is a graph of increases in the ALP activity in C2C12 cells (of a mouse myoblast cell line) treated with an anti-RANKL polyclonal antibody.
Figs. 12A to 12C show increases in the ALP and type I collagen gene expression levels in human mesenchymal stem cells treated with peptide D. Fig. 12A shows results in electrophoresis. Figs. 12B and C show results normalized by the GAPDH expression level.
Fig. 13 is a graph of increases in the ALP activity in C2C12 expressing membrane-bound RANK.
Fig. 14A and 14B are graphs showing increases in the ALP activity in ST2 expressing membrane-bound RANK. Figs. 14A and 14B show results for culture in a maintenance medium and in the presence of Dexamethasone (10⁻⁷ M) and activated Vitamin D₃ (10⁻⁸M), respectively.
Fig. 15 is a graph of unit bone masses of cervical bone for mice treated with or without RANKL.
Fig. 16 is a graph of cervical osteoclast numbers for mice treated with or without RANKL.
Fig. 17 is a graph of cervical trabecular numbers for mice treated with or without RANKL.
Fig. 18 shows bone morphology images (obtained with µCT) of femurs of mice treated with or without RANKL.
Fig. 19 is a graph of cervical osteoblast surface areas for mice treated with or without RANKL.
Fig. 20A is a graph of femur bone mineral contents for mice treated with peptide D.
Fig. 20B is a graph of femur bone surface areas for mice treated with peptide D.
Fig. 20C is a graph of femur bone densities for mice treated with peptide D.
Fig. 21 is a graph of bone densities (in individual femur regions) for mice treated with peptide D.
Fig. 22A is a graph of bone densities (in regions 5 mm proximal to the distal femoral end) for mice treated with peptide D.
Fig. 22B is a graph of cortical bone masses (in regions 5 mm proximal to the distal femoral end) for mice treated with peptide D.
Fig. 23 shows images depicting results of three-dimensional structural analysis (with µCT) in regions 2 mm proximal to the distal femoral end for mice treated with peptide D.
Fig. 24A is a graph of BV/TV in µCT analysis in regions 2 mm proximal to the distal femoral end for mice treated with peptide D.
Fig. 24B is a graph of trabecular widths in µCT analysis in regions 2 mm proximal to the distal femoral end for mice treated with peptide D.
Fig. 24C is a graph of trabecular numbers in µCT analysis in regions 2 mm proximal to the distal femoral end for mice treated with peptide D.
Fig. 25 shows graphs of calcification rates (A) and proliferation rates (B) for mice treated with peptide D.
Fig. 26 shows images of p38 phosphorylation obtained 12 hours after the stimulation with peptide D.
Fig. 27 shows images of p38 phosphorylation obtained in a short period of time after the stimulation with peptide D.
Fig. 28 shows an image of GSK3β phosphorylation by peptide D.
Fig. 29 shows images of Smad phosphorylation by peptide D.
Fig. 30 is a graph showing inhibition of increase in the ALP activity by peptide D with the use of SB203580.
Fig. 31 is a graph showing inhibition of increase in the ALP activity by peptide D with the use of Dkk-1.
Fig. 32 is a graph showing inhibition of increase in the ALP activity by peptide D with the use of BMPR-IA.
Fig. 33 is a graph showing the synergistic effect on increase in ALP activity in C2C12 cells treated with peptide D and BMP-2..
Fig. 34 is a graph showing the synergistic effect on increase in ALP activity in MC3T3-E1 cells treated with peptide D and BMP-2.
Fig. 35 shows an image of promotion of RANKL expression in C2C12 cells treated with BMP-2.
Fig. 36 is a graph showing the inhibition of TRAP activity by peptides D and E in RAW264 cells.
Fig. 37 shows increases in the ALP activity by peptides D and E in MC3T3-E1 cells.
Fig. 38 shows the inhibition of increase in ALP activity by peptide D in RANKL knock-down MC3T3-E1 cells.
Fig. 39A is a graph showing increases in the ALP activity by different peptide D salt substitutes.
Fig. 39B is the dose-dependent increase in the ALP activity by peptide D in the form of acetate.
Fig. 39C is a graph showing increases in the ALP activity by a combination of peptide D and BMP-4.
Fig. 40 shows graphs of increases in the ALP activity by RANKL antibodies and by combinations of RANKL antibodies and BMP-2 in C2C12 cells.
Fig. 41 shows graphs of increases in the ALP activity by RANKL antibodies in mouse osteoblasts.
Fig. 42 shows a graph of increases in the ALP activity by combinations of RANKL antibodies and BMP-2 in mouse osteoblasts.
Fig. 43 is a graph showing influence of GST-RANKL on the ALP activity by a combination of peptide D and BMP-2 in MC3T3-E1 cells.
Fig. 44 is a graph showing the proliferative response of mouse osteoblasts treated with peptide D and RANKL antibodies.
Fig. 45 is a graph showing changes in gene expression in MC3T3-E1 cells treated by peptide D (12 hours later).
Fig. 46 is a graph showing changes in gene expression in MC3T3-E1 cells treated by peptide D (96 hours later).
Fig. 47A shows an electrophoresis image showing changes in gene expression (ALP, Col1, and OC) in MC3T3-E1 cells treated by peptide D and BMP-2.
Fig. 47B is a graph showing changes in gene expression (ALP, Col1, and OC) in MC3T3-E1 cells treated by peptide D and BMP-2.
Fig. 48 is a graph showing increases in bone formation markers by peptide D.
Fig. 49 is a graph showing bone anabolic activities of peptide D in gene expression.
Fig. 50A shows an image of expression in differention factors and receptors by peptide D.
Fig. 50B is a graph of expression in differention factors and receptors by peptide D.
Fig. 51 is a graph showing an increase in the ALP activity by Fc fusion peptide D.
Fig. 52 is a graph showing influence of peptide D salt substitutes on the activity of osteoclastogenesis.
Fig. 53 is a graph of neutralization abilities of different RANKL antibodies with respect to RANKL-induced osteoclastogenesis activity.
Fig. 54 is a graph of neutralization abilities of anti-human RANKL monoclonal antibodies with respect to RANKL-induced osteoclastogenesis activity.
Fig. 55 is a graph showing an increase in the ALP activity byGST fusion peptide D.
Fig. 56 is a graph showing increases in the ALP activity by anti-human RANKL monoclonal antibodies.

### Best Mode for Carrying Out the Invention

Hereinafter, the present invention is described in detail.

RANKL (receptor activator of NF-κB ligand) is a ligand for RANK (receptor activator of NF-κB), which is a TNF receptor superfamily member, and a type 2 transmembrane protein having an intracellular domain (a domain corresponding to the N-terminal 48 amino acids (residues 1 to 48), a transmembrane domain, and an extracellular domain). (JP Patent Publication (Kohyo) No. 2002-509430 A and WO98/46644 (currently JP Patent No. 3523650)). RANKL is expressed on osteoblasts or cells capable of differentiating into osteoblasts in response to stimulation by bone resorption factors. Herein, cells capable of differentiating into osteoblasts include any types of cells, as long as such cells can differentiate into osteoblasts. Examples thereof include osteoblast precursor cells, mesenchymal stem cells, stromal cells, and myoblasts. In the extracellular domain, the domain corresponding to amino acid residues 153 to 317 is a TNF ligand family homologous domain. The full-length nucleotide sequence and the amino acid sequence of human-derived RANKL are shown in SEQ ID NOS: 1 and 2, respectively. The full-length nucleotide sequence and the amino acid sequence of RANK are shown in SEQ ID NOS: 3 and 4, respectively.

OPG (osteoprotegerin) is a protein structurally similar to RANK, and it binds to RANKL. The full length nucleotide sequence and the amino acid sequence of OPG are shown in SEQ ID NOS: 5 and 6, respectively.

The present invention relates to a compound for use as defined in the claims. The compound acts on osteoblasts or cells capable of differentiating into osteoblasts and promotes differentiation, proliferation, maturation, or calcification of osteoblasts or cells capable of differentiating into osteoblasts so as to induce osteogenesis, thereby causing bone mass enhancement and the like. Such a compound

transmits signals to osteoblasts or cells capable of differentiating into osteoblasts and promotes differentiation, proliferation, maturation, or calcification of osteoblasts or cells capable of differentiating into osteoblasts so as to induce osteogenesis, thereby causing bone mass enhancement and the like. An example thereof is a compound as defined in the claim that acts on RANKL to cause RANKL to transmit signals to osteoblasts or cells capable of differentiating into osteoblasts and promotes differentiation, proliferation, maturation, or calcification of osteoblasts or cells capable of differentiating into osteoblasts so as to induce osteogenesis, thereby causing bone mass enhancement and the like. When the compound acts on RANKL, the animal species from which a target RANKL is derived is not limited. Examples of a target RANKL include RANKLs from various types of animal species such as human RANKL, mouse RANKL, and rat RANKL. Herein, the expression "acts on RANKL" means that such compound acts on RANKL to cause RANKL to transmit signals to osteoblasts or cells capable of differentiating into osteoblasts. For instance, the compound may bind to RANKL to cause RANKL to transmit signals to osteoblasts or cells capable of differentiating into osteoblasts.

Exampels of a compound that acts on RANKL and promotes differentiation, proliferation, maturation, or calcification of osteoblasts or cells capable of differentiating into osteoblasts so as to induce osteogenesis, thereby causing bone mass enhancement and the like, include various compounds capable of acting on RANKL which are derived from various animal species. Such compounds include natural and non-natural peptides and chemically synthesized and microorganism-derived low-molecular compounds.

Examples of compounds that promote differentiation, proliferation, maturation, or calcification of osteoblasts or cells capable of differentiating into osteoblasts so as to induce osteogenesis, thereby causing bone mass enhancement and the like, of the present invention include a variant of fragment peptide of RANK, a peptide structurally similar to RANK, a peptide structurally similar to to a fragment peptide of RANK, a chemical substance structurally similar to RANK, and a chemical substance structurally similar to a fragment peptide of RANK. Examples of such compound include RANK; a variant of fragment peptide of RANK capable of acting on RANKL, a peptide structurally similar to RANK and capable of acting on RANKL, a peptide structurally similar to a fragment peptide of RANK and capable of acting on RANKL, a chemical substance structurally similar to RANK and capable of acting on RANKL, and a chemical substance structurally similar to a fragment peptide of RANK and capable of acting on RANKL.

In addition, the term "chemical substance" used herein refers to a non-peptide or non-protein compound. The term "RANK" refers to a membrane-bound RANK and a soluble RANK. The term "membrane-bound RANK" refers to RANK bound to the surface of a cell, which has a transmembrane region. Examples of a cell include animal cells such as cells on which natural RANK has been expressed and human and manmalian cells on which recombinant RANK has been expressed. Also, the term "RANK" refers to RANKFc. Herein, RANKFc is a fusion protein obtained by allowing the Fc region of human IgG₁ to bind to the extracellular region of human RANK.

Further, in the present disclosure the expression "structurally similar to" refers to a situation in which the resultant compound has a portion capable of acting on RANKL that is structurally similar to RANK. In the cases of peptides and proteins, such portion can be similar to RANK in terms of a primary structure that is generally represented by an amino acid sequence. However, in the present disclosure, a compound similar to RANK in terms of the conformation but not the amino acid sequence and capable of acting on RANKL can also be used.

Furthermore, examples of a compound that promotes differentiation, proliferation, maturation, or calcification of osteoblasts or cells capable of differentiating into osteoblasts so as to induce bone formation, thereby causing bone mass enhancement and the like, include OPG, a variant or fragment peptide of OPG, a peptide structurally similar to OPG, a peptide structurally similar to a fragment peptide of OPG, a chemical substance structurally similar to OPG, and a chemical substance structurally similar to a fragment peptide of OPG. For example, such compound may be a compound that acts on RANKL and promotes differentiation, proliferation, maturation, or calcification of osteoblasts or cells capable of differentiating into osteoblasts so as to induce osteogenesis, thereby causing bone mass enhancement and the like. Examples thereof include OPG, a variant or fragment peptide of OPG capable of acting on RANKL, a peptide structurally similar to OPG and capable of acting on RANKL, a peptide structurally similar to a fragment peptide of OPG and capable of acting on RANKL, a chemical substance structurally similar to OPG and capable of acting on RANKL, and a chemical substance structurally similar to a fragment peptide of OPG and capable of acting on RANKL.

In addition, the term "chemical substance" used herein refers to a non-peptide or non-protein compound. The term "OPG" refers to a membrane-bound OPG and a soluble OPG. The term "membrane-bound OPG" refers to OPG with a C-terminal region bound to the surface of a cell. Examples of such a cell include animal cells such as cells on which natural OPG has been expressed and human cells on which recombinant OPG has been expressed. Also, the term "OPG" refers to OPGFc. Herein, OPGFc is a fusion protein obtained by allowing the Fc region of human IgG₁ to bind to the extracellular region of human OPG (an Fc fusion protein).

An example of an RANK or OPG analog is a protein or peptide comprising an amino acid sequence derived from the amino acid sequence of RANK, OPG, or a fragment peptide of either thereof by deletion, substitution, or addition of one or more amino acids and having the RANK or OPG activity. Herein, the expression "one or more" means "1 to 9," preferably "1 to 5," and more preferably "1 or 2."

Examples of a peptide structurally similar to a portion of RANK that binds to RANKL include a peptide comprising the amino acid sequence represented by SEQ ID NO: 7 (peptide D) and a peptide comprising the amino acid sequence represented by SEQ ID NO: 16 (peptide E). These peptides are cyclic peptides in which Cys at position 2 is bound to Cys at position 8 via a disulfide bond.

Further, a peptide salt structurally similar to a portion of RANK that binds to RANKL can be used. Such peptide salt is not limited as long as it is a pharmacologically acceptable salt. Examples thereof include acid addition salts and base addition salts. Specific examples of acid addition salts include: salts comprising organic acids such as acetic acid, malic acid, succinic acid, trifluoroacetate (TFA), tartaric acid, and citric acid; and salts comprising inorganic acids such as hydrochloric acid, sulfuric acid, nitric acid, and phosphoric acid. In addition, specific examples of base addition salts include salts comprising alkali metals such as sodium and potassium, salts comprising alkaline earth metals such as calcium and magnesium, and salts comprising amines such as ammonium and triethylamine. Of these, acetate is preferable. In particular, a peptide in the form of acetate comprising the amino acid sequence represented by SEQ ID NO: 7 or SEQ ID NO: 16 is preferable.

Also, it is possible to use a fusion protein obtained by allowing GST (glutathione-S-transferase) or the Fc region of human IgG₁ (GST fusion protein or Fc fusion protein) to bind to the peptide structurally similar to a portion of RANK that binds to RANKL. An example of such fusion protein is a fusion protein obtained by allowing GST (glutathione-S-transferase) or the Fc region of human IgG₁ to bind to peptide D (GST fusion peptide D or Fc fusion peptide D). The above fusion proteins have improved degrees of *in vivo* stability and thus have prolonged serum half-lives. Also, a fusion protein comprising GST and an epitope tag other than the Fc region can be used. Examples of such epitope tag include polyhistidine comprising 2 to 12, preferably 4 or more, more preferably 4 to 7, and further preferably 5 or 6 histidines, FLAG tag, Myc tag, V5 tag, Xpress tag, HQ tag, HA tag, AU1 tag, T7 tag, VSV-G tag, DDDDK tag, S tag, CruzTag09, CruzTag22, CruzTag41, Glu-Glu tag, Ha.11 tag, KT3 tag, thioredoxin, maltose binding protein (MBP), and β-galactosidase.

In the present invention, a compound that acts on RANKL and promotes differentiation, proliferation, maturation, or calcification of osteoblasts or cells capable of differentiating into osteoblasts so as to induce osteogenesis, thereby causing bone mass enhancement and the like, may be referred to as an RANKL agonist substance.

Further, such compounds include anti-RANKL antibodies that act on RANKL to promote differentiation, proliferation, maturation, or calcification of osteoblasts or cells capable of differentiating into osteoblasts so as to induce osteogenesis, thereby causing bone mass enhancement and the like, and also include functional fragments thereof. In the present invention, such an antibody may be referred to as an RANKL agonist antibody. An anti-RANKL antibody can be obtained in the form of a polyclonal antibody or a monoclonal antibody by known methods. Preferably, it is a monoclonal antibody. Examples of a monoclonal-antibody include a monoclonal antibody produced by a hybridoma and a monoclonal antibody produced by a host that has been transformed by genetic engineering procedures with the use of an expression vector comprising the antibody gene. A monoclonal antibody-producing hybridoma can be produced by a known method as described below. Specifically, such hybridoma can be produced by carrying out immunization with the use of membrane-bound or soluble RANKL or a fragment peptide thereof as a sensitized antigen by a known immunization method, fusing the resulting immunized cell with a known parent cell by a general cell fusion method, and screening for a monoclonal-antibody-producing cell by a known screening method. Upon immunization with RANKL, RANKL may be allowed to bind to a carrier protein such as bovine serum albumin (BSA), keyhole lympet haemocyanin, or the like before use. A monoclonal antibody that can be used is a recombinant monoclonal antibody produced by cloning the antibody gene with the use of a hybridoma, incorporating the cloned gene into an appropriate vector, and introducing the vector into a host by the gene recombinant technique (e.g., see Vandamme, A. M. et al., Eur. J. Biochem. 1990; 192: 767-775). In such case, it is possible to separately incorporate DNAs encoding the antibody heavy chain (H chain) and the light chain (L chain) into expression vectors for simultaneous transformation of a host cell. Alternatively, it is possible to incorporate DNA encoding the H chain and the L chain into a single expression vector for transformation of a host cell (see WO 94/11523). Also, it is possible to produce a recombinant antibody with the use of a transgenic animal. For instance, a fusion gene may be prepared by inserting the antibody gene into a non-terminal region of the gene encoding a protein peculiarly produced in milk (e.g., goat β-casein). A DNA fragment comprising the fusion gene into which the antibody gene has been inserted is injected into a goat embryo and the thus obtained embryo is introduced into a female goat. A desired antibody can be obtained from milk produced by a transgenic goat born from the goat into which the embryo had been introduced or by a progeny thereof (Ebert, K. M. et al., Bio/Technology 1994; 12: 699-702).

Examples of the anti-RANKL antibody of the present invention include gene recombinant antibodies that have been artificially modified so as to, for example, have decreased levels of heterologous antigenicity to humans, such as chimeric antibodies and humanized antibodies. Specific examples of such antibody include chimeric antibodies, humanized antibodies, and human antibodies, which can be produced by known methods. A chimeric antibody can be obtained by obtaining DNA encoding the antibody V region, ligating the DNA to DNA encoding the human antibody C region, incorporating the resultant into an expression vector, and introducing the vector into a host for antibody production. A humanized antibody may be referred to as reconstituted (reshaped) human antibody in some cases. A humanized antibody is obtained by transplanting the complementary determining region (CDR) of a non-human mammal antibody such as a mouse antibody into the complementary determining region of a human antibody. It can be produced by known methods (see EP 125023 and WO96/02576). The C region of a human antibody is used for that of a chimeric antibody or a humanized antibody. For instance, Cγ1, Cγ2, Cγ3, or Cγ4 can be used for the H chain and Cκ or Cλ can be used for the L chain. Further, in order to improve the stability of an antibody or production stability, the human antibody C region may be modified.

A human antibody can be obtained by administering an antigen to a transgenic animal having the ability to produce a human-derived antibody that has been imparted via introduction of, for example, a human antibody gene locus. Examples of such transgenic animal include a mouse. A method of producing a mouse capable of producing a human antibody is described in WO02/43478 and the like.

An anti-RANKL antibody includes not only a complete antibody but also a functional fragment thereof. A functional antibody fragment corresponds to a portion of an antibody (a partial fragment) having at least one action of the antibody on a relevant antigen. Specific examples thereof include F(ab')₂, Fab', Fab, Fv, disulfide-bond Fv, single chain Fv (scFv), and polymers of any thereof [D. J. King., Applications and Engineering of Monoclonal Antibodies., 1998 T. J. International Ltd].

In addition, when a monoclonal antibody is used, a single type of a monoclonal antibody may be used. However, 2 or more types, for example, 2 types, 3 types, 4 types, or 5 types of monoclonal antibodies, which recognize different epitopes, may be used.

It is possible to determine whether or not the above compound has agonist activity that promotes signal transmission via RANKL by, for example, administering an antibody to osteoblasts or osteoblast precursor cells capable of expressing RANKL or cells having characteristics similar to osteoblasts such as myoblasts, stromal cells, and mesenchymal stem cells, allowing the antibody to act on RANKL, and examining if differentiation or proliferation of such cells takes place. The occurrence or nonoccurrence of differentiation or proliferation can be determined based on, for example, an increase in the alkaline phosphatase activity of cells, calcification, and the like.

Further, when the above compound is administered to an animal, the bone density, the bone mineral content, and the bone surface area will increase. The term "bone density" refers to the numerical density of a mineral component such as bone calcium. The bone density can be determined by pQCT (peripheral quantitative computerized tomography with a peripheral bone X-ray CT apparatus), SXA (single energy X-ray absorptiometry), DXA (dual energy X-ray absorptiometry; a double energy X-ray absorption method), or the like. Furthermore, when the above compound is administered to an animal, an increase in cancellous bone density can be confirmed by three-dimensional structural analysis of bone with µCT. Further, increases in BV/TV (unit bone mass: bone volume/total tissue volume), trabecular width, and trabecular number can be confirmed by measurement of cancellous bone trabecular structure. Moreover, when the above compound is administered to an animal, an increase in the bone density in the cortical bone region can be confirmed by bone morphology measurement using pQCT.

The above indicates that the compound acts on RANKL located on osteoblasts, osteoblast precursor cells, mesenchymal stem cells, stromal cells, and myoblasts such that reverse signals are transmitted, which results in osteogenesis promotion. The composition of the present invention can enhance *in vitro* osteogenesis and thus can be used as a research reagent. Alternatively, it can be used *in vivo* as a pharmaceutical composition.

The compound of the present invention is for use in the treatment and prevention of bone metabolism diseases associated with osteopenia. Examples of such bone metabolism diseases include osteoporosis, juvenile osteoporosis, dysosteogenesis, hypercalcemia, hyperparathyroidism, osteomalacia, osteohalisteresis, osteolytic bone diseases, osteonecrosis, the Paget's disease, rheumatoid arthritis, bone mass reduction due to osteoarthritis, inflammatory arthritis, osteomyelitis, glucocorticoid treatment, metastatic bone diseases, periodontal bone loss, bone loss due to cancer, bone loss due to aging, and other osteopenia-related diseases.

The dosage of the compound would vary depending on symptoms, patient age and weight, and the like. However, for oral administration, the dosage is generally approximately 0.01 mg to 1000 mg per day for adults. Administration can be carried out in the form of single-dose administration or multiple-dose administration. In addition, for parenteral administration, a single dose of approximately 0.01 mg to 1000 mg can be administered via subcutaneous injection, muscle injection, or intravenous injection. In addition, in terms of administration time, administration can be carried out before or after clinical symptoms of arteriosclerotic diseases have been developed. A pharmaceutical composition embodying the compound for use in accordance with the invention may comprise a carrier, a diluent, and an excipient that are generally used in the field of formulations. For example, lactose, magnesium stearate, and the like can be used as carriers or excipients for tablets. Examples of an injectable aqueous liquid that can be used include a physiological salt solution and an isotonic solution comprising glucose and other adjuvants. Such injectable aqueous liquid may be used in combination with an appropriate solubilizing agent such as alcohol, polyalcohol (e.g., propylene glycol), or a nonionic surface active agent. Examples of an oily liquid that can be used include sesame oil and soybean oil. Such oily liquid may be used in combination with a solubilizing agent such as benzyl benzoate or benzyl alcohol.

The compound of the present invention may be for use in combination with a BMP (bone morphogenetic protein; osteogenesis protein) family member.

That is, further excellent effects can be obtained with the combined use of a BMP family member and a compound that acts or does not act on RANKL and promotes differentiation, proliferation, maturation, or calcification of osteoblasts or cells capable of differentiating into osteoblasts so as to induce osteogenesis, thereby causing bone mass enhancement and the like. In particular, the combined use of a BMP family member and peptide D, peptide E, or an anti-RANKL antibody is preferable.

When the compound of the present invention is for use in combination with a BMP family member, it is possible to prepare a pharmaceutical formulation comprising both substances for administration. Alternatively, it is also possible to separately administer the compound of the present invention and a BMP family member. Specifically, the pharmaceutical composition of the present disclosure comprises a combination preparation of a BMP family member and a compound that promotes differentiation, proliferation, maturation, or calcification of osteoblasts or cells capable of differentiating into osteoblasts, and particularly, peptide D, peptide E, or an anti-RANKL antibody. Examples of a BMP family member include BMP-4, BMP-2, BMP-7, and BMP-6. The compound of the present invention and a BMP family member interact to promote differentiation, proliferation, maturation, and calcification of osteoblasts or cells capable of differentiating into osteoblasts so as to induce osteogenesis, thereby inducing bone mass enhancement and the like. The content of a BMP member is not limited. However, for example, the single dose thereof is approximately 0.01 mg to 1000 mg.

The present disclosure further encompasses a method of screening for a compound that promotes differentiation, proliferation, maturation, and calcification of osteoblasts or cells capable of differentiating into osteoblasts so as to induce osteogenesis, thereby inducing bone mass enhancement and the like. One example is a compound that acts on RANKL and promotes differentiation, proliferation, maturation, and calcification of osteoblasts or cells capable of differentiating into osteoblasts so as to induce osteogenesis, thereby inducing bone mass enhancement and the like.

For the screening method, a candidate substance is administered to osteoblasts, osteoblast precursor cells, or cells having characteristics similar to those of osteoblast precursor cells such as stromal cells, mesenchymal stem cells, or myoblasts. Then, it is examined whether or not the candidate compound promotes differentiation and proliferation of the cells. For instance, a candidate compound is administered to osteoblasts, osteoblast precursor cells, or cells having characteristics similar to those of osteoblast precursor cells such as stromal cells, mesenchymal stem cells, or myoblasts, on which RANKL has been expressed. Then, it is examined whether or not the candidate compound acts on RANKL and promotes differentiation and proliferation of the cells. The occurrence of differentiation and proliferation can be judged based on an increase in the alkaline phosphatase activity of the cells, the degree of calcification of the cells, and the like. If differentiation or proliferation is promoted, it can be judged that the candidate compound is a compound that promotes differentiation, proliferation, maturation, or calcification of osteoblasts or cells capable of differentiating into osteoblasts so as to induce osteogenesis, thereby causing bone mass enhancement and the like. One example is a compound that acts on RANKL and promotes differentiation, proliferation, maturation, or calcification of osteoblasts or cells capable of differentiating into osteoblasts so as to induce osteogenesis, thereby causing bone mass enhancement and the like.

In addition, when a candidate compound is administered to a mouse (e.g., C57BL/6CrjCrlj), it can be judged whether or not the candidate compound is a target compound by examining whether or not the bone density, the bone mineral content, the bone surface area, or the like increases. In such case, the target compound is a compound that promotes differentiation, proliferation, maturation, or calcification of osteoblasts or cells capable of differentiating into osteoblasts so as to induce osteogenesis, thereby causing bone mass enhancement and the like. One example is a compound that acts on RANKL and promotes differentiation, proliferation, maturation, or calcification of osteoblasts or cells capable of differentiating into osteoblasts so as to induce osteogenesis, thereby causing bone mass enhancement and the like.

The present invention is hereafter described in greater detail with reference to the following examples, although the technical scope of the present invention is not limited thereto.

### Example 1: Differentiation of human mesenchymal stem cells

### Reagents

A synthetic peptide was used as a reagent for experiments. Synthetic peptide D is a peptide comprising the amino acid sequence represented by SEQ ID NO: 7, which is a cyclic peptide consisting of 9 amino acids and comprising two cysteine residues binding to each other via a disulfide bond. It has been reported that synthetic peptide D binds to RANKL (Aoki et al., J Clin Invest 116: 1525, 2006). As a control peptide, a synthetic peptide lacking the above function was used.

### Culture cells

Human mesenchymal stem cells were purchased from Cambrex Corporation. Maintenance medium produced by Lonza was used for subculture.

### Differentiation of human mesenchymal stem cells

Human mesenchymal stem cells were seeded on a 96-well plate (1 × 10³ cells/well) (Nunc) and a 48-well plate (2.4 × 10³ cells/well) (IWAKI). After 24 hours, the culture supernatant was removed from each plate and an osteoblast differentiation induction medium (Lonza) was introduced thereonto. The medium was replaced with new medium every 3 or 4 days.

At such time, peptide D was added at a concentration of 100 µM (group treated with peptide D). As a negative control, the control peptide was added at the same concentration.

### ALP (alkaline phosphatase) activity determination

Seven days after differentiation, the culture supernatant was removed from each plate and the cells were fixed with an acetone/ethanol solution. The ALP activity was determined using p-nitrophenyl phosphate as a substrate. Specifically, a carbonate buffer (5 mM MgCl₂, 50 mM NaHCO₃) containing p-nitrophenyl phosphate (Nacalai) (1 mg/mL) was added to each well (100 µL/well), followed by incubation at 37°C. Then, the OD value at 405 nm was determined with the use of a microplate reader (BMG Labtech) for each well.

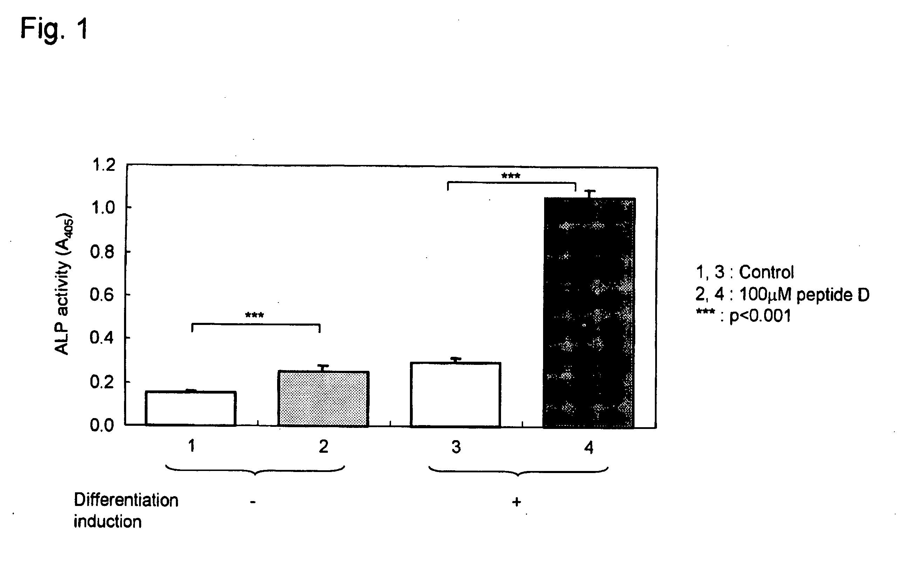

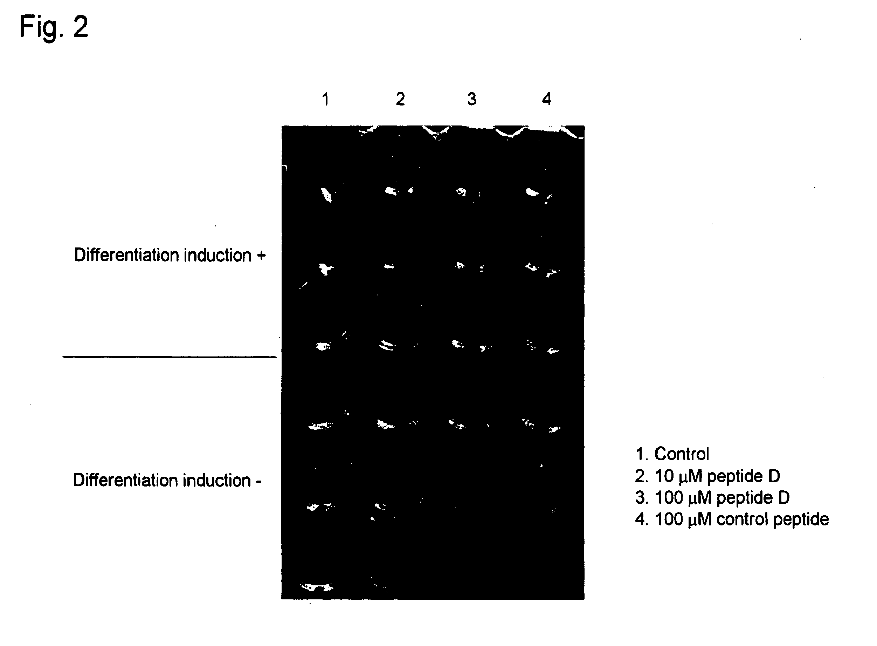

In the group of human mesenchymal stem cells to which peptide D (100 µM) had been added, the ALP activity significantly increased in the differentiation medium and in the maintenance medium on Day 7 (fig. 1). In addition, ALP staining was observed in a concentration-dependent manner in the group treated with peptide D when compared with the control group (fig. 2).

### Example 2: Calcification of human mesenchymal stem cells

### ALP staining

Peptide D was added at a concentration of 300 µM during differentiation. On Day 7, cells were fixed with a 10% neutral buffer formalin solution, followed by re-fixation with an acetone/ethanol solution.

A stain solution (500 µL) prepared with the composition described below was added to the cells, followed by incubation at 37°C for 10 minutes, washing with water, and drying.

### (Composition of stain solution)

| | |
|---|---|
| Naphthol AS-MX phosphate (SIGMA): | 5 mg |
| N-N-dimethylformamide (Wako): | 0.5 mL |
| 0.1 M Tris-HCl (pH 8.5): | 50 mL |
| Fast blue hemi-salt (SIGMA): | 30 mg |

### Alizarin red S staining

On Day 21 after differentiation, the cells were washed with PBS, followed by fixation with a 10% neutral buffer formalin solution.

The cells were washed with water after removal of the fixative solution. A 1% alizarin red S stain solution (Nacalai) (150 µL) was added thereto. The plate was left at room temperature for 3 minutes. Thereafter, the stain solution was discarded, followed by washing with water and drying. Then, microscopic observation was carried out.

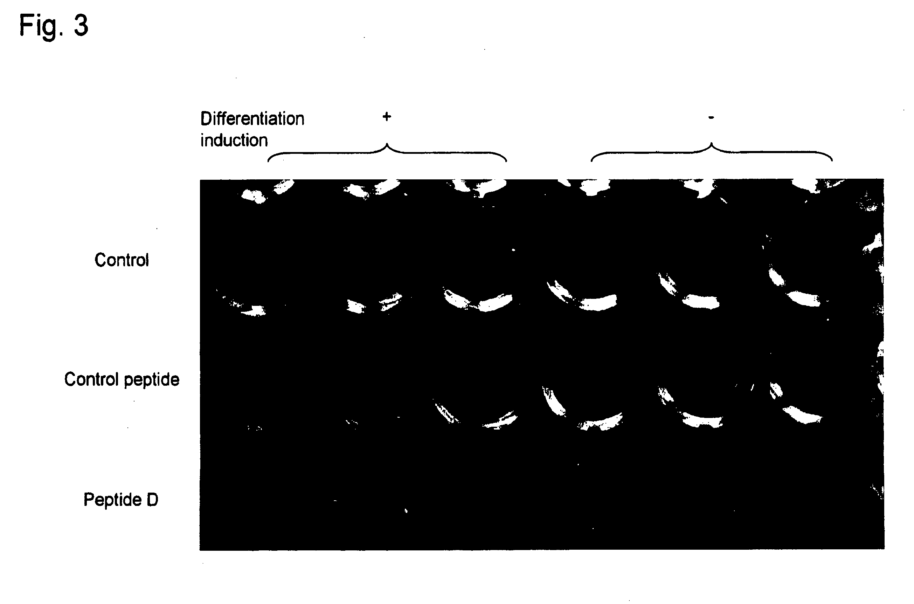

Human mesenchymal stem cells were cultured in the same manner as in Example 1. Peptide D (300 µM) was added to human mesenchymal stem cells. Accordingly, regardless of induction of differentiation, a strong degree of alizarin red staining was observed on Day 21. This indicated that peptide D induced calcification in addition to an increase in ALP activity (fig. 3).

### Example 3: Differentiation of a mouse osteoblast precursor cell line (MC3T3-E1) Culture cells

MC3T3-E1 (subclone No. 4) cells of a mouse osteoblast precursor cell line were purchased from ATCC.

### Mouse osteoblast precursor cells (MC3T3-E1)

Cells mixed with 10%FBS + αMEM (GIBCQ) were seeded on a 96-well plate (8 × 10³ cells/well) and a 48-well plate (2 x 10⁴ cells/well). The culture supernatant of each plate was replaced with 10% FBS + αMEM containing 5 mM β glycerophosphoric acid (SIGMA) + 10 µg/mL sodium ascorbate (SIGMA) 48 hours later. Then, the medium was replaced with new medium every 3 or 4 days. As a control, MC3T3-E1 was cultured with the use of 10% FBS + αMEM as a maintenance medium. Upon medium replacement, a peptide was added to each plate at a concentration of 300 µM. ALP activity determination and alizarin red S staining were carried out by the method described in Example 1 on Days 7 and 21.

### Differentiation of a mouse osteoblast precursor cell line

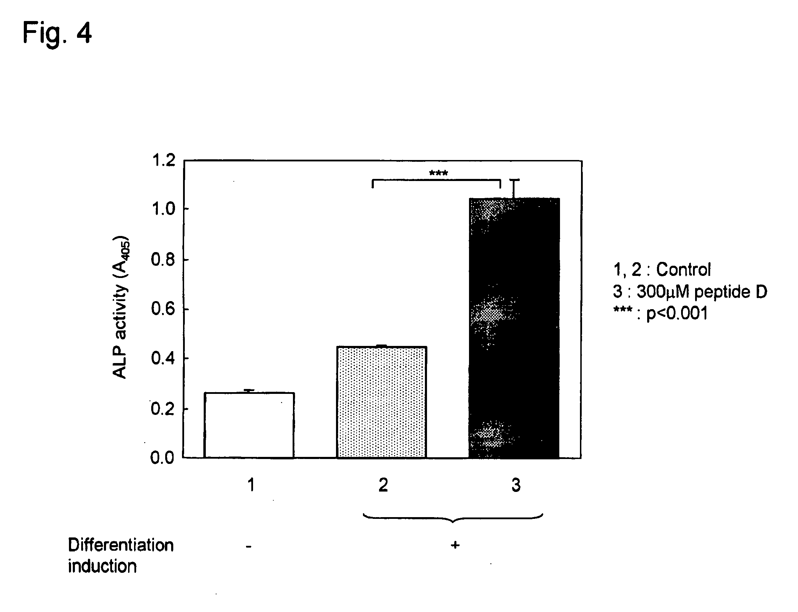

In the group to which peptide D (300 µM) had been added, the ALP activity had significantly increased by Day 7 after differentiation of MC3T3-E1 cells (fig. 4).

### The effect of peptide D on calcification

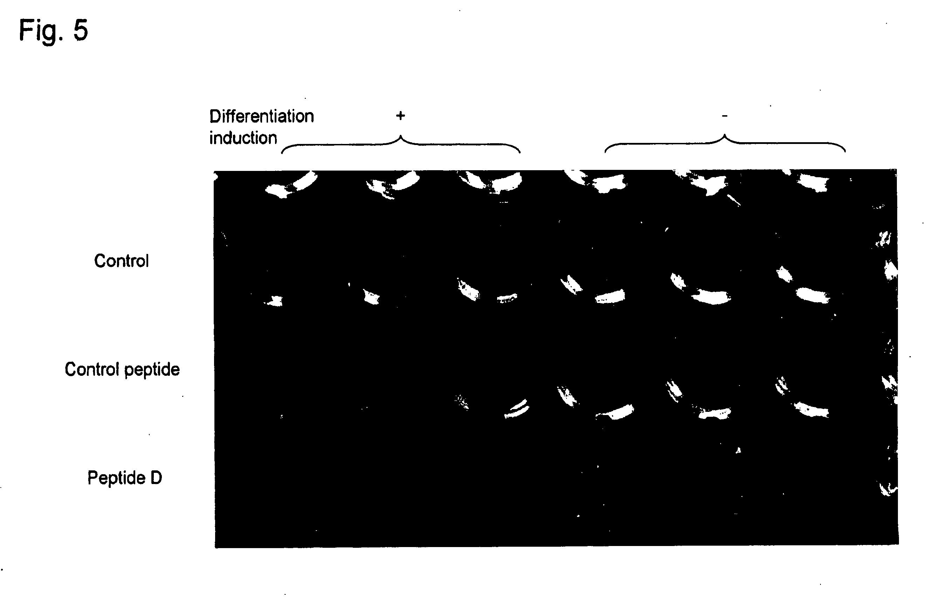

In the group to which peptide D (300 µM) had been added, a strong degree of alizarin red staining was observed on Day 21 after differentiation of MC3T3-E1 cells (fig. 5). This indicated peptide D induces calcification in addition to an increase in ALP activity. Such phenomenon was observed not only in the culture in the differentiation medium but also in that in the maintenance medium.

### Example 4: Differentiation of mouse osteoblasts

### Reagents

For experiments, a goat polyclonal mRANKL antibody (R&D, Saint Cruz), monoclonal mRANKL antibodies ((A): clone 88227 (R&D); (B): clone 12A668) (ALEXIS), and synthetic peptide D were used. As negative controls, goat IgG (ZYMED) and a control peptide were used. Synthetic peptide D and the control peptide used were the same as those used in Example 1. In addition, as a positive control, 300 ng/mL BMP-2 (R&D) was used. ALP activity determination was carried out as in Example 1.

### Collection of mouse osteoblasts

Newborn mouse calvarias were immersed in an enzyme solution (0.1% collagenase (Wako) + 0.2% Dispase (GODO SHUSEI CO., LTD.)), followed by shaking in a thermostatic bath at 37°C for 5 minutes. The initial floating cell fraction was removed therefrom and a new enzyme solution (10 mL) was added thereto, followed by another instance of shaking in a thermostatic bath at 37°C for 10 minutes. The above operations were repeated 4 times. Collection of the floating cell solution was carried out each time. Each floating cell solution was centrifuged at 250 × g for 5 minutes. The resultant was suspended in a medium, followed by culture in a CO₂ incubator for 3 or 4 days. Cells were collected with the use of a trypsin-EDTA solution (Nacalai), followed by cryopreservation with the use of a Cell Banker (Juji Kagaku (Juji Field Inc.)).

### Differentiation of mouse osteoblasts

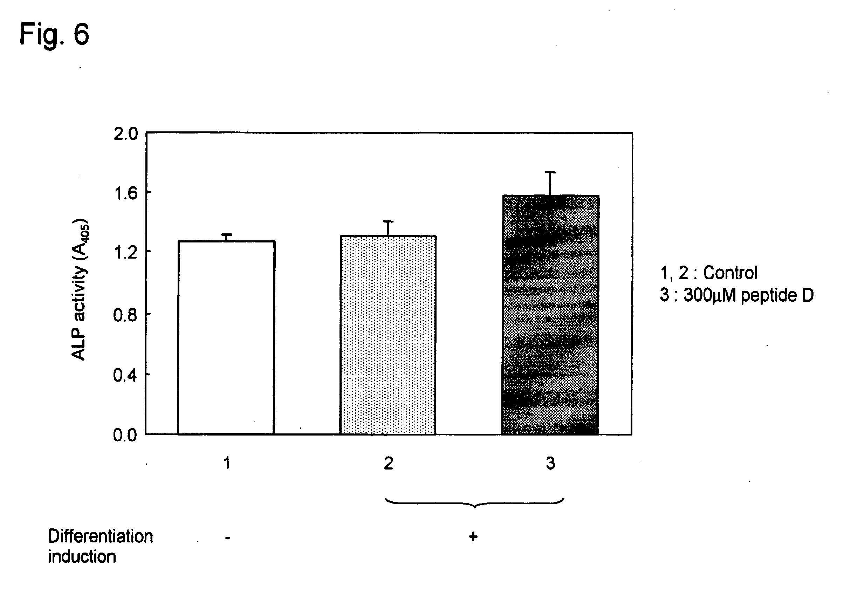

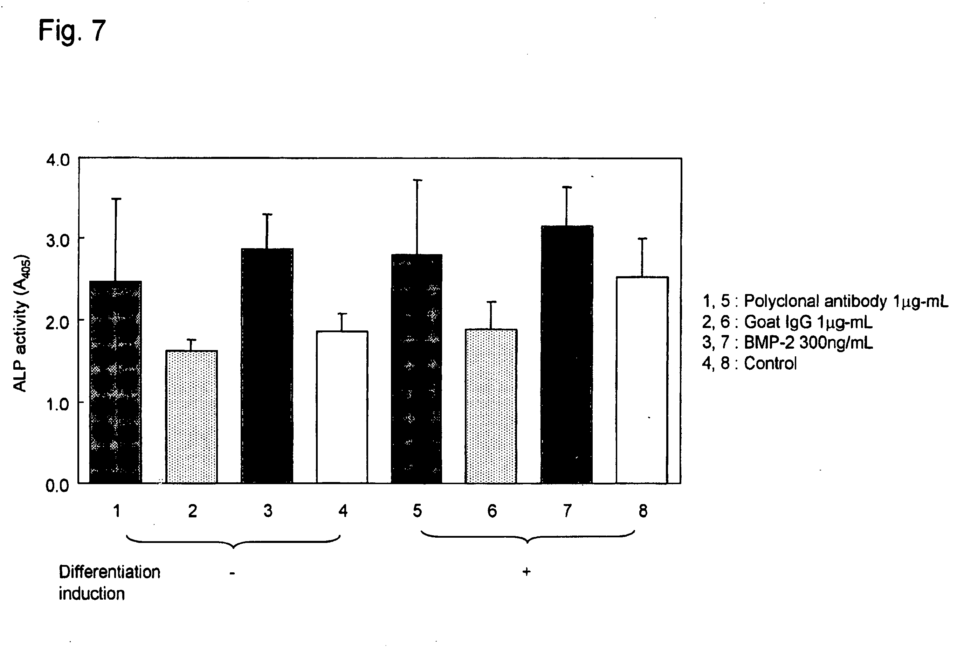

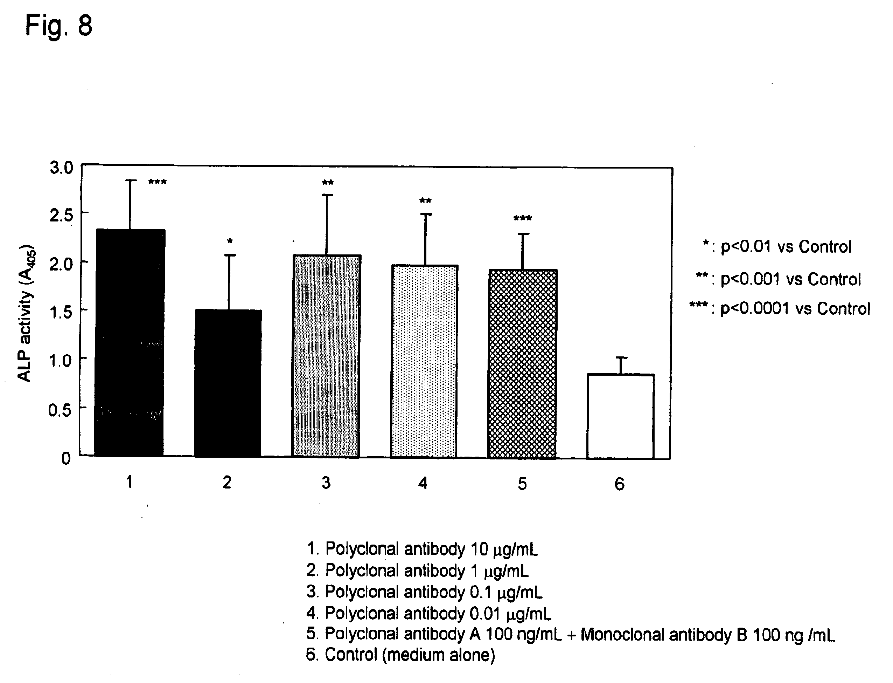

The obtained mouse osteoblasts were seeded on a 96-well plate (0.8 x 10⁴/well) with the use of 10%FBS + αMEM. After cell adhesion had taken place, cell differentiation was induced in a medium containing 5 mM β-glycerophosphoric acid + 10 µg/mL ascorbic acid. As a result, it was confirmed that 300 µM synthetic peptide D had caused an increase in the degree of ALP activation in the differentiation medium by Day 7 (fig. 6). The above antibodies (1 µg/mL each) were added upon differentiation. On Day 7 after differentiation, a significant increase in ALP activity was confirmed in the groups to which the relevant factors had been added (fig. 7). This phenomenon was observed not only in the mouse osteoblasts cultured in the differentiation medium but also in the mouse osteoblasts cultured in the maintenance medium. That is, a polyclonal antibody capable of binding to RANKL significantly induced differentiation of mouse osteoblasts compared with the control antibody. In order to confirm the effects of the antibodies in further detail, the polyclonal antibody was added to mouse osteoblasts at a different concentration, followed by culture in a maintenance medium for 7 days. At such time, the anti-RANKL monoclonal antibodies A and B were used to examine the possibility of confirming similar differentiation actions also with the use of monoclonal antibodies. As a result, the anti-RANKL polyclonal antibody was found to cause an increase in ALP activity of mouse osteoblasts in a concentration-dependent manner, although dispersion was observed to some extent (fig. 8). Further, a combination of the anti-RANKL monoclonal antibodies A and B caused an increase in ALP activity of mouse osteoblasts in a similar manner (fig. 8). Based on the above, it was found that mouse RANKL polyclonal antibodies and monoclonal antibodies can induce differentiation of mouse osteoblasts.

### Example 5: ALP activation actions of human osteoblasts

The reagents such as anti-RANKL polyclonal antibodies and anti-RANKL. monoclonal antibodies used in this Example were the same as those used in Example 4. In addition, an anti-RANKL monoclonal antibody ((C): clone 12A380) (ALEXIS), human OPGFc, and human RANKFc (R&D)) were used. ALP activity determination was carried out as in Example 1. All of the above antibodies are antibodies against mouse RANKL. In addition, these antibodies have been found to be cross-reacted with and bind to human RANKL.

### Culture cells

Human osteoblasts were purchased from Cambrex Corporation. A dedicated medium (Lonza) was used for subculture.

### Differentiation of human osteoblasts

Human osteoblasts were seeded on a 96-well plate (3.1 × 10³/well) and a 48-well plate (7.65 × 10³/well). After cell adhesion had taken place, differentiation was carried out using a medium containing 5 mM β-glycerophosphoric acid. The antibodies were added (100 ng/mL or 1 ng/mL) upon differentiation. Culture was carried out for 5 or 6 days, followed by ALP activity determination.

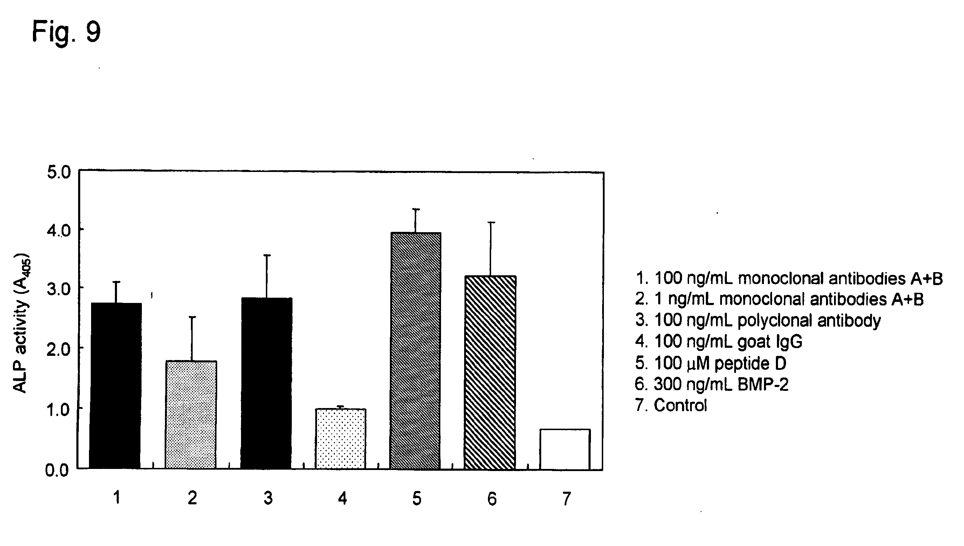

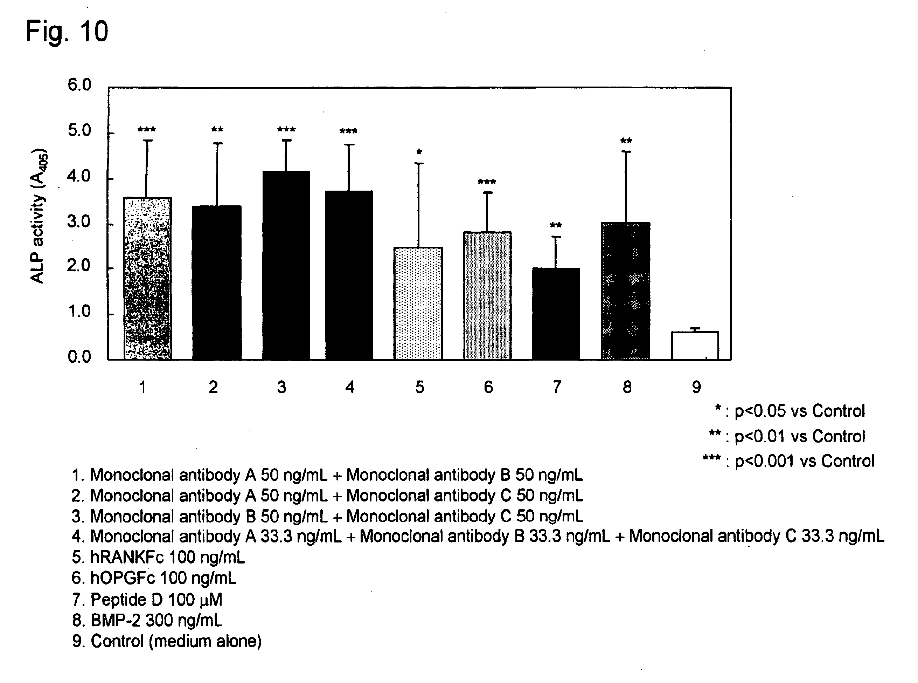

Differentiation was confirmed with a significant increase in ALP activity of human osteoblasts caused by anti-RANKL polyclonal antibodies, anti-RANKL monoclonal antibodies, peptide D, OPGFc, and RANKFc (figs. 9 and 10). Based on the above, it was found that that polyclonal antibodies, monoclonal antibodies, OPGFc, and RANKFc against mouse RANKL can induce differentiation of human osteoblasts.

### Example 6: Differentiation of mouse myoblasts

The reagents such as monoclonal mRANKL antibodies used in this Example were the same as those used in Example 4. ALP activity determination was carried out as in Example 1.

### Culture cells

C2C12 cells (of a mouse myoblast precursor cell line) were purchased from RIKEN.

### Differentiation of mouse myoblasts

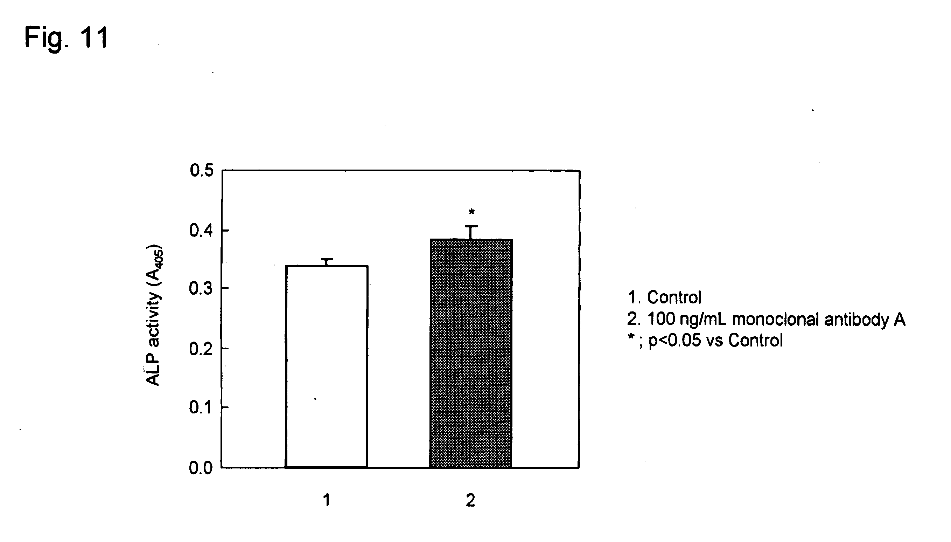

C2C12 cells, which are mouse myoblast precursor cells, were seeded on a 96-well plate (6.5 × 10³ cells/well). After 48 hours, the culture supernatant was replaced with 5% FBS + DMEM (SIGMA) containing 300 ng/mL BMP-2 (R&D). During medium replacement, an antibody (100 ng/mL) was added thereto, followed by culture for 7 days. It was confirmed that a monoclonal mRANKL antibody ((A) clone 88227 (R&D)) caused a significant increase in ALP activity of mouse myoblasts, resulting in differentiation into osteoblasts (fig. 11). As a result of the experiment, it was revealed that an anti-RANKL monoclonal antibody acted alone on mouse myoblasts having the features of osteoblast precursor cells so as to induce differentiation of the cells into osteoblasts.

### Example 7: ALP activation actions of peptide D upon human mesenchymal stem cells RT-PCR analysis

Human mesenchymal stem cells (3 × 10⁴ cells) were seeded on a 6-well plate, followed by culture for 7 days in the presence of an osteoblast differentiation medium (Lonza) or a maintenance medium (Lonza). Peptide D (100 µM and 300 µM) was added to each well. Further, a peptide solvent was added to a control group. After culture, the cells were washed with PBS and dissolved in QIAzol Lysis Reagent (QIAGEN) (0.75 mL). Then, the solution was collected. The solution was left at room temperature for 5 minutes. Then, chloroform (Wako) (0.15 mL) was added, the resultant was admixed by turning upside down, and then the resultant was centrifuged under conditions of 4°C and 12000 × g for 15 minutes. The supernatant was collected in a new tube. RNA was isolated from the supernatant with the use of an EZ1 RNA universal tissue kit (QIAGEN) and Magtration System 12GC (QIAGEN). After determination of the RNA concentration, each RNA (250 ng) was subjected to electrophoresis with 1% agarose gel for confirmation of the occurrence or nonoccurrence of RNA degradation. Non-degraded RNAs (500 ng each) were subjected to RT-PCR. RT-PCR was performed using a ThermoScript RT-PCR System (Invitrogen) and random primers.

After cDNA synthesis, PCR was performed using primers specific to human alkaline phosphatase (hALP) and human type I collagen (hCollagen I). For standardization, PCR was performed using primers specific to human GAPDH. The PCR primer sequences used are shown below. In addition, PCR was performed using Ex Taq^{™} Hot Start Version (Takara Bio Inc., Shiga, Japan) under the following conditions. Alkaline phosphatase (hALP) was allowed to undergo initial thermal denaturation at 94°C for 15 minutes, followed by 28 cycles of 94°C for 1 minute, 58°C for 1 minute, and 72°C for 30 seconds and an elongation reaction at 72°C for 10 minutes. type I collagen (hCollagen I) was allowed to undergo initial thermal denaturation at 94°C for 15 minutes, followed by 25 cycles of 94°C for 1 minute, 58°C for I minute, and 72°C for 30 seconds and an elongation reaction at 72°C for 10 minutes. GAPDH was allowed to undergo initial thermal denaturation at 95°C for 3 minutes, followed by 28 cycles at 95°C for 10 seconds, 60°C for 15 seconds, and 68°C for 1 minute and an elongation reaction at 68°C for 10 minutes.

### PCR primer sequence

hALP-F: 5'-GGGGGTGGCCGGAAATACAT-3' (SEQ ID NO: 8)
hALP-R: 5'-GGGGGCCAGACCAAAGATAGAGTT-3' (SEQ ID NO: 9)
hCollagenI-F: 5'-ATTCCAGTTCGAGTATGGCG-3' (SEQ ID NO: 10)
hCollagenI-R: 5'-TTTTGTATTCAATCACTGTCTTGCC-3' (SEQ ID NO: 11)
hGAPDH-F: 5'-TGAAGGTCGGAGTCAACGGATTTGGT-3' (SEQ ID NO: 12)
hGAPDH-R: 5'-CATGTGGGCCATGAGGTCCACCAC-3' (SEQ ID NO: 13)

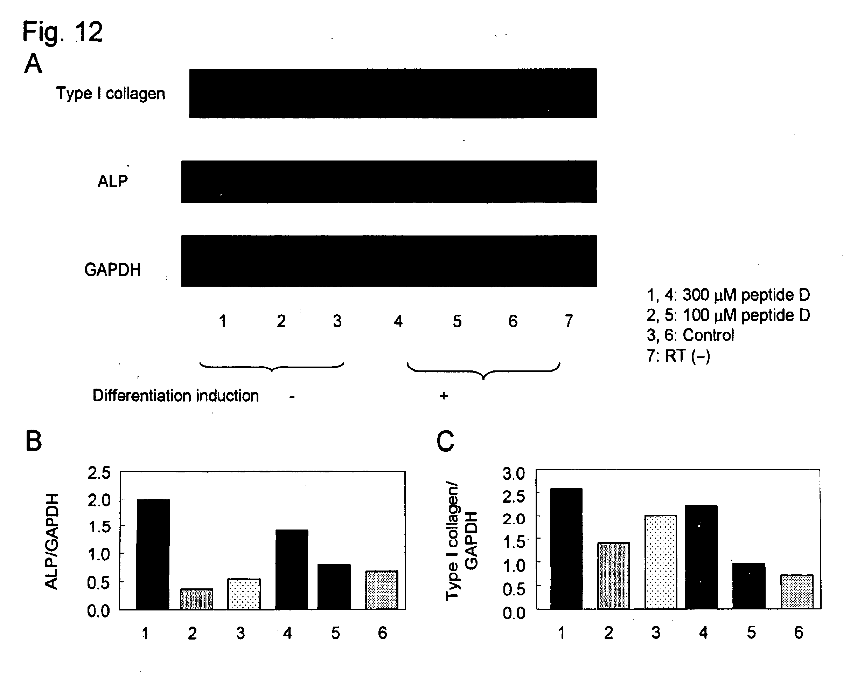

Samples obtained after the PCR reaction were subjected to electrophoresis with 1% agarose gel. Formation of specific bands was confirmed under UV light with the use of ethidium bromide (fig. 12A). The obtained images were analyzed with a CSAnalyzer. Figs. 12B and 12C show the results of standardization based on the GAPDH expression level.

In the group of human mesenchymal stem cells to which peptide D (300 µM) had been added, a significant increase in ALP activity was observed in both the differentiation medium and the maintenance medium by Day 7 after differentiation. In addition, when ALP staining of the cells was carried out on Day 7 after differentiation, ALP staining was confirmed to take place in a concentration-dependent manner in the group treated with peptide D, compared with the control group. However, as a result of PCR analysis, it was confirmed that the expression levels of alkaline phosphatase and type I collagen had increased in a peptide-concentration-dependent manner after 7-day differentiation of human mesenchymal stem cells in the presence of the peptide (fig. 12A). Also in the maintenance medium, the peptide at a concentration of 300 µM caused increases in the mRNA expression levels of alkaline phosphatase and type I collagen (figs. 12B and C).

### Example 8: Differentiation of C2C12 into osteoblasts caused by membrane-bound RANK

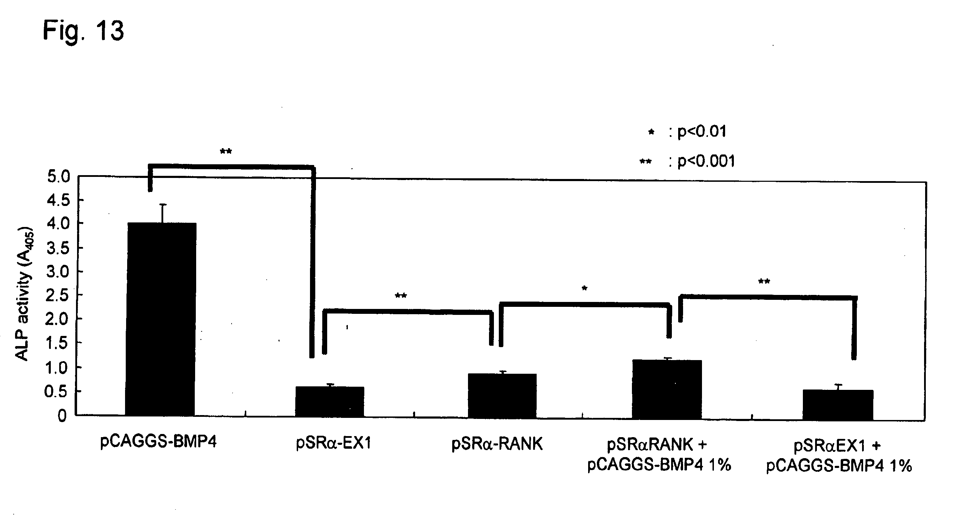

COS1 cells mixed with DMEM-5% FBS were seeded on a 96-well plate (10000 cells/well), followed by culture for 1 day. Thereafter, the cells were transfected with a variety of plasmid DNAs (pSRα-EX1 (control expression vector 1), pSRα-mRANK (mouse RANK expression vector), and pCAGGS-mBMP-4 (mouse BMP-4 expression vector) purified with a QIAwell8 plasmid purification kit (Qiagen)) with the use of FuGENE HD (Roche) (50 ng per well). In a similar manner, pCAGGS-mBMP-4 (0.5 ng) was mixed with pCAGGS (control expression vector 2) (24.5 ng) and pSRα-mRANK (25 ng) and then subjected to transfection. As a control, pCAGGS-mBMP-4 (0.5 ng) was mixed with pCAGGS (24.5 ng) and pSRα-EX1 (25 ng) and then subjected to transfection. On the next day, C2C12 cells were seeded on a COS1 plate subjected to transfection (10000 cells per well), followed by coculture. The medium was replaced with DMEM-2.5% FBS every 3 days. After 1 week, the medium was removed therefrom, followed by fixation of the cells with the addition of a mixture solution (acetone : ethanol = 1 : 1). The fixative solution was removed after 30 seconds. The plate was dried for approximately 30 minutes, during which an ALP detection solution (5 mM MgCl₂, 40 mM NaHCO₃, 1 mg/ml p-nitrophenyl phosphate) was prepared and added to the plate in a step-wise manner (in an amount of 100 µl per a single instance of addition) for initiation of reaction. After 60 minutes, determination (ABS: 405 nm) was carried out with a microplate reader. As a result, pSRα-mRANK was found to have caused a significant increase in ALP activity, thereby exhibiting osteoblast differentiation activity, although the activity level was weaker than that strongly exhibited by pCAGGS-mBMP-4 serving as a positive control (fig. 13). In addition, when the proportion of pCAGGS-mBMP-4 was reduced to 1%, no significant difference was observed between pCAGGS-mBMP-4 and pSR α-EX1 serving as a control expression vector. However, as a result of simultaneous transfection with pSR-α-mRANK, a significant increase in ALP activity was observed (fig. 13). Further, when the proportion of pCAGGS-mBMP-4 was reduced to 1%, followed by simultaneous transfection with pSR α-mRANK, a more significant increase in ALP activity was observed than in the case of transfection with pSRα-mRANK alone (fig. 13). This indicates that membrane-bound RANK acted in cooperation with BMP-4. As described above, membrane-bound RANK induced differentiation of myoblastic C2C12 cells of a mouse myoblast strain, which have the features of osteoblast precursor cells, into osteoblasts independently or in corporation with BMP-4.

### Example 9: Differentiation of ST2 cells into osteoblasts caused by membrane-bound RANK

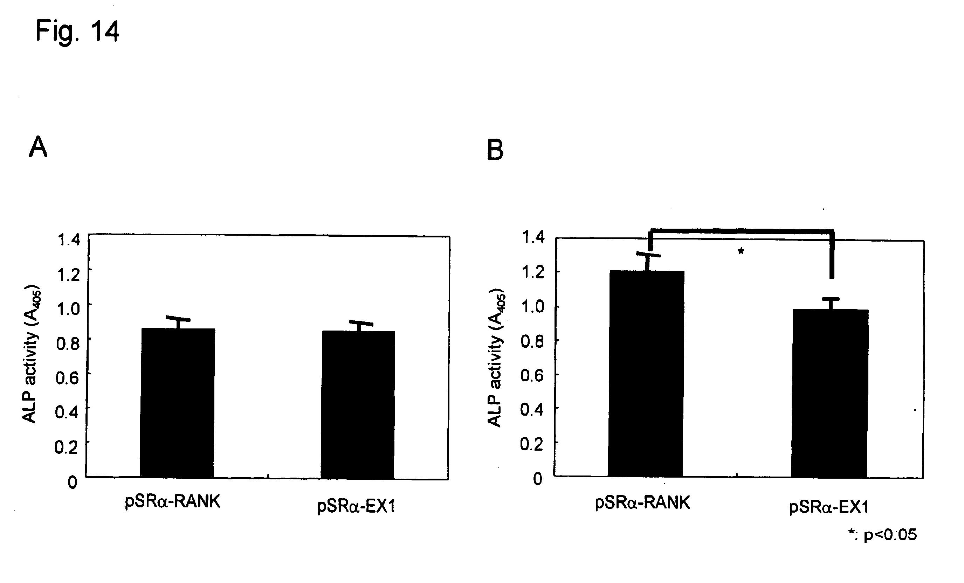

ST2 cells (of a mouse stromal cell line) were purchased from RIKEN. COS1 cells mixed with DMEM-5% FBS were seeded on a 96-well plate (10000 cells/well), followed by culture for 1 day. Thereafter, the cells were transfected with a variety of plasmid DNA (pSR α-EX1 (control expression vector 1) and pSR α-mRANK (mouse RANK expression vector) purified with a QIAwell 8 plasmid purification kit (Qiagen)) with FuGENE HD (Roche) (50 ng per well). On the next day, ST2 cells were seeded on the COS1 plate subjected to transfection (5000 cells per well), followed by coculture. In addition, in order to induce RANKL expression in ST2 cells, a system containing dexamethasone (10⁻⁷ M) and activated vitamin D₃ (10⁻⁸ M) for induction of RANKL expression in ST2 cells and a system containing none thereof (for lack of induction of RANKL expression) were prepared. After 3 days, DMEM-2.5% FBS was added thereto. Three days later, the medium was replaced with DMEM-2.5% FBS. One week thereafter, the medium was removed and a mixture solution (acetone : ethanol = 1 : 1) was added thereto for cell fixation. The fixative solution was removed after 30 seconds. The plate was dried for approximately 30 minutes, during which an ALP detection solution (5mM MgCl₂, 40 mM NaHCO₃, and 1 mg/ml p-nitrophenyl phosphate) was prepared and added to the plate in a step-wise manner (in an amount of 100 µl per a single instance of addition) for initiation of reaction. After 60 minutes, determination (ABS: 405 nm) was carried out with a microplate reader. As a result, pSR α-mRANK was found to have caused a significant increase in ALP activity exclusively in the system containing dexamethasone (10⁻⁷ M) and to have activated vitamin D₃ (10⁻⁸ M) (RANKL induction). In the system, ST2 cells exhibited osteoblast differentiation activity (figs. 14A and B). As described above, membrane-bound RANK independently induced differentiation of ST2 cells (of a mouse stromal cell line) into osteoblasts, such ST2 cells having the features of osteoblast precursor cells and adipocyte precursor cells. This phenomenon was observed exclusively in the case involving the use of ST2 cells that induced RANKL expression. This indicates that membrane-bound RANK bound to membrane-bound RANKL that had been induced to be expressed on ST2 cells so that osteoblast differentiation signals were transmitted inside the ST2 cells.

### Example 10: Proliferation and differentiation of osteoblasts caused by in vivo differentiation of osteoclasts

### Preparation of GST-RANKL

*Sal*I and *Not*I sites were added to cDNA encoding human RANKL residues 140-317 by PCR. The endonucleases were used for cloning of the cDNA downstream of Glutathione S-transferase of pGEX-4T-2 (GE healthcare; Genbank Accession Number: U13854). SEQ ID NOS: 14 and 15 represent the nucleotide sequence of DNA encoding a GST fusion protein having an amino acid sequence comprising amino acids at positions 140 to 317 of the amino acid sequence of RANKL and the amino acid sequence of the protein, respectively. After induction of protein expression in BL21 (DE3) *Escherischia coli* (Invitrogen) with the use of IPTG (final concentration: 0.5 mM), cells were suspended in an extraction buffer (50 mM Tris-HCl, pH 8.0, 100 mM NaCl, 1 mM EDTA, 1mM DTT, and 1% (v/v) TritonX-100), followed by fragmentation at 4°C with a sonicator. Centrifugation was performed at 18000 × g for 15 min. Then, the supernatant was collected and used to fill a Glutathione Sepharose column. Subsequently, washing was carried out with a wash buffer (50 mM Tris-HCl, pH 8.0, 100 mM NaCl, 1 mM DTT, and 0.1% (v/v) TritonX-100). Thereafter, elution was caused with a glutathione solution (20 mM reduced glutathione, 50 mM Tris-HCl, and pH 8.0). The molecular weight and the purity of GST-RANKL purified by SDS-PAGE were determined, followed by filter filtration. The molecular weight was 47.0 kDa and the purity was 95% or more. In addition, the endotoxin concentration was determined to be less than 1 EU/ug by limulus amebocyte lysate assay.

### RANKL administration test

GST-RANKL was intraperitoneally administered at 57 nmol (low dose) or at 426 nmol (high dose) to 7-week-old female C57BL/6N mice (10 individuals) every 24 hours for three times. The mice were dissected 1.5 hours after the third administration. For comparison, a group to which PBS had been administered was used as a control.

The following organs were collected from each dissected mouse: femur, tibia, cerebrum, lungs, heart, liver, thymus, spleen, kidneys, and skin. Naturally occurring lesions were observed by HE staining of the cerebrum, the lungs, the heart, the liver, the thymus, the spleen, the kidneys, and the skin.

### Bone morphology measurement

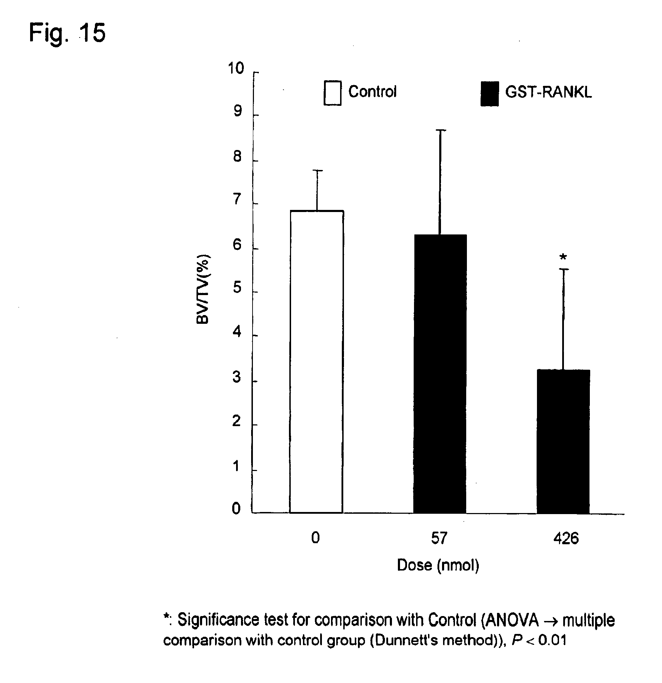

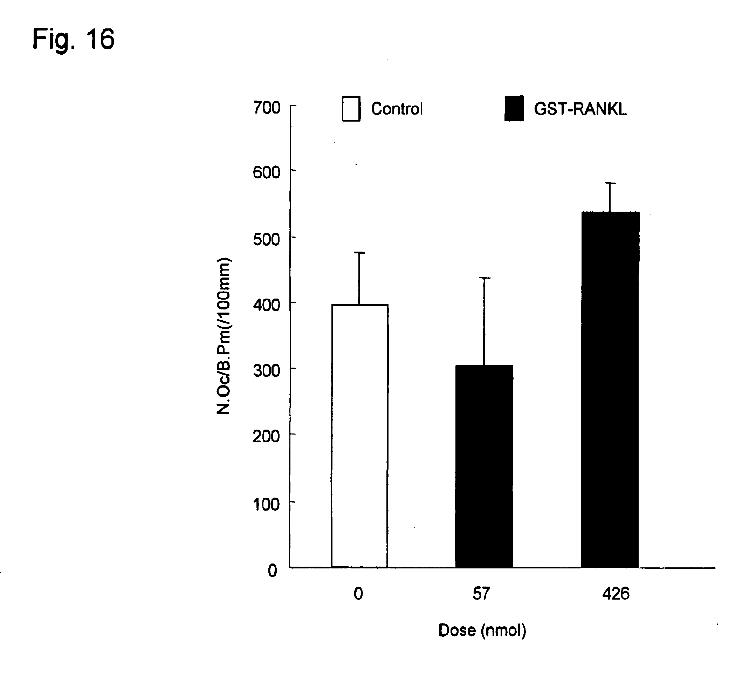

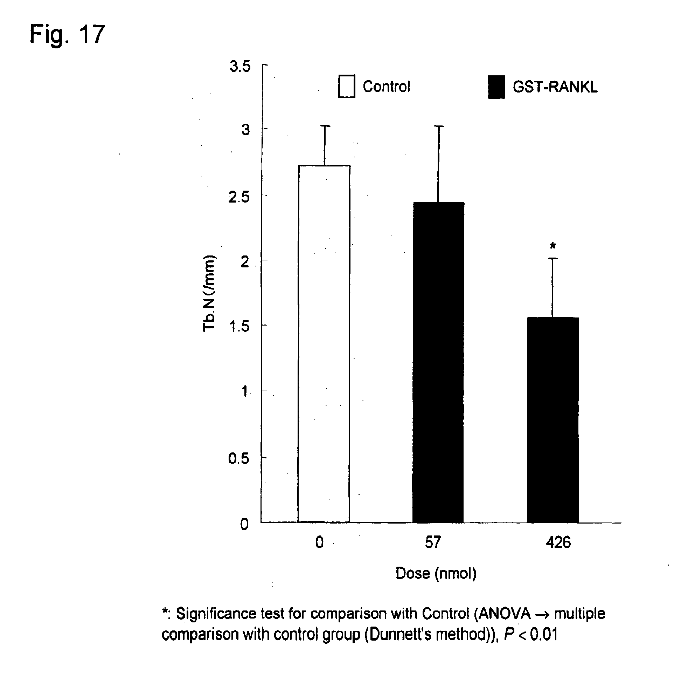

As a result of bone morphology measurement, the unit bone mass and the trabecular number were found to have decreased to approximately 50% as a result of high-dose GST-RANKL administration. Meanwhile, the osteoclast number was found to have increased. In addition, no such decrease was observed in the case of low-dose administration (figs. 15, 16 and 17).

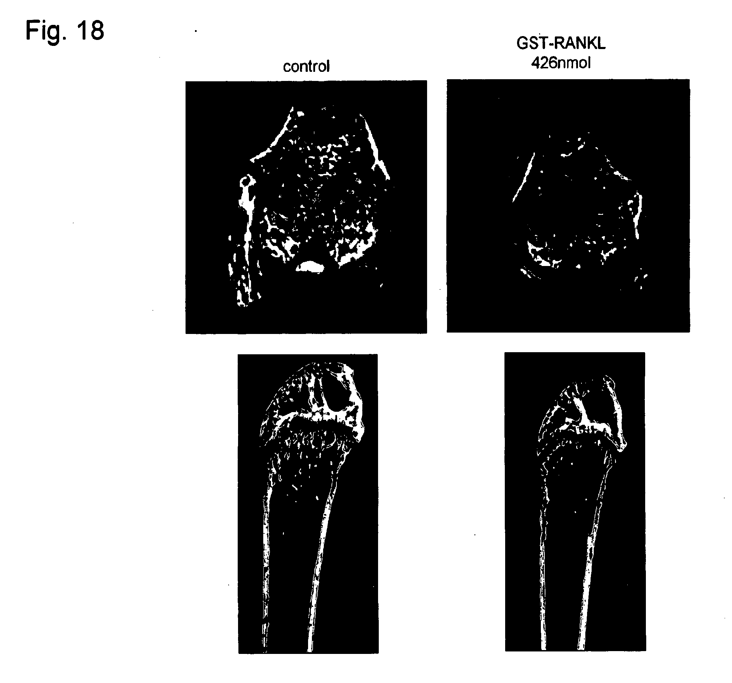

The femur bone morphology was determined by µCT. As a result, a significant bone decrease was observed in the high-dose GST-RANKL administration group (fig. 18).

The cerebrum, the lungs, the heart, the liver, the thymus, the spleen, the kidneys, and the skin removed from each mouse by dissection were subjected to HE staining and observed. However, abnormal findings and naturally occurring lesions were not observed in any group.

### Osteoblast surface

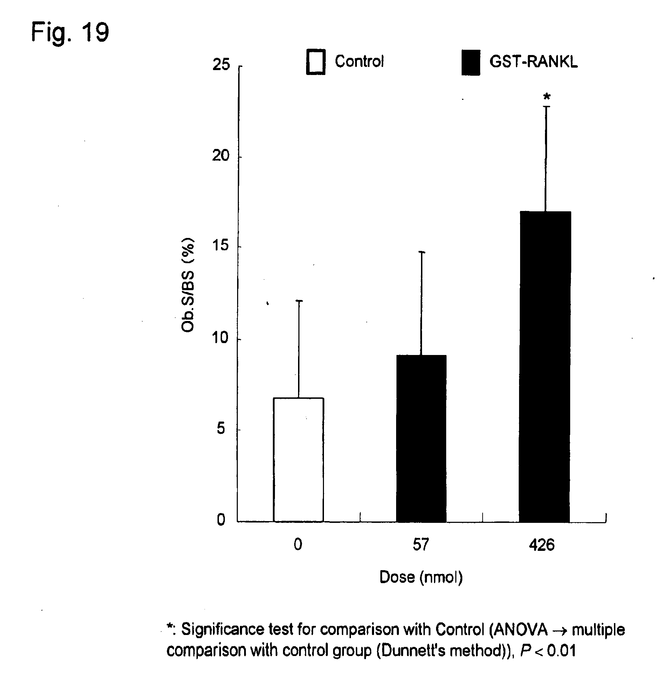

As a result of high-dose GST-RANKL administration, an increase in osteoclast number, a decrease in bone mass, and bone resorption were confirmed. Further, when the osteoblast surface area was examined, it was found to have significantly increased (fig. 19). This relates to a phenomenon in which osteogenesis is promoted by bone resorption enhanced by an increase in osteoclast number and osteoclast activation, indicating the occurrence of coupling of bone resorption and osteogenesis. It is thought that RANK located on osteoclasts, which had been increased and activated in a bone microenvironment, acted on RANKL on osteoblasts so as to transmit signals for differentiation, proliferation, maturation, or calcification in the mice to which high-dose GST-RANKL had been administered.

### Example 1-1: In vivo bone mass increase caused by administration of a synthetic peptide Reagents

A synthetic peptide was used for experiments. Synthetic peptide D is a cyclic peptide comprising the amino acid sequence represented by SEQ ID NO: 7, which consists of 9 amino acids and contains two cysteine residues binding to each other via a disulfide bond. It has been reported that synthetic peptide D binds to RANKL (Aoki et al., J Clin Invest 116: 1525, 2006). The synthetic peptide was dissolved in 10% DMSO (Nacalai)/PBS to a concentration of 1 mg/ml.

### Experimental animals

C57BL/6CrjCrlj mice were purchased from Oriental Bio Service. The mice used were C57BL/6CrjCrlj inbred mice characterized by weakened depression of the cellular immunity caused by aging. The mice were preliminarily raised in an environment at a temperature of 23°C ± 3°C and a humidity of 50% ± 30% for 1 week. Lighting time was 8:00 to 20:00.

During the experiments, all mice were fed with CR-LPF (Oriental Yeast Co., Ltd.).

A control group (n = 5) and a group treated with peptide D (n = 4) were raised in cages.

### Administration method and period

Subcutaneous administration of peptide D was carried out in a dose of 10 mg/kg at 8:00, 14:00, and 20:00 (three times daily) for 5 days. 5% DMSO/PBS was administered to the control group. Mice were dissected 12 hour after the completion of administration for 5 days and subjected to exsanguination. Thereafter, the femur and the tibia were collected from each mouse. The whole blood of each mouse was left at room temperature for 1 hour and then centrifuged under conditions of 5000 rpm and 4°C for 5 min. The serum was collected in a new tube. The femur and the tibia of each mouse were fixed with cold 70% ethanol.

### Bone density analysis

Each femur fixed with ethanol was subjected to single energy X-ray absorptiometry (SXA) analysis (DCS-600EX-IIIR DXA for animals, ALOKA) for determination of bone density, bone mineral content, and bone surface area. The full length of each bone was divided into 20 regions. The bone density for each region was determined and actions of the peptide in each region were analyzed.

### Bone structure analysis and trabecular structure analysis

Bone structure analysis was carried out by peripheral quantitative computed tomography (hereinafter abbreviated as pQCT) and micro-computed tomography (hereinafter abbreviated as µCT). Scan-Xmate-A080 (Comscantecno Co., Ltd.) was used for µCT and XCT-Research SA+ (Stratec Medizintechnik GmbH) was used for pQCT. The dedicated software (3D-BON (RATOC)) was used for preparation of three-dimensional structural image and trabecular structure analysis with the use of µCT data. In addition, for analysis by pQCT, a region with a bone density of 395 mg/cm³ or less was determined to be cancellous bone and a region with a bone density of 690 mg/cm³ or more was determined to be cortical bone.

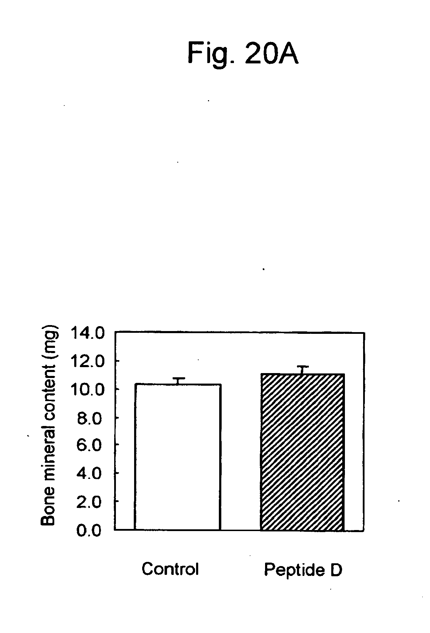

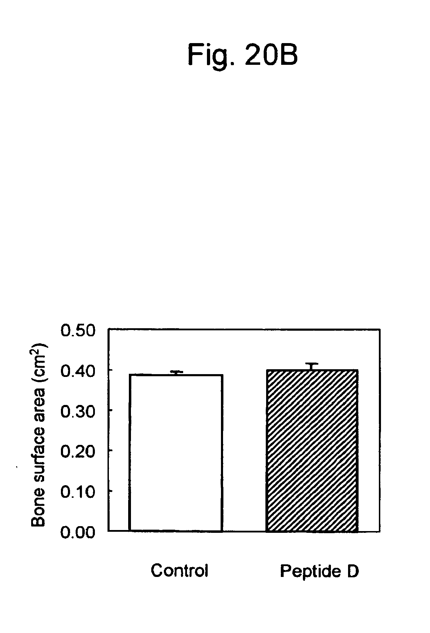

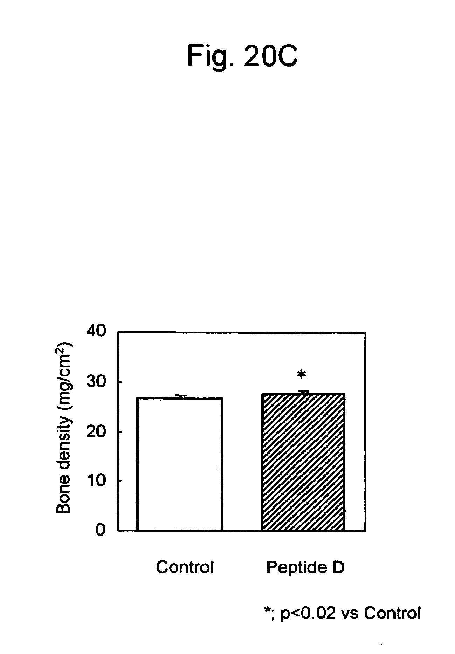

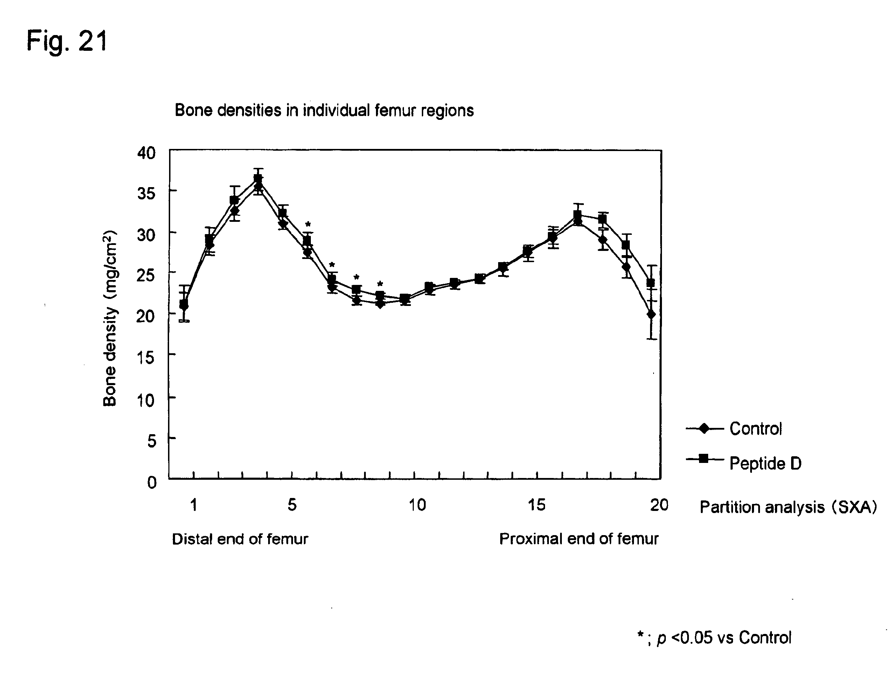

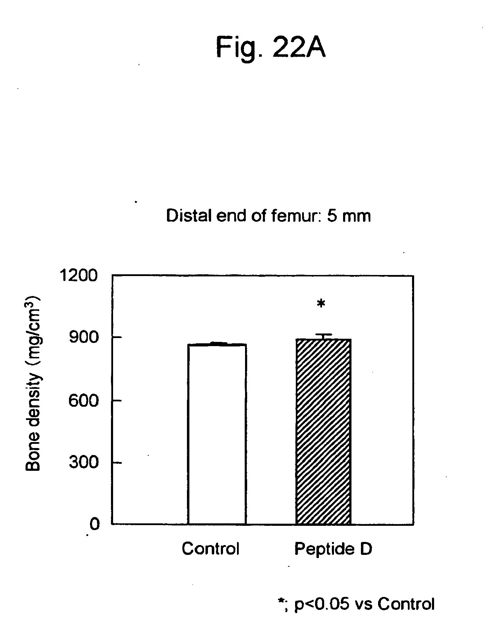

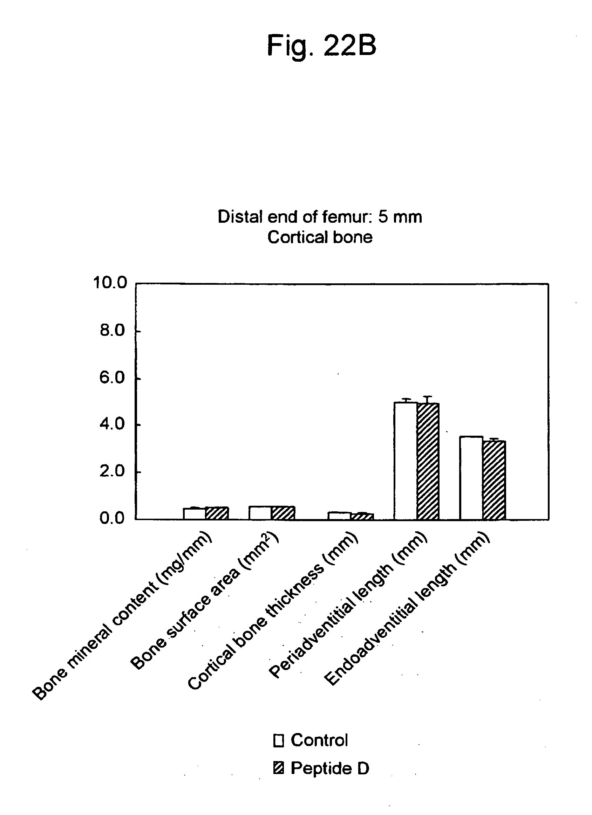

Each femur to which peptide D (10 mg/kg) had been administered 3 times daily for 5 days was subjected to SXA analysis for determination of total bone mineral content, bone surface area, and bone density. As a result, it was found that administration of peptide D resulted in an increase in bone mineral content and a significant increase in bone density (*p* < 0.05 vs control group) (figs. 20A to 20C). Further, as a result of determination of bone density of each region by SXA20 partition analysis, a significant increase in bone density was confirmed in the 6^{th} to 9^{th} regions from the distal end of the femur (*p* < 0.05 vs control group) (fig. 21). In addition, as a result of femur bone density analysis by pQCT, a significant increase in cortical bone density (*p* < 0.05 vs control group) was confirmed in a region located 5 mm away from the distal end of the femur (fig. 22A). As a result of determination of thickness, periadventitial circumference, endoadventitial circumference, bone mineral content, and bone surface area of the cortical bone in the region, the intimal circumference was found to be shortened, suggesting that the quantity of cortical bone increased toward the inside (fig. 22B).

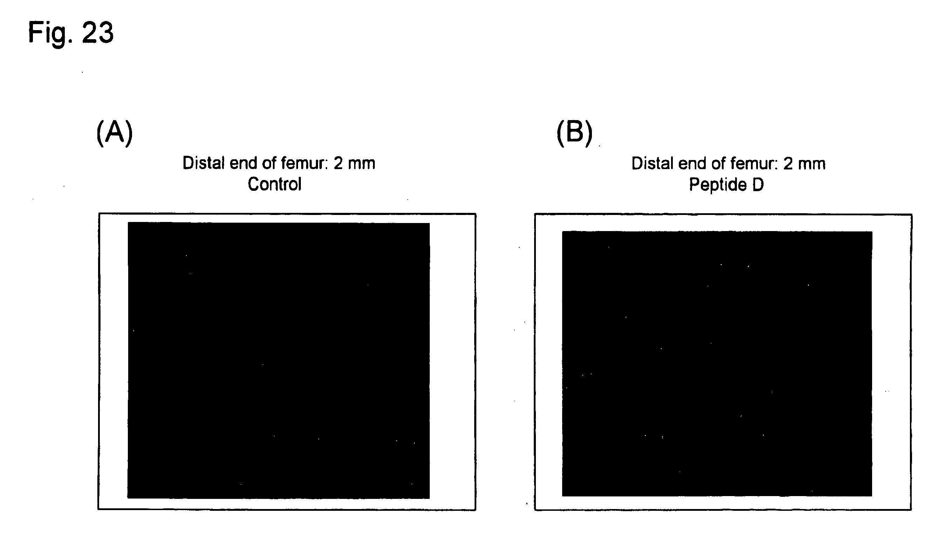

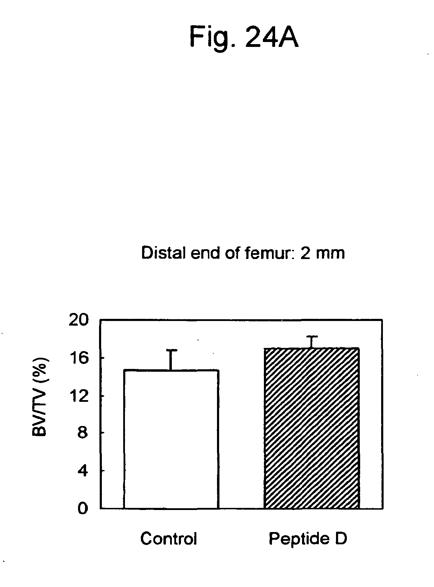

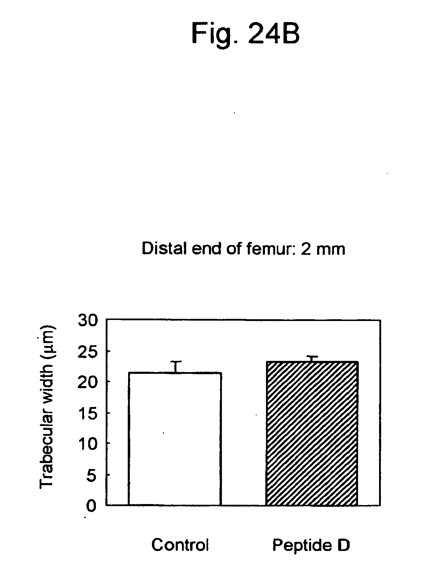

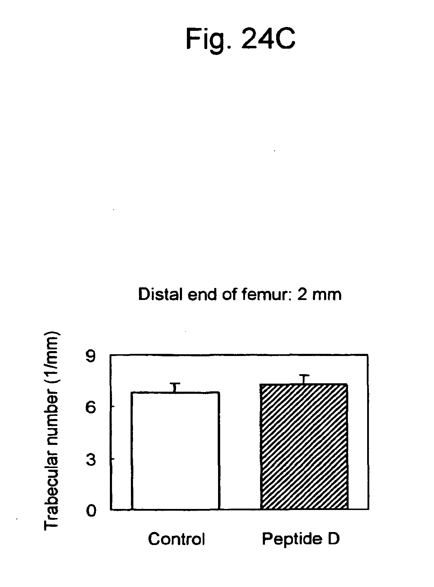

Meanwhile, a metaphyseal region rich in cancellous bone located in the proximity of a growth plate was subjected to three-dimensional structural analysis by µCT. As a result, the cancellous bone content in a site located 2 mm away from the distal end of the femur was found to have increased with the administration of peptide D (fig. 23). Thus, the cancellous bone region was subjected to trabecular structure measurement by µCT. As a result, BV/TV and trabecular width of the cancellous bone were found to have increased (figs. 24A to 24C).

In terms of actions of peptide D upon bone, bone resorption inhibition actions were confirmed based on the results of trabecular structure measurement by µCT, although weak actions were observed. However, the reason why the peptide caused an increase in the bone density of the cortical bone in the metaphyseal region as a result of administration for only 5 days cannot be explained by the above weak inhibition of bone resorption. The results suggest that the peptide has osteogenesis actions.

### Example 12: Bone morphology measurement

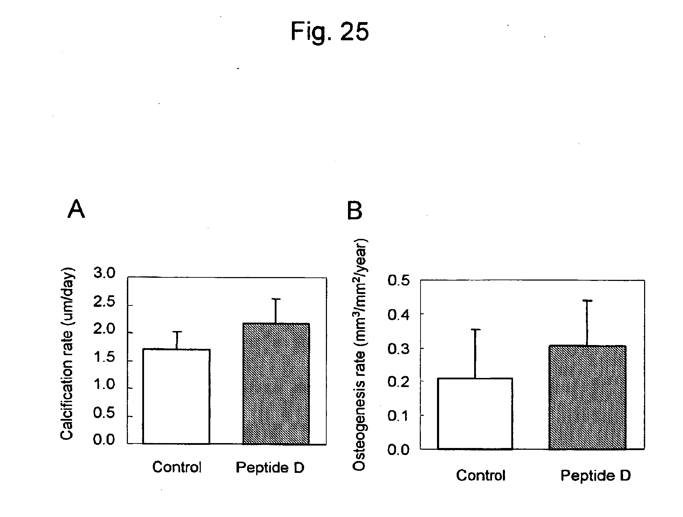

In the experiment in Example 11 in which synthetic peptide D had been administered to mice, calcein (Nacalai) mixed with 2% hydrogen carbonate/ethanol aqueous solution was intraperitoneally administered in doses of 0.01 mL/g (weight) to individual mice of each group for calcein labeling on Days 1 and 4 after the initiation of administration of synthetic peptide D. A region located 5 mm away from the ethanol-fixed distal end of the femur collected from each mouse upon dissection was embedded in a methylmethacrylate (MMA) resin so that a non-decalcified specimen was prepared. The region was identical to the region confirmed to exhibit a significant increase in bone density as a result of SXA analysis in Example 11 in the group treated with peptide D. The specimen was subjected to toluidine blue staining, followed by determination of osteoid surface area, osteoblast surface area, bone calcification surface area, calcification rate, bone formation rate, and the like. As a result, in the group treated with peptide D, increases in calcification rate and osteogenesis rate were observed (figs. 25A and 25B). A significant increase in cortical bone density was confirmed by SXA analysis and pQCT analysis in the group treated with peptide D. This is probably because the calcification rate and the bone formation rate increased *in vivo* as a result of peptide administration, causing an increase in cortical bone density.

### Example 13: Analysis of the mechanism of actions of ALP activity enhancement caused by peptide D (phosphorylation of signal molecules)

MC3T3-E1 cells mixed with 10% FBS + αMEM (SIGMA) were seeded on a 6-well plate (7.5 × 10⁴ cells/well). After 12 hours, the medium was removed therefrom and a medium containing 200 µM peptide D or 200 ng/ml BMP-2 was added thereto. The medium was removed at the respective time points shown in the relevant figures. PBS was added to the cells, and the cells were collected with a scraper (Falcon). A cell pellet was obtained via centrifugation at 1200 rpm for 5 min at 4°C. RIPA buffer (100 µL) was added to the collected pellet for dissolution of the cellular membrane. Further, the resultant was centrifuged under conditions of 14500 rpm for 25 min at 4°C. The supernatant was collected as a cell extract. A portion of the cell extract was used for quantification of the protein concentration with the use of a BCA protein assay kit (PIERCE). For SDS-PAGE, a sample buffer in an amount that was 1/4 that of the cell extract (Fermentas) was added thereto, followed by heating at 95°C for 5 min. The prepared sample was applied in an amount of 7.5 or 10 µg to 10% polyacrylamide gel (BioRad), followed by electrophoresis at 170 V for 1 hour. After electrophoresis, the electrophoresed sample was transferred to a PVDF membrane (Millipore) at 80 mA for 40 min. The membrane was shaken in a blocking solution (Nacalai) at room temperature for 1 hour and then a primary antibody was added thereto, followed by shaking at room temperature for 1 hour at 4°C for 12 hours. After removal of the primary antibody solution, the membrane was washed 3 times, followed by shaking in a blocking solution containing a secondary antibody at room temperature for I hour. After removal of the secondary antibody solution, the membrane was washed 3 times. Detection was carried out with the use of ECL plus (GE Healthcare Bioscience).

In addition, combinations of a primary antibody and a secondary antibody are given shown below.
(1) Primary antibody: phosphorylation p38 antibody (Cell signaling); secondary antibody: goat anti rabbit IgG HRP conjugated (Santa Cruz)
(2) Primary antibody: p38 antibody (Santa Cruz); secondary antibody: goat anti-mouse IgG1-HRP conjugated (SouthemBiotech)
(3) Primary antibody: β-actine antibody (Santa Cruz); secondary antibody: goat anti-rabbit IgG HRP conjugated (Santa Cruz)
(4) Primary antibody: GSK3α/β antibody (Santa Cruz); secondary antibody: goat anti-mouse IgG HRP conjugated (SIGMA)
(5) Primary antibody: phosphorylation GSK3α/β antibody (Cell signaling); secondary antibody: goat anti mouse IgG HRP conjugated (SIGMA)
(6) Primary antibody: phosphorylated smad1/5/8 (Cell signaling); secondary antibody: goat anti rabbit IgG HRP conjugated (Santa Cruz)

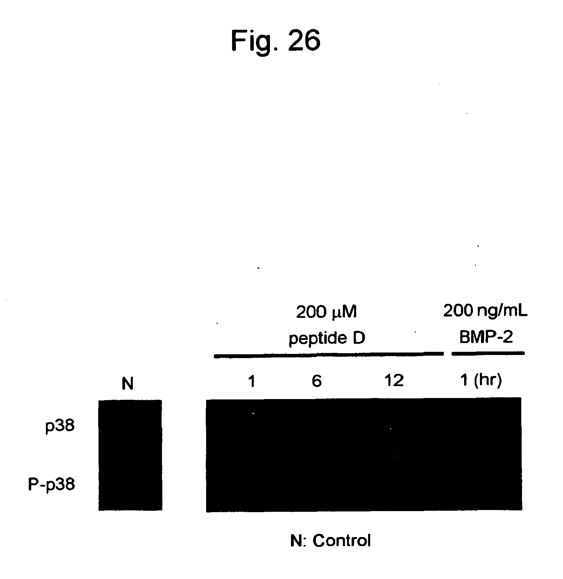

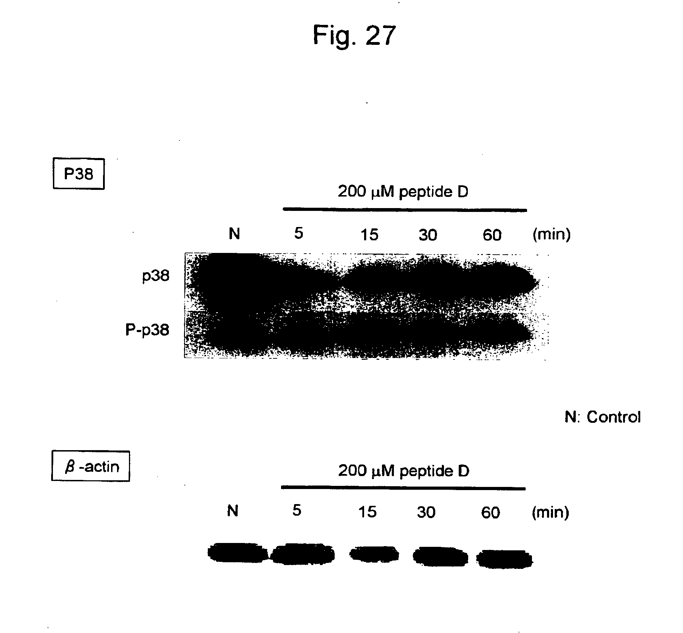

Phosphorylation of MAP kinase p38, which is involved in the main signal transduction pathway through which BMP-2 and PTH serving as known bone morphogenetic factors induce osteogenesis, was detected by Western blotting. As a result, significant p38 phosphorylation could be detected 12 hours after the addition of peptide D (fig. 26). In addition, p38 phosphorylation taking place within a short period of time after the addition of peptide D was also examined. However, no significant changes were observed (fig. 27). Meanwhile, p38 phosphorylation was observed 1 hour after the addition of BMP-2 used as a control.

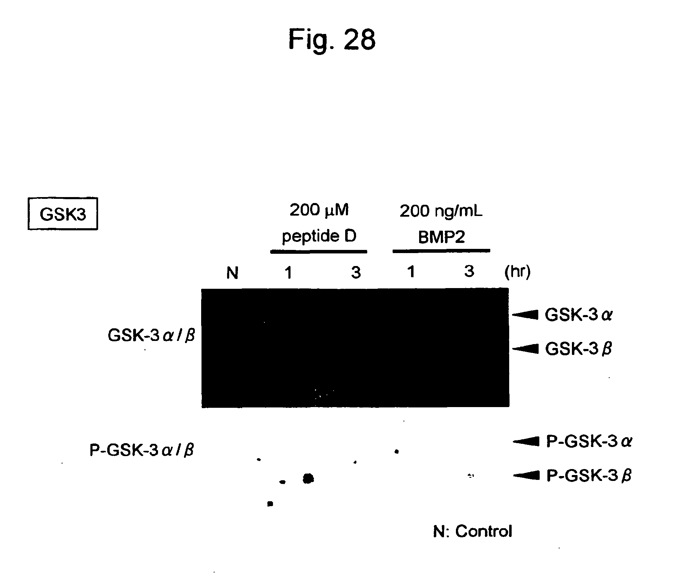

Next, phosphorylation of GSK3β used in a signal transduction pathway for Wnt serving as a bone morphogenetic factor was detected by Western blotting. Phosphorylation of GSK3β was confirmed to be induced I and 3 hours after the addition of peptide D (fig. 28). Also, phosphorylation of GSK3β was observed to a similar extent 1 and 3 hours after the addition of BMP-2 used as a control.

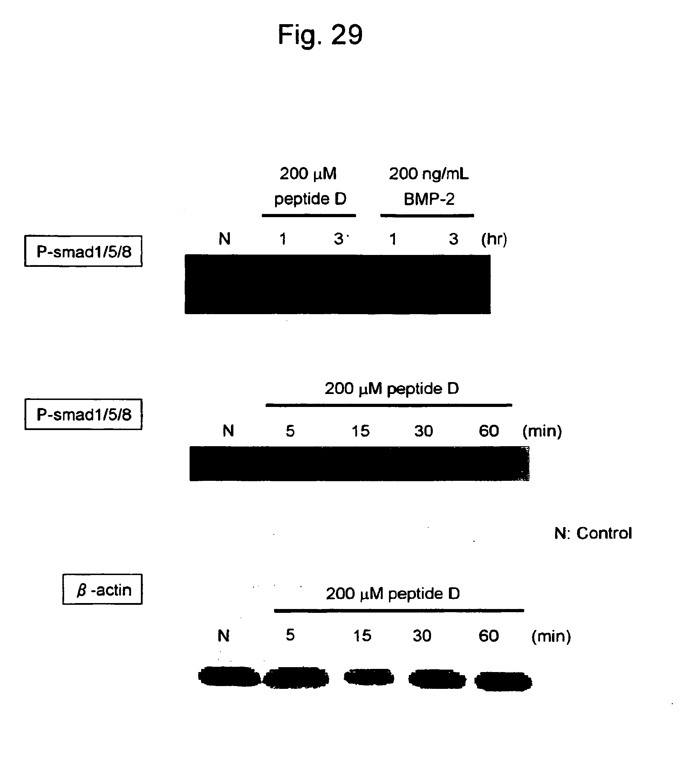

Further, phosphorylation of Smad1/5/8 used in a signal transmission pathway for BMP serving as a bone morphogenetic factor was detected by Western blotting. As a result, it was shown that Smad was phosphorylated in non-stimulated MC3T3-E1 cells. Phosphorylation of Smad1/5/8 was not induced within at least 3 hours after the addition of peptide D (fig. 29). Meanwhile, phosphorylation of Smad1/5/8 was observed 3 hours after the addition of BMP-2 used as a control. In view of the above, it was found that activation of Smad1/5/8 did not take place to an extent comparable to activation of BMP-2 at least within 3 hours after the addition of peptide D. However, significant p38 phosphorylation was observed 12 hours after the addition of peptide D to a greater extent than that observed 1 hour after the addition of BMP-2. Therefore, it was shown that peptide. D uses signals that are obviously different from BMP-2. Meanwhile, phosphorylation of GSK3β was observed 1 and 3 hours after the addition of peptide D to an extent almost comparable to that observed with the addition of BMP-2, indicating peptide D partially uses signals similar to those used by BMP-2.

### Example 14: Analysis of the mechanism of ALP activity enhancement caused by peptide D (actions of an inhibitor)

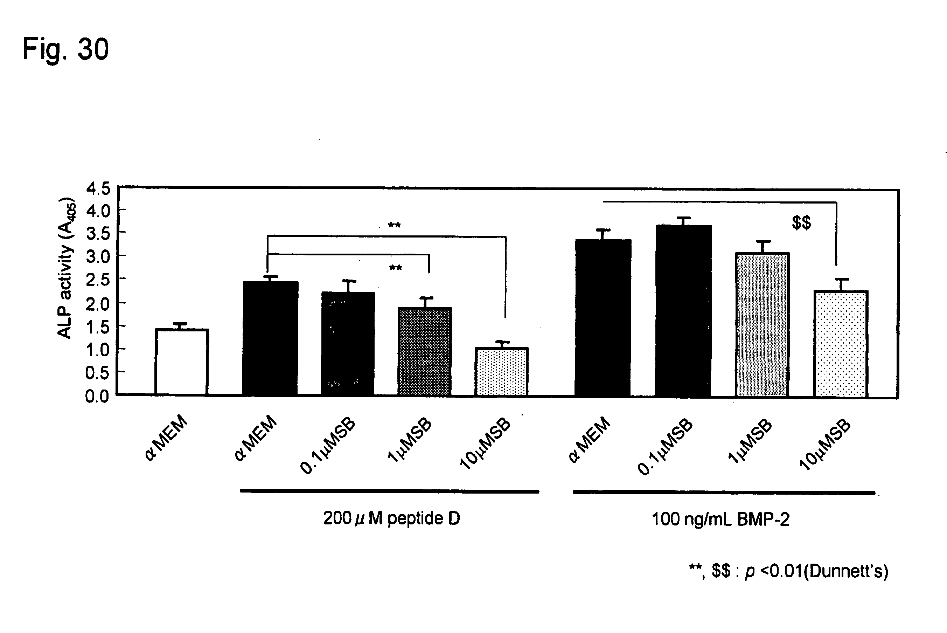

MC3T3-E1 cells mixed with 10% FBS + αMEM (SIGMA) were seeded on a 96-well plate (1.5 × 10⁴ cells/well). After 12 hours, the medium was removed therefrom and a medium containing an SB203580 p38 inhibitor (Calbiochem) was added thereto. Further, after 1 hour, 200 µM peptide D or 100 ng/ml BMP-2 was added thereto, followed by culture for 5 days. Then, ALP activity determination was carried out by the method described in Example 1. As a result, ALP activity enhancement caused by peptide D was inhibited in a SB203580-concentration-dependent manner. Significant inhibition was observed at a concentration of 1 µM and complete inhibition was observed at a concentration of 10 µM (fig. 30). On the other hand, in the case in which BMP-2 was used as a control, significant inhibition of ALP activity enhancement was not observed at a concentration of 1 µM and weak effects of inhibition were observed at a concentration of 10 µM.

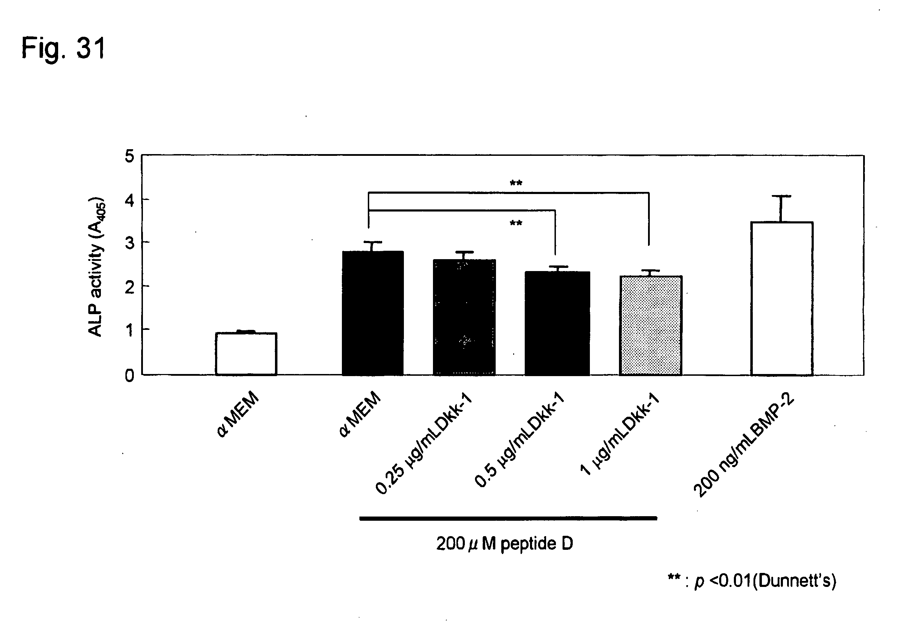

Similarly, rhDkk-1(R&D) was added as a Wnt antagonist at concentrations of 0.25, 0.5, and 1 µg/ml. After 1 hour, 200 µM peptide D was added thereto, followed by culture for 5 days. Then, ALP activity determination was carried out by the method described in Example 1. As a result, Dkk-1 was found to weakly but significantly inhibit ALP activity enhancement in a concentration-dependent manner (fig. 31).

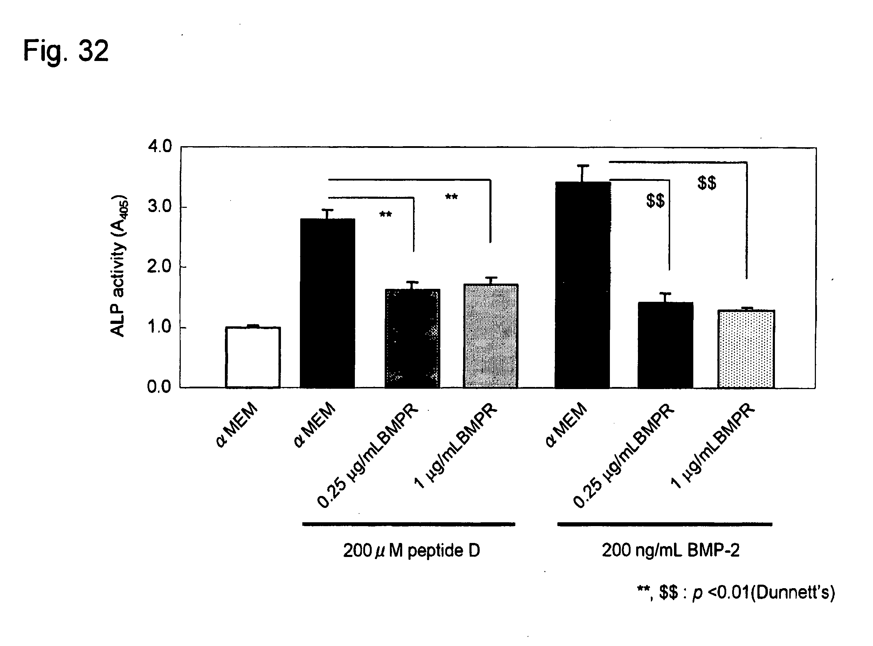

In addition, as described above, BMPR-IA (R&D) was added as a BMP antagonist at concentrations of 0.25 and 1 µg/ml. After 1 hour, 200 µM peptide D or 200 ng/ml BMP-2 was added thereto, followed by culture for 5 days. Then, ALP activity determination was carried out by the method described in Example 1. As a result, BMPR-IA was found to significantly inhibit ALP activity enhancement caused by peptide D and BMP-2 (fig. 32). Based on the above results, it was likely that actions of peptide D depend on BMP induction or BMP produced constantly and spontaneously by cells.

### Example 15: Analysis of the mechanism of ALP activity enhancement caused by peptide D (coordinated actions of peptide D and BMP-2)

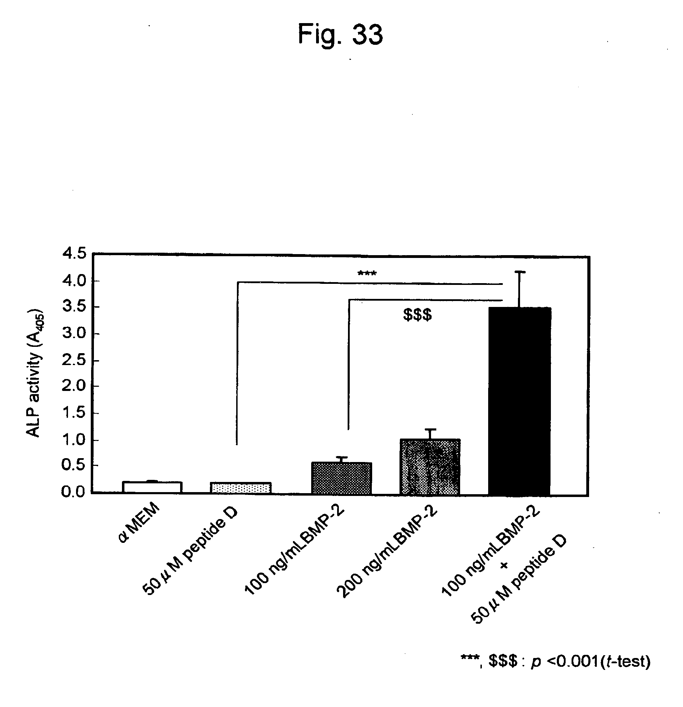

In order to examine synergistic effects of peptide D and BMP-2, ALP activity determination was carried out using C2C12 cells in which ALP activity enhancement takes place depending on the addition of BMP-2. C2C12 cells mixed with 5% FBS + αMEM (SIGMA) were seeded on a 96-well plate (1 × 10⁴ cells/ well). After 6 hours, the medium was removed therefrom. A medium containing 50 µM peptide D, a medium containing 100 ng/ml BMP-2, a medium containing 200 ng/ml BMP-2, and a medium containing a combination of 50 µM peptide D and 100 ng/ml BMP-2 were separately added thereto, followed by culture for 6 days. ALP activity determination was carried out by the method described in Example 1. As a result, in the case of the medium containing 50 µM peptide D alone, actions of ALP activity enhancement were not observed. BMP-2 enhanced ALP activity in a concentration-dependent manner. Meanwhile, in the case of the medium containing 50 µM peptide D and 100 ng/mL BMP-2, an increase in the ALP activity was confirmed at a level at least 5 times as great as that in the presence of BMP-2 alone (fig. 33). These results support the contention that the effects of peptide D described in Example 14 probably depend on BMP induction or BMP produced constantly and spontaneously by cells. C2C12 cells derived from a myoblast strain differ from MC3T3-E1 cells derived from an osteoblast strain, and therefore the ALP activity level in C2C12 cells is extremely low under ordinary culture conditions. When C2C12 cells are cultured with the addition of BMP-2, the ALP activity is enhanced, as shown in this Example, resulting in differentiation of the cells into osteoblasts. C2C12 cells are thought to be non-responding to peptide D without BMP2. Meanwhile, as shown in the Examples above, MC3T3-E1 cells exhibit marked increases in the ALP activity even with the addition of peptide D alone. This strongly suggests that actions of peptide D depend on BMP induction or BMP produced constantly and spontaneously in MC3T3-E1 cells.

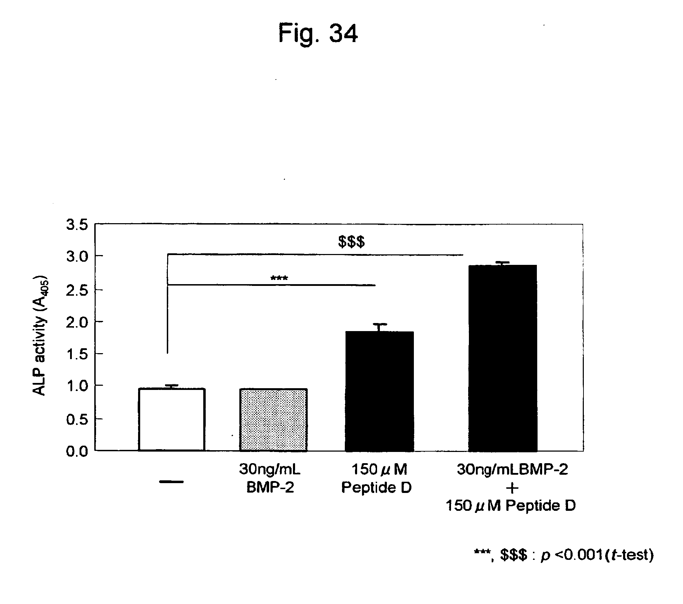

Further, coordinated actions of BMP-2 and peptide D upon MC3T3-E1 cells were also examined. MC3T3-E1 cells mixed with 10% FBS +α MEM (SIGMA) were seeded on a 96-well plate (1.5 × 10⁴ cells/ well). After 12 hours, the medium was removed therefrom, followed by ALP activity determination in the case involving the addition of 30 ng/mL BMP-2 and 150 µM peptide D. As a result, peptide D and BMP-2 exhibited additive actions of ALP activity enhancement upon MC3T3-E1 cells (fig. 34).

Synergistic effects of BMP-2 and peptide D upon C2C12 cells and MC3T3-E1 cells in terms of ALP activity are shown in fig. 33. In addition, it has been reported that the RANKL expression levels in C2C12 cells increase when BMP-2 is added to the cells (Fujita et al., Molecular Cancer 6: 71, 2007). Therefore, RANKL expression in the case involving the addition of BMP was confirmed by RT-PCR.

C2C12 cells were seeded on a 96-well plate (5 × 10³ cells/well). BMP-2 was added thereto in a manner such that the concentration thereof became 100 ng/mL after cell adhesion. After 36 hours, a TRIZOL solution (Invitrogen) (84 µL) was added to each well for cell lysis, thereby causing RNA extraction. A sample was prepared by collecting the resultants from 6 wells per group. Chloroform (Wako) (0.1 mL) was added to the sample, followed by vigorous end-over-end mixing and centrifugation under conditions of 4°C at 12000 × g for 15 minutes. The supernatant was collected in a new tube. Isopropanol (Nacalai) (0.25 mL) was added thereto, followed by end-over-end mixing. The resultant was left at room temperature for 10 min, followed by centrifugation under conditions of 4°C at 12000 × g for 10 minutes. The supernatant was removed therefrom. Then, 70% ethanol (1 mL) was added thereto, followed by centrifugation under conditions of 4°C at 12000 × g for 5 minutes. The further supernatant was removed therefrom. The RNA concentration thereof was determined, and 2 µg of the resultant was subjected to RT-PCR. RT-PCR was performed using ThermoScript RT-PCR System (Invitrogen) and random primers. After cDNA synthesis, PCR was performed using primers specific to mouse RANKL. For standardization, PCR was performed using primers specific to mouse GAPDH. The PCR primer sequences used are shown below. PCR was performed using Ex Taq^{™} Hot Start Version (Takara Bio Inc., Shiga, Japan) under the following conditions.

Initial thermal denaturation of mouse RANKL was carried out at 94°C for 2 minutes, followed by 35 cycles of 94°C for 20 seconds, 60°C for 20 seconds, and 72°C for 40 seconds and an elongation reaction at 72°C for 10 minutes. Initial thermal denaturation of GAPDH was carried out at 95°C for 3 minutes, followed by 25 cycles of 95°C for 10 seconds, 60°C for 15 seconds, and 68°C for 1 minutes and an elongation reaction at 68°C for 10 minutes.
mRANKL-F: 5'-GGCAAGCCTGAGGCCCAGCCATTT-3' (SEQ ID NO: 17)
mRANKL-R: 5'-GTCTCAGTCTATGTCCTGAACTTT-3' (SEQ ID NO: 18)
mGAPDH-F: 5'-CACCATGGAGAAGGCCGGGG-3' (SEQ ID NO: 19)
mGAPDH-R: 5'-GACGGACACATTGGGGGTAG-3' (SEQ ID NO: 20)

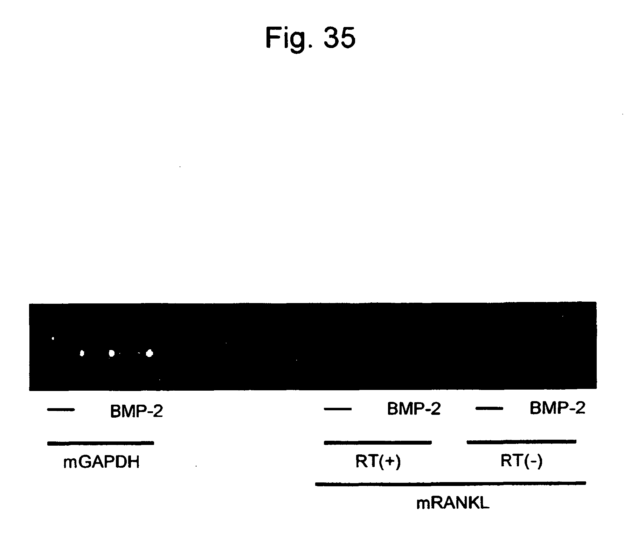

As a result, it was confirmed that RANKL expression levels in C2C12 cells increase when BMP-2 is added to the cells (fig. 35). The results suggest that BMP-2 acts on C2C12 cells to promote RANKL expression, thereby supporting peptide D action on RANKL.

### Example 16: Comparison of peptide D and peptide E in terms of activity

Peptide E (SEQ ID NO: 16) was prepared from peptide D with the substitution of a single amino acid. TRAP activity and ALP activity were determined to compare peptide D and peptide E in terms of effects of causing osteoclast differentiation and osteoblast differentiation. RAW264 cells mixed with 10% FBS + αMEM (SIGMA) were seeded on a 96-well plate (2 × 10³ cells/well). After confirmation of cell adhesion, the medium was replaced with 10% FBS + αMEM containing 10 nM GST-RANKL (Oriental Yeast Co., Ltd.). Peptide D and peptide E were separately added thereto at concentrations of 25, 50, 100, and 200 µM, followed by culture for 4 days. After the termination of culture, 100 µL acetone/ethanol was added to each well for cell fixation, followed by drying in a draft for 30 min.

In order to obtain a TRAP solution buffer at a concentration of 1.5 mg/mL, a solution was prepared by adjusting p-nitrophenyl phosphate (Nacalai) with 50 mM citric acid buffer and adding a 0.2 M sodium tartrate solution in a volume 1/10 that of citric acid buffer. The obtained TRAP solution buffer was added to each well in an amount of 100 µL, followed by incubation at 37°C for 45 min. Then, a 1N NaOH solution (50 µL) was added thereto to terminate the reaction. The OD value at 405 nm was determined for each well with a microplate reader (BMG Labtech) in the same manner as in Example 1.

Further, peptide D and peptide E were compared with each other in terms of ALP activity enhancement actions in MC3T3-E1 cells. Peptide D and peptide E were used at concentrations of 25, 50, 100, and 200 µM for culture under the conditions described in Example 14, followed by determination of ALP activity by the method described in Example 1.

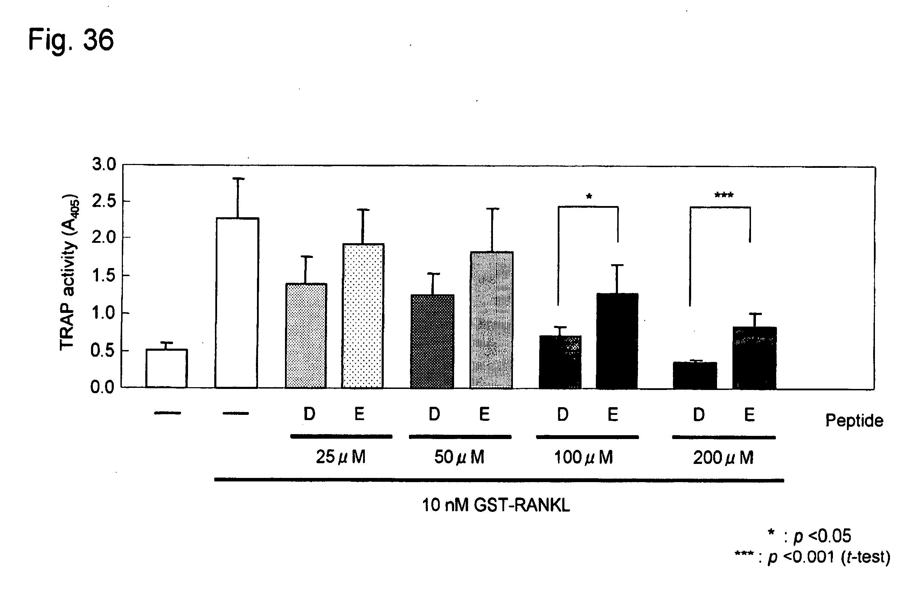

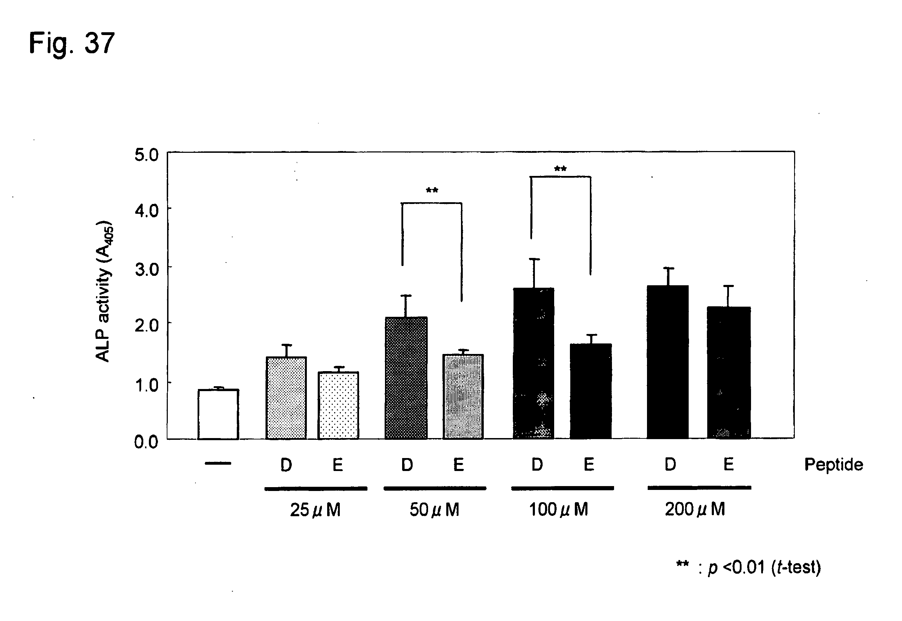

As a result, both of peptide D and peptide E were found to inhibit TRAP activity and to enhance ALP activity in a concentration-dependent manner (figs. 36 and 37). Regarding TRAP activity, peptide D exhibited significant inhibition actions compared with peptide E at concentrations 100 and 200 µM (fig. 36). In addition, regarding ALP activity, peptide D exhibited significant enhancement actions compared with peptide E at concentrations of 50 and 100 µM (fig. 37). When peptides D and E were compared with each other at concentrations at which almost comparable effects were obtained, it was found that substitution of a single amino acid resulted in reduction of TRAP activity inhibition actions to a level one-half of the initial level. Also, ALP activity enhancement actions were reduced to a level approximately 1/4 of the initial level. It has been known that such substitution of a single amino acid results in reduction in the affinity of peptide D to sRANKL to a level approximately 1/3 of the initial level (Aoki et al., J Clin Invest 116: 1525, 2006). The above results suggest that peptide D acts on RANKL so as to exhibit a neutralizing effect on TRAP activity in osteoclasts and a promoting effect on ALP activity in osteoblasts.

### Example 17: Analysis of the mechanism of increase in ALP activity by peptide D with knockdown of RANKL

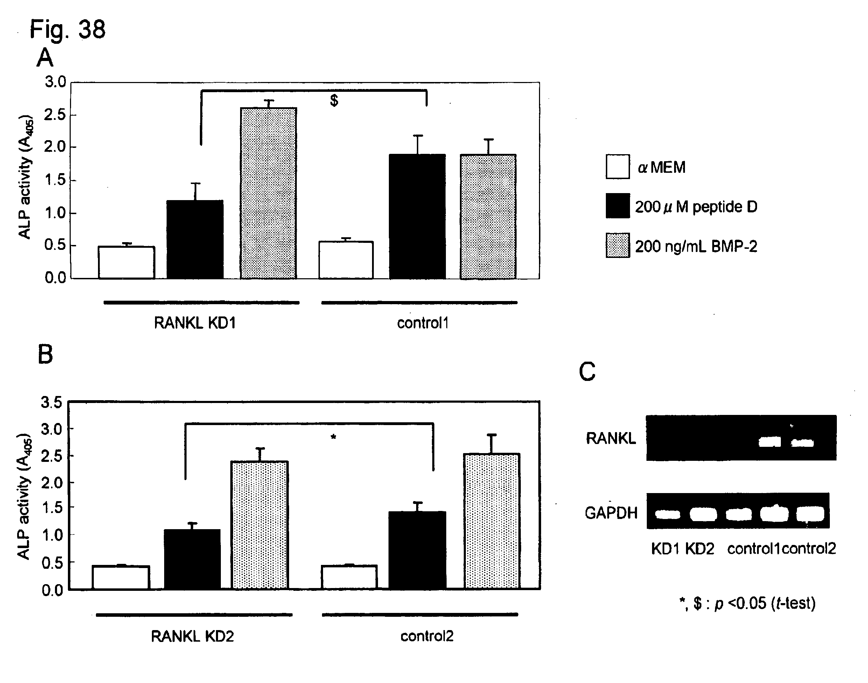

Synergistic effects of BMP-2 and peptide D on C2C12 cells are shown in fig. 33. In this regard, the RANKL expression levels in C2C12 cells were confirmed to increase upon the addition of BMP-2 (fig. 35). Then, RANKL knockdown was performed in MC3T3-E1 cells with RNAi stealth in order to examine ALP activity enhancement actions of peptide D and the influence of RANKL knockdown. OPTiMEM (Invitrogen) (20 µL) and RNA-stealth select (tnfrsf11) (Invitrogen) (1.2 pmol) were gently mixed in each well. After 5 minutes, Lipofectamine RNAiMAX (Invitrogen) (0.2 µL) was added thereto and each resultant was left at room temperature for 20 minutes. Further, MC3T3-E1 cells were seeded in each well (4 × 10³ cells), followed by culture in a CO₂ incubator for 48 hours. The culture solution was removed therefrom and αMEM containing 200 µM peptide D or 200 ng/ml BMP-2 was added thereto, followed by culture for further 5 days. Then, ALP activity determination was carried out by the method described in Example 1. In addition, for 3 types of RANKL (TNFRSF11) (KD) negative controls, similar experiments were carried out using Stealth RNAi negative universal control (Invitrogen). Cells were recovered in a necessary amount and mRNA was extracted therefrom by the method described in Example 7. Then, RANKL knockdown was confirmed using primers represented by SEQ ID NOS: 17 and 18 by RT-PCR. When KD1 and KD2 were compared with control 1 and control 2 serving as negative controls, respectively, it was revealed that the RANKL mRNA levels were significantly reduced in a specific manner while the GAPDH mRNA level was not affected (fig. 38). In the RANKL knockdown groups, including both KD1 and KD2 cases, the degree of ALP activity enhancement was significantly reduced upon the addition of peptide D. This suggests that RANKL serves as a receptor of peptide D (fig. 38). In addition, ALP activity enhancement caused by BMP-2 alone in MC3T3-E1 cells was not influenced by RANKL knockdown.

These results suggested that peptide D acts on osteoblasts and cells capable of differentiating into osteoblasts, such as osteoblast precursor cells, mesenchymal stem cells, stromal cells, and myoblasts, so as to enhance the actions of BMP or act in combination with BMP, thereby promoting osteoblast differentiation. In addition, it has been suggested that BMP promotes RANKL expression so as to act in combination with peptide D in some cells such as C2C12 cells.

### Example 18. Influence of a method of purifying synthetic peptide D upon increase in ALP activity

In general, purified synthetic peptide D contains trifluoroacetate (TFA). Therefore, it had been considered that an increase in the concentration of synthetic peptide D would cause damage to cells. In view of the above, trifluoroacetate in synthetic peptide D was substituted with acetate or hydrochloride. The obtained products were used for the experiment described below in order to obtain a peptide exhibiting a low toxicity and high ALP activity. BMP-2 (R&D) prepared with *Escherichia coli* and synthetic peptide D (50 and 150 µM) not subjected to any substitution with a salt were used as positive controls.

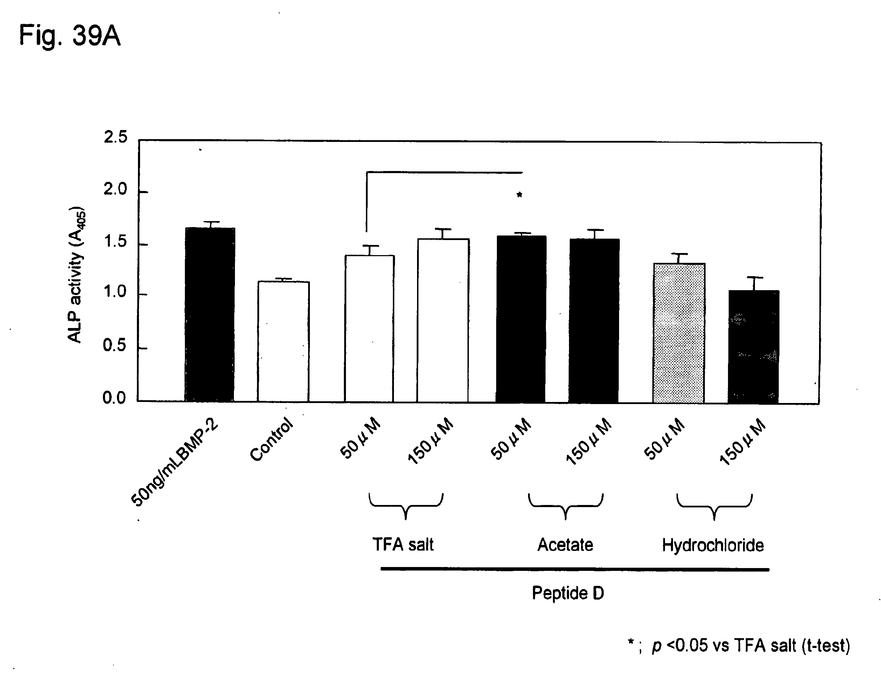

MC3T3-E1 cells (mouse osteoblast precursor cells) mixed with 10% FBS + αMEM (SIGMA) were seeded on a 96-well plate (2 x 10⁴ cells/well). After cell adhesion, the medium was removed therefrom, followed by ALP activity determination in the cases involving the addition of 50 and 150 µM peptide D (acetate and hydrochloride). 50 ng/mL BMP-2 (R&D) prepared with *Escherichia coli,* BMP-2 (R&D) produced by CHO cells, BMP-4 (R&D) produced by NS0 cells, and synthetic peptide D (50 and 150 µM) (TFA salt) were added as positive controls. In addition, the ED50 value of BMP-2 expressed in CHO cells was 40 to 200 ng/mL. The ED50 value of BMP-2 expressed in *Escherichia coli* was 0.3 to 1.0 µg/mL. On Day 5 after the addition of the individual factors, the culture supernatant was removed, followed by ALP activity determination by the method described in Example 1. As a result, peptide D in the form of acetate exhibited the highest levels of ALP activity enhancement actions in MC3T3-E1 cells (fig. 39A). Meanwhile, in the case of peptide D in the form of hydrochloride, high levels of such activity were not observed. As described above, it was found that such activity is influenced by differences in salts to be used, even if synthetic peptides contain a common amino acid sequence.

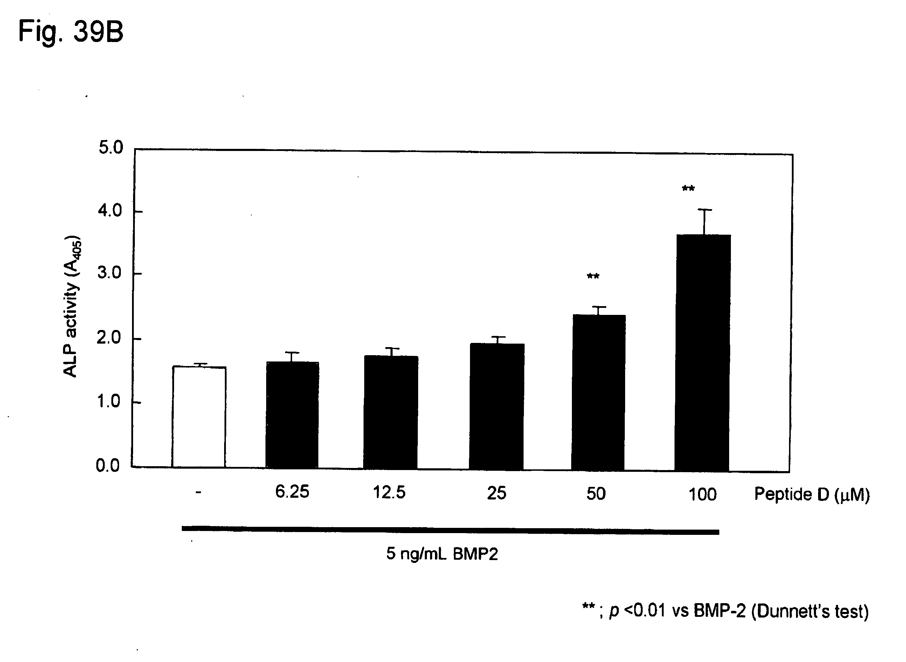

Next, synergistic effects of synthetic peptide D in the form of acetate and BMP-2 upon ALP activity were examined. MC3T3-E1 cells (mouse osteoblast precursor cells) mixed with 10% FBS + αMEM (SIGMA) were seeded on a 96-well plate (2 x 10⁴ cells/well). After cell adhesion had taken place, the medium was removed therefrom, followed by determination of ALP activity in the cases involving the mixing of 5 ng/mLBMP-2 (produced by CHO cells) with 6.25, 12.5, 25, 50, and 100 µM synthetic peptide D. On Day 5 after the addition of the individual factors, the culture supernatant was removed, followed by ALP activity determination by the method described in Example 1. As a result, increases in ALP activity were confirmed, and they depended on the dose of synthetic peptide D in the form of acetate (fig. 39B).

At the end of the experiment, synergistic effects of synthetic peptide D in the form of acetate and BMP-4 (R&D) were also examined. MC3T3-E1 cells (mouse osteoblast precursor cells) mixed with 10% FBS + αMEM (SIGMA) were seeded on a 96-well plate (2 x 10⁴ cells/well). After cell adhesion had taken place, the medium was removed therefrom, followed by the addition of 100 µM synthetic peptide D in the form of acetate to 2 ng/mL BMP-4. On Day 5 after the addition of the individual factors, the culture supernatant was removed, followed by ALP activity determination by the method described in Example 1.

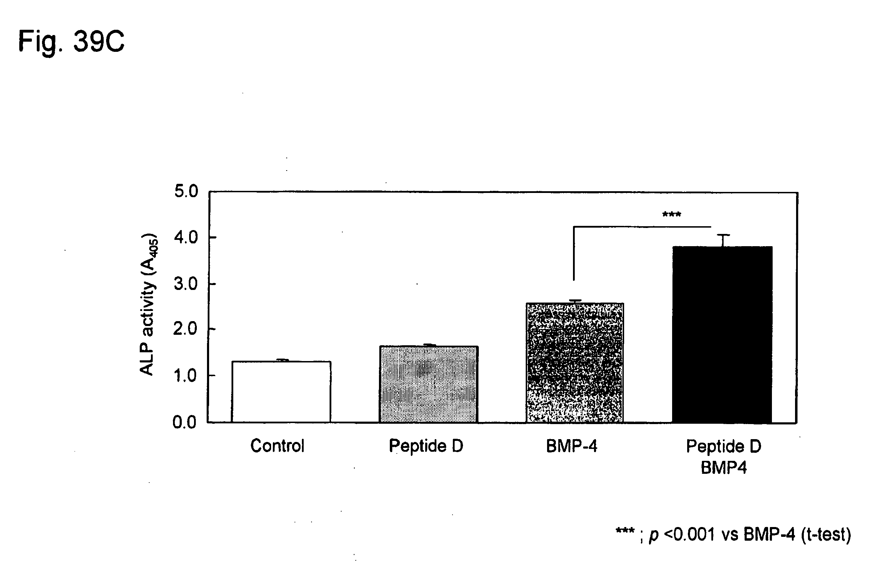

As a result of the simultaneous addition of synthetic peptide D and BMP-4, significant increases in ALP activity were confirmed. It was confirmed that synthetic peptide D in the form of acetate exhibits synergistic effects not only in the case of BMP-2 but also in the case of BMP-4 (fig. 39C).

The above results show that ALP activity can be induced to a greater extent with the use of an acetate but not a trifuoroacetic (TFA) salt that can cause damage to cells upon purification of synthetic peptide D. Also, regarding ALP activity enhancement capacity, synergistic effects were exhibited with the use of a combination of synthetic peptide D subjected to substitution with an acetate and such cases included not only BMP-2 but also BMP-4. Therefore, it was decided to use peptide D in the form of acetate in the subsequent experiments.

Example 19: Analysis of the ALP activity enhancement mechanism of an RANKL antibody (a synergistic effect of an RANKL antibody and BMP-2)

For experiments, monoclonal mRANKL antibodies ((A): clone 88227 (R&D); and (B): clone 12A668 (ALEXIS)), a control antibody (Oriental Yeast Co., Ltd.), and monoclonal mRANKL antibodies #22 (clone IKK22/5) and #36 (clone IKK36/12) were used. Antibodies #22 and #36 were assigned by Prof. Ko Okumura (School of Medicine, Juntendo University). These antibodies are described in Biochemical and Biophysical Research Communication 2006; 347, 124-132. Synthetic peptide D subjected to substitution with an acetate was used. BMP-2 produced by mammalian cells (CHO cells) (R&D) (used for C2C12 cells) and BMP-2 expressed in *Escherichia coli* (R&D) (used for mouse osteoblasts) were used as BMP-2 to be added. The manufacturer's instructions describe that BMP-2 produced by mammalian cells has activity approximately 10 times stronger than that of BMP-2 produced by *Escherichia coli.* The culture supernatant was removed on Day 5 after the addition of the individual factors, followed by ALP activity determination by the method described in Example 1.

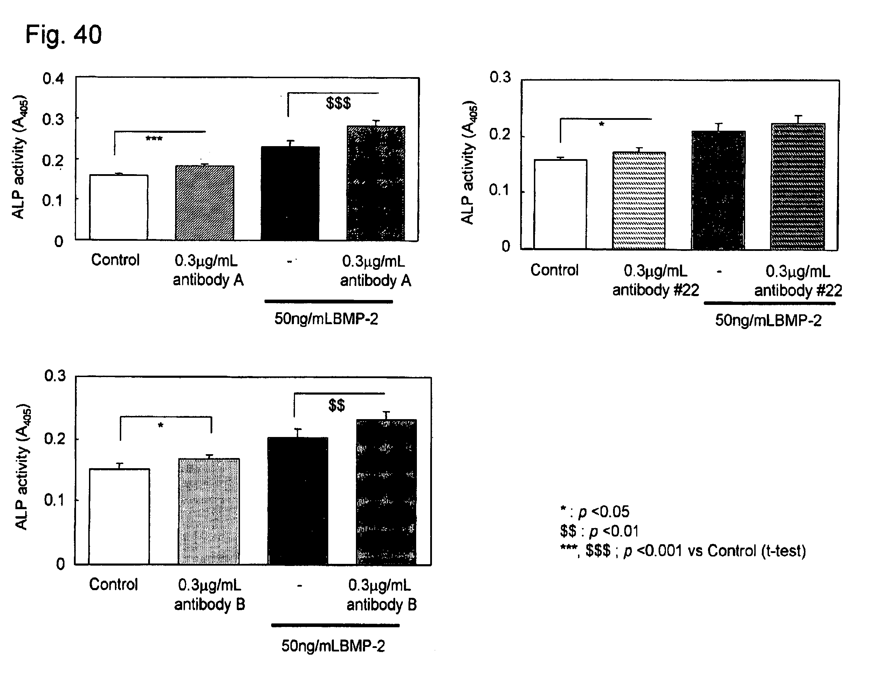

In order to examine synergistic effects of monoclonal RANKL antibodies and BMP-2, ALP activity determination was carried out using C2C12 cells, in which ALP activity enhancement is induced depending on the addition of BMP-2. C2C12 cells mixed with 5% FBS + αMEM (SIGMA) were seeded on a 96-well plate (1 × 10⁴ cells/ well). After 6 hours, the medium was removed therefrom. A medium containing a 0.3 µg/mL monoclonal RANKL antibody, a medium containing 50 ng/ml BMP-2 (expressed in CHO cells (R&D)), and a medium containing a combination of a monoclonal RANKL antibody and 50 ng/ml BMP-2 were separately added thereto. On Day 6 after the addition of the individual factors, each culture supernatant was removed therefrom, followed by ALP activity determination by the method described in Example 1. As a result, significant ALP activity enhancement action was observed in each separate medium containing a 0.3 µg/mL monoclonal antibody A, B, or #22 alone. In addition, a significant increase in ALP activity was observed in each separate medium containing a monoclonal antibody A or B to which BMP-2 had been added (fig. 40).

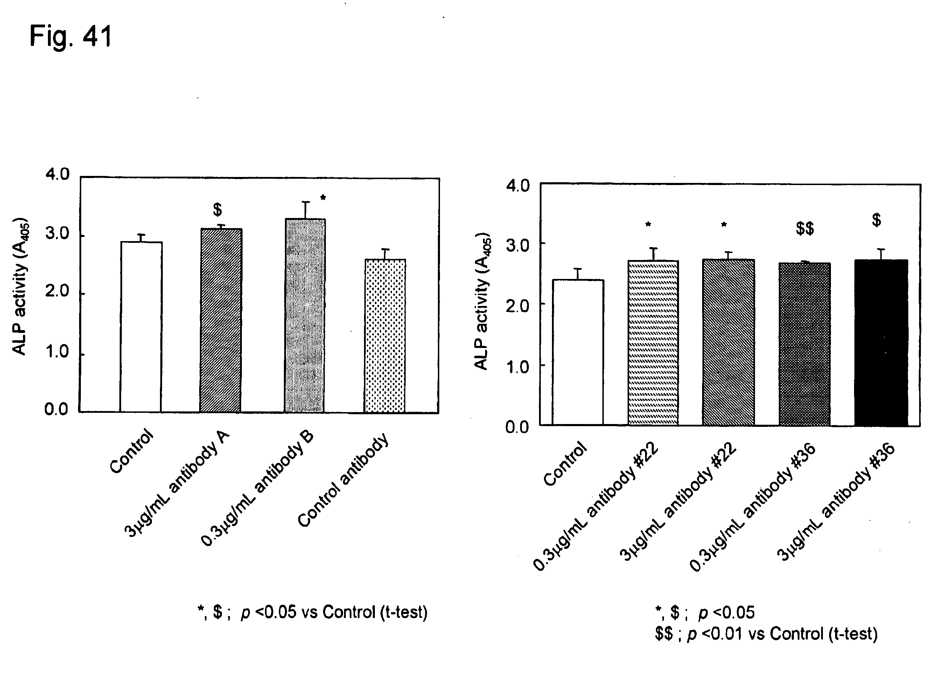

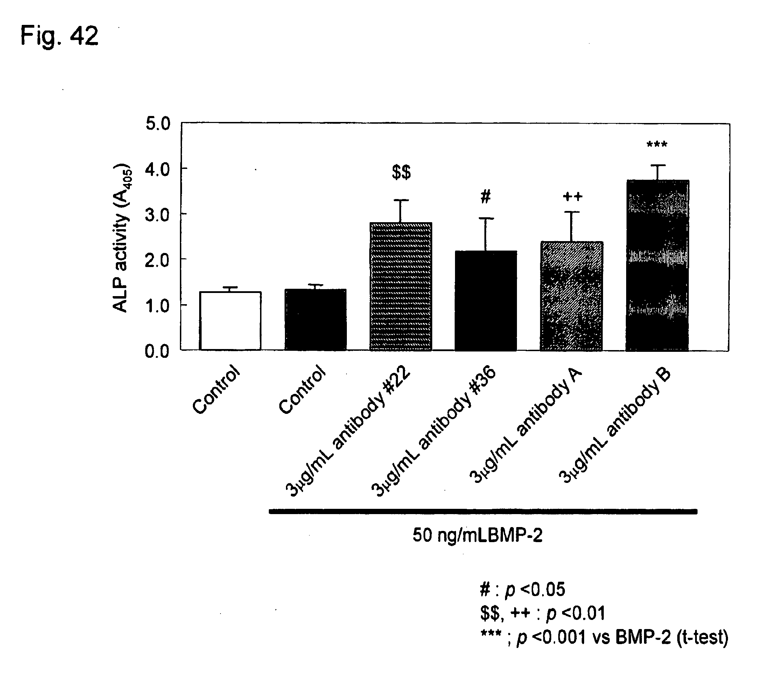

Further examination was carried out using mouse osteoblasts. Mouse osteoblasts mixed with 10% FBS + αMEM (SIGMA) were seeded on a 96-well plate (8 × 10³ cells/well). After cell adhesion had taken place, the medium was removed therefrom, followed by ALP activity determination in the cases of 0.3-3 µg/mL RANKL antibodies or in the cases of RANKL antibodies mixed with 50 ng/mL BMP-2 (expressed in *Escherichia coli* (R&D)). On Day 4 after the addition of the individual factors, each culture supernatant was removed therefrom, followed by ALP activity determination by the method described in Example 1. As a result, a small but significant increase in ALP activity was confirmed in mouse osteoblasts with the use of monoclonal antibody A at a concentration of 3 µg/mL and monoclonal antibody B at a concentration of 0.3 µg/mL. In the case of the control antibody (Oriental Yeast Co., Ltd.) used as a negative control, such action was not observed. In addition, in the cases of monoclonal antibodies #36 and #22, a significant increase in ALP activity was confirmed at concentrations of 0.3 and 3 µg/mL (fig. 41). Further, a coordinated action of BMP-2 and a monoclonal RANKL antibody upon ALP activity were confirmed in the cases of all of the above monoclonal antibodies (fig. 42). As described above, all anti-RANKL monoclonal antibodies used in the experiments exhibited the ALP activity enhancement action in mouse osteoblasts, although the degree of enhancement differed depending on the antibodies. In addition, the antibodies exhibited synergistic ALP activity enhancement action with BMP-2.

### Example 20: GST-RANKL action in relation to synergistic effects of peptide D (acetate) and BMP-2

Simultaneous addition of BMP-2 and peptide D in the form of acetate to MC3T3-E1 cells resulted in synergistic effects of ALP activity enhancement. The influence of the addition of GST-RANKL upon such effects of ALP activity enhancement was examined.

The RANKL antibodies described in Example 19 were used. GST-RANKL and GST used were those produced by Oriental Yeast Co., Ltd. In addition, BMP-2 (produced by CHO cells) (R&D) was used. Synthetic peptide D subjected to substitution with an acetate was used.

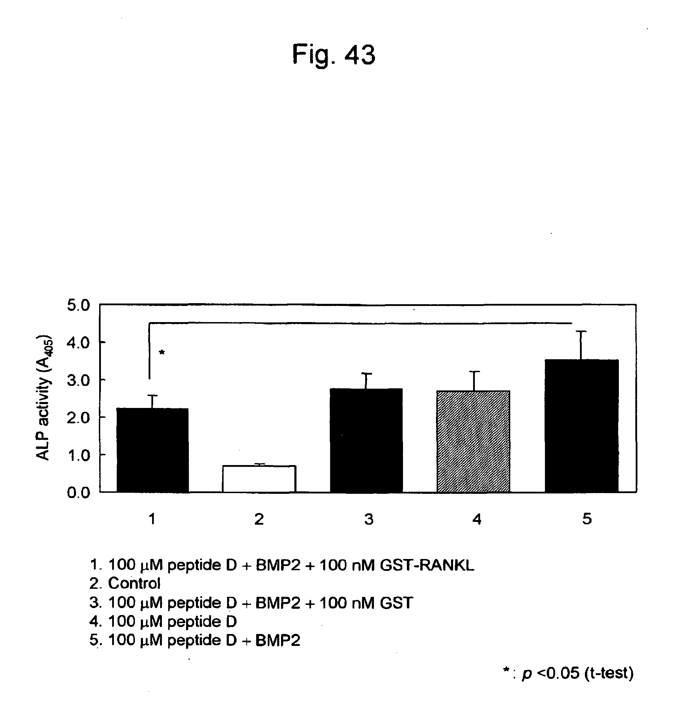

MC3T3-E1 cells (mouse osteoblast precursor cells) mixed with 10% FBS + αMEM (SIGMA) were seeded on a 96-well plate (2 × 10⁴ cells/well). After cell adhesion had taken place, the medium was removed therefrom. For replacement, a medium containing 100 µM peptide D in the form of acetate, a medium containing 100 µM peptide D in the form of acetate mixed with 5 ng/mL BMP-2, and a medium containing a mixture of peptide D and BMP-2 to which 100 nM GST-RANKL or GST had been added were added thereto. On Day 5 after the addition of the individual factors, each culture supernatant was removed therefrom, followed by ALP activity determination by the method described in Example 1. As a result, GST-RANKL was found to have significantly inhibited synergistic effects of ALP activity enhancement of peptide D (acetate) and BMP-2 in MC3T3-E1 cells (fig. 43). Meanwhile, a significant degree of inhibition was not observed in the group to which GST had been added. These results indicate that peptide D acts on RANKL expressed on MC3T3-E1 cells so as to cause ALP activity enhancement. GST-RANKL was thought to have been antagonistic to RANKL on the cell membrane, thereby inhibiting the action of peptide D.

### Example 21: Proliferative response in mouse osteoblasts treated with peptide D or an RANKL antibody

It was described in Examples 19 and 20 that the ALP activity enhancement action can be observed with the simultaneous addition of peptide D or an RANKL antibody and BMP-2. Peptide D and an RANKL antibody capable of exhibiting synergistic effects with BMP-2 were examined as to whether they would induce the proliferative response in mouse osteoblasts.

Synthetic peptide D subjected to substitution with an acetate was used. In addition, BMP-2 (produced in CHO cells) (R&D) was used.

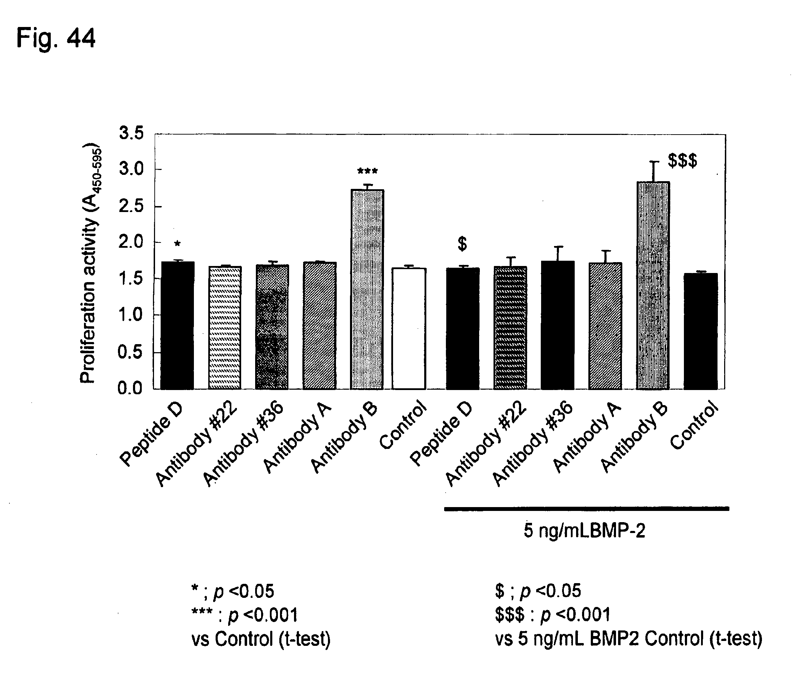

Mouse osteoblasts mixed with 10% FBS + αMEM (SIGMA) were seeded on a 96-well plate (2 × 10³ cells/well). After cell adhesion had taken place, the medium was removed therefrom. A medium containing 100 µM peptide D (acetate), a medium containing any one of the RANKL antibodies (3 µg/mL) described in Example 19, and a medium containing any one of the above factors mixed with 5 ng/mL BMP-2 were separately added thereto. After 72-hour culture, WST-1 (Roche) was added thereto in an amount 1/10 that of each medium, followed by incubation at 37°C for 3 hours. Then, the OD value (450 nm) for each well was determined with a microplate reader (BMG Labtech) (reference wavelength: 595 nm). As a result, regardless of the presence or absence of BMP-2, peptide D and RANKL antibody B were found to have promoted mouse osteoblast proliferation (fig. 44). However, peptide D showed weak effects and the other RANKL antibodies (#22, #36, and A) did not exhibit proliferation promotion effects.

Based on the above, it was found that there are anti-RANKL monoclonal antibodies capable of promoting mouse osteoblast proliferation and those incapable of promoting mouse osteoblast proliferation, depending on differences in the epitopes recognized by such antibodies. Meanwhile, as described in Example 19, it was found that even anti-RANKL monoclonal antibodies incapable of promoting mouse osteoblast proliferation could exhibit ALP activity enhancement action in mouse osteoblasts; that is to say, differentiation promotion action. In short, it was possible to cause osteoblast proliferation or differentiation by appropriately selecting an anti-RANKL monoclonal antibody capable of recognizing a different epitope.

### Example 22: Analysis of the peptide D action mechanism on MC3T3-E1 cells (DNA microarray)

MC3T3-E1 cells mixed with 10% FBS + αMEM (SIGMA) were seeded on a 10 cm dish (2 × 10⁵ cells/ well). After 12 hours, the medium was removed therefrom. A medium containing 200 µM peptide D (acetate) or 150 ng/ml BMP-2 (produced in *Escherichia coli* (R&D)) was added thereto. The medium was partially removed at 12 and 96 hours, at which time TRIZOL solution (Invitrogen) (3 mL) was added to each well for cell lysis. Then, total RNA extraction was carried out by the method described in Example 15. Each extracted total RNA (2 µg) was subjected to DNA microarray analysis (Mouse Genome 430 2.0 Affymetrix). In addition, scanning was performed with a GeneChip Scanner 3000 (Affymetrix 690036) and digitalization was performed with the Gene Chip Operating Software ver. 1.4.

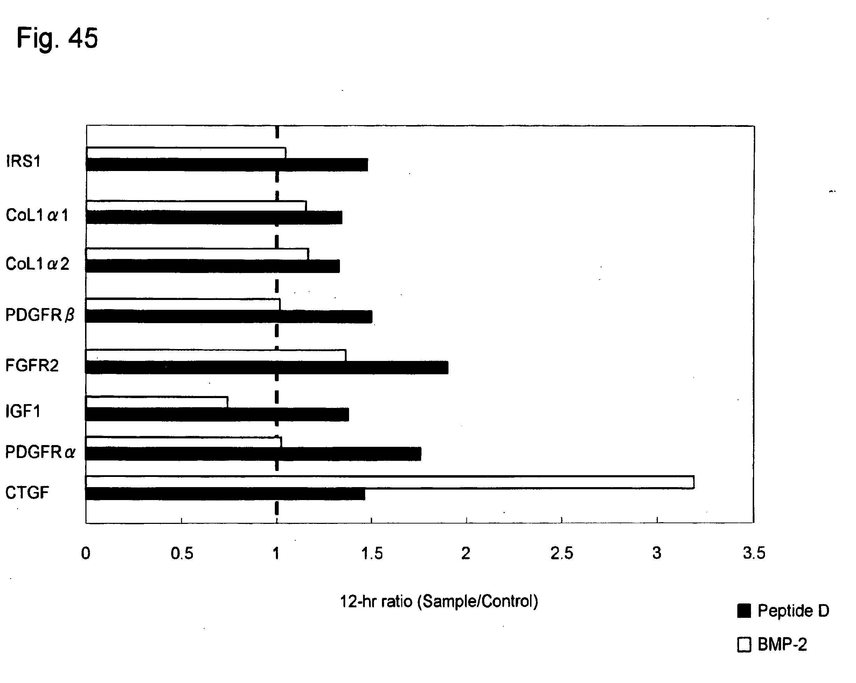

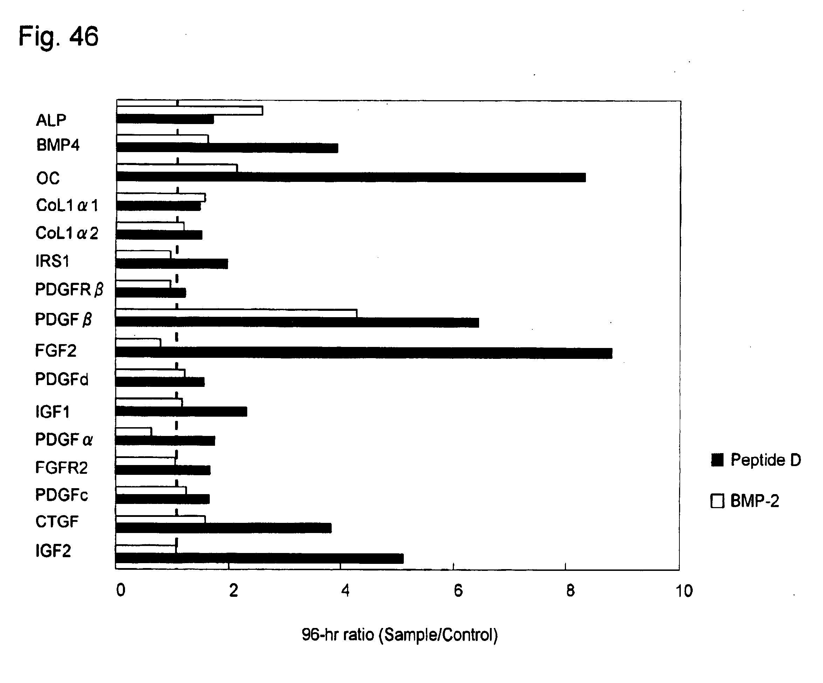

As a result, at 12 hours after the addition of peptide D, the gene expression levels of IRS-1, IGF-1, FGF receptor 2, PDGF receptor β, PDGF receptor α, CTGF, and type I collagen α1 and α2 chains were found to have increased significantly (fig. 45). Further, the gene expression levels of IGF-2, ALP, BMP-4, OC (osteocalcin), FGF2, PDGFc, PDGFα, and PDGFβ had increased significantly after 96 hours (fig. 46). Type I collagen, ALP, and osteocalcin are known as osteoblast markers. In Example 7, peptide D caused increases in the gene expression levels of type I collagen and ALP in human mesenchymal stem cells. However, also in MC3T3-E1 cells, the expression levels of the two genes were found to have increased. Further, the expression level of the osteocalcin (OC) gene known as a late-phase osteoblast differentiation marker increased sharply. Therefore, it is thought that peptide D caused MC3T3-E1 cells to differentiate into osteoblasts.

### Example 23: Verification based on DNA array analysis by RT-PCR

Among the genes that had been found to exhibit increased expression signals with the use of a DNA microarray, alkaline phosphatase (ALP), type I collagen (Col 1), and osteocalcin (OC), which are known as osteoblast markers, were subjected to RT-PCR for confirmation. Total RNA of each gene collected in Example 22 (2 µg) was subjected to RT-PCR. RT-PCR was performed using the ThermoScript RT-PCR System (Invitrogen) and random primers.

After cDNA synthesis, PCR was performed using primers specific to mouse alkaline phosphatase (mALP), mouse type I collagen α1 (mColI), and mouse osteocalcin (mOC). For standardization, PCR was performed using primers specific to mouse GAPDH. The used PCR primer sequences are shown below. PCR was performed using Ex TaqTM Hot Start Version (Takara Bio Inc., Shiga, Japan) under the following conditions. Alkaline phosphatase (mALP) was subjected to initial thermal denaturation at 95°C for 3 minutes, followed by 28 cycles of 95°C for 10 seconds, 60°C for 15 seconds, and 68°C for 1 minute and an elongation reaction at 68°C for 10 minutes. Type I collagen α1 (mcolI) was subjected to initial thermal denaturation at 93°C for 3 minutes, followed by 20 cycles of 94°C for 30 seconds, 58°C for 30 seconds, and 72°C for 15 seconds and an elongation reaction at 72°C for 10 minutes. Osteocalcin (mOC) was subjected to initial thermal denaturation at 95°C for 3 minutes, followed by 28 and 30 cycles of 94°C for 30 seconds, 58°C for 30 seconds, and 72°C for 15 seconds and an elongation reaction at 72°C for 10 minutes. GAPDH was subjected to initial thermal denaturation at 95°C for 3 minutes, followed by 20 cycles of 94°C for 10 seconds, 58°C for 15 seconds, and 68°C for 1 minute and an elongation reaction at 68°C for 10 minutes. PCR primer sequence
mALP-F: 5'-CCAAGCAGGCTCTGCATGAA-3' (SEQ ID NO: 21)
mALP-R: 5'-GCCAGACCAAAGATGGAGTT-3' (SEQ ID NO: 22)
mOC-F: 5'-TCTGACAAAGCCTTCATGTCC-3' (SEQ ID NO: 23)
mOC-R: 5'-AAATAGTGATACCATAGATGCG-3' (SEQ ID NO: 24)
mCol1-F: 5'-CCTGGTAAAGATGGTGCC-3' (SEQ ID NO: 25)
mCol1-R: 5'-CACCAGGTTCACCTTTCGCACC-3' (SEQ ID NO: 26)
mGAPDH-F: 5'-CACCATGGAGAAGGCCGGGG-3' (SEQ ID NO: 19)
mGAPDH-R: 5'-GACGGACACATTGGGGGTAG-3' (SEQ ID NO: 20)

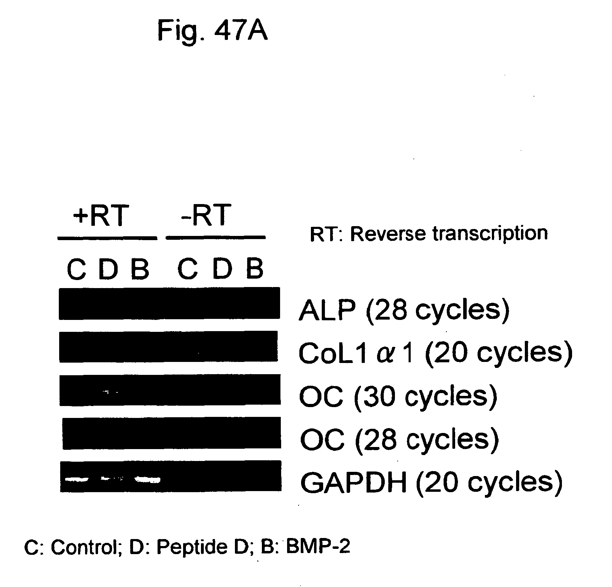

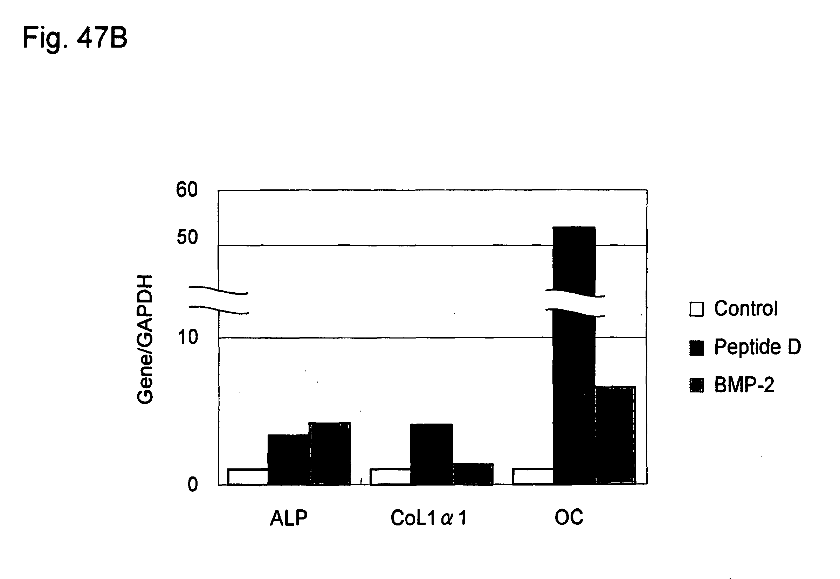

A portion of each reaction solution was subjected to agarose gel electrophoresis, followed by staining with an ethidium bromide solution. As a result, as in the cases of the DNA microarray analysis results, it was confirmed that peptide D and BMP-2 had caused significant increases in the gene expression levels of ALP, Col1, and OC (fig. 47A). Each expression intensity was digitalized and a graph was created by designating each control gene expression level as 1. As a result, significant increases in the gene expression levels were more obviously observed than those confirmed by DNA microarray analysis (fig. 47B).

Accordingly, also based on changes in the gene expression levels observed via RT-PCR, peptide D was confirmed to cause differentiation of MC3T3-E1 cells into osteoblasts.

### Example 24: In vivo analysis of osteogenesis markers with the administration of synthetic peptide

### Reagent

Synthetic peptide D subjected to substitution with an acetate was used in this experiment. The synthetic peptide D was dissolved in PBS at a concentration of 1 mg/mL. PBS was administered to a control group.

### Experimental animals

C57BL/6CrjCrlj mice were purchased from KITAYAMA LABES Co., Ltd. The mice used were C57BL/6CrCrlj inbred mice characterized in that they experience a small decrease in cellular immune competence caused by aging. The mice were preliminarily raised in an environment at a temperature of 23°C ± 3°C and a humidity of 50% ± 30% for 1 week. Lighting time was 8:00 to 20:00.

During the experiments, all mice were fed with MF (Oriental Yeast Co., Ltd.).

A control group (n = 6) and a group treated with peptide D (n = 7) were raised in cages.

### Administration method and period

Subcutaneous administration of synthetic peptide D in the form of acetate was carried out in a dose of 10 mg/kg at 8:00, 14:00, and 20:00 (three times daily) for 5 days. PBS was administered to the control group. Mice were dissected 12 hour after the completion of administration for 5 days and subjected to exsanguination. Thereafter, the femur and the tibia were collected from each mouse. The whole blood of each mouse was left at room temperature for 1 hour and then centrifuged under conditions of 5000 rpm and 4°C for 5 min. The serum was collected in a new tube. The femur and the tibia of each mouse were fixed with cold 70% ethanol. Muscles and the like were carefully removed from each tibia. Then, each tibia was washed with PBS and cut into 1-mm fragments with scissors, followed by freezing with liquid nitrogen. A TRIZOL solution (Invitrogen) (1 mL) was added to each frozen tibia, followed by homogenization by Polytron. Extraction of total RNA was carried out by the method described in Example 7. Each obtained sample of total RNA was dissolved in DEPC water (50 µL). Total RNA (500 ng) extracted for each mouse was subjected to RT-PCR by the method described in Example 7. RT-PCR was performed using the ThermoScript RT-PCR System (Invitrogen) and random primers.

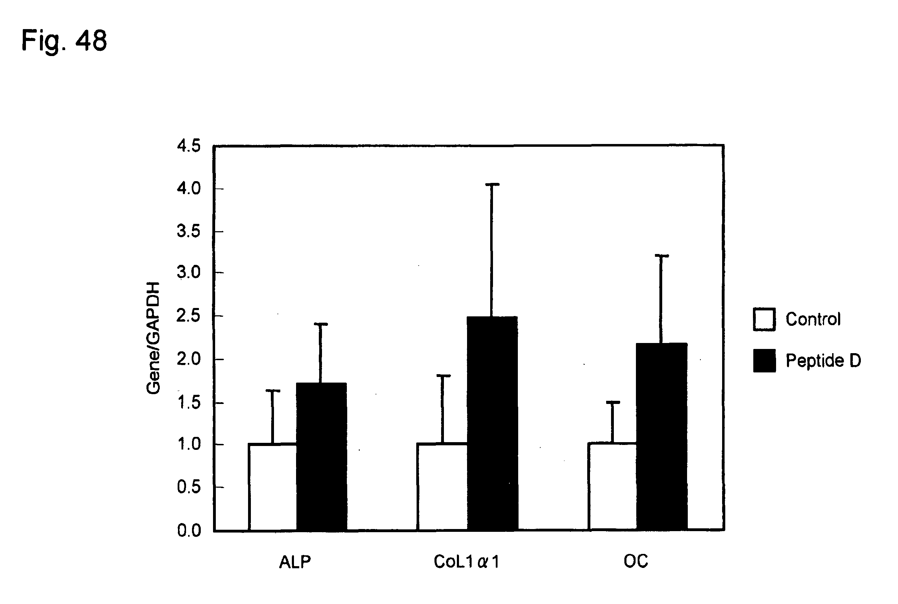

After cDNA synthesis, as in the case of Example 23, PCR was performed using primers specific to mouse alkaline phosphatase (mALP), mouse type I collagen αI chain (mCollagen αI), and mouse osteocalcin (mOC). For standardization, PCR was performed using primers specific to mouse GAPDH. PCR conditions were the same as those in Example 23. The number of cycles for denaturation was 23 cycles for mOC, 20 cycles for mCollagen αI, and 28 cycles for mALP. In the case of data for mGAPDH, the figure was 23 cycles. Samples obtained after PCR reaction were subjected to electrophoresis with 1% agarose gel. Formation of specific bands was confirmed under UV light with the use of ethidium bromide. The obtained images were analyzed with a CSAnalyzer and standardized based on the GAPDH expression levels. As a result, an increase in the expression level of each factor was observed in the group treated with peptide D (fig. 48). Based on the above, it was also confirmed that the expression of the osteoblast differentiation marker gene was caused by the administration of peptide D in the case of mouse bone tissue.

### Example 25: Analysis of the peptide D action mechanism on mouse tibia (DNA microarray)

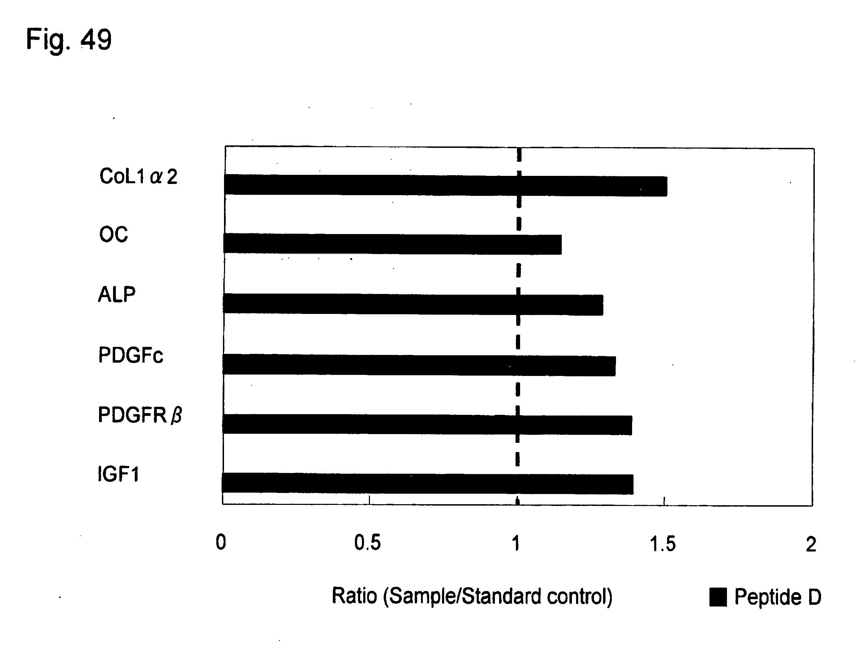

Total RNA (2 µg) extracted from the tibia of each mouse described in Example 24 was used for a DNA microarray (each group: n = 2). According to a standard method, DNA microarray analysis (Mouse Genome 430 2.0 Affymetrix) was carried out. In addition, scanning was performed with a GeneChip Scanner 3000 (Affymetrix 690036) and digitalization was performed using the Gene Chip Operating Software ver. 1.4. As a result, OC, ALP, type I collagen α2 chain (CoL1 α2), a platelet-derived growth factor C (PDGFc) peptide, a platelet-derived growth factor receptor (PDGFRβ), and an insulin-like growth factor (IGF-1) were found to exhibit significantly higher signals in mouse tibia samples of the group treated with peptide D than those exhibited in a standard sample (fig. 49). Increases in bone morphogenetic factors such as OC and ALP were confirmed not only in mouse osteoblast precursor cells to which peptide D had been added but also in mouse tibias to which peptide D had been administered, indicating that peptide D can also enhance bone formation *in vivo.*

### Example 26: Verification 2: DNA array analysis by RT-PCR

ALP, CoL1, and OC were subjected to RT-PCR as in Example 23 and verification data were obtained using the DNA microarray. In order to obtain more detailed verification data, a variety of growth factors and their receptors were subjected to RT-PCR in order to confirm signal increases observed therein via DNA microarray analysis with the use of cDNA of each group obtained from MC3T3-E1 cells in Example 23.

Each cDNA was synthesized by the method described in Example 23. After synthesis, PCR was performed using primers specific to mouse bone morphogenetic protein 4 (mBMP-4), a mouse connective tissue growth factor (mCTGF), a mouse platelet-derived growth factor (mPDGFc peptide) and a receptor thereof (mPDGFRβ), mouse fibroblast growth factor 2 (mFGF2) and a receptor thereof (mFGFR2), insulin-like growth factor 2 (mIGF-2), and insulin receptor substrate (mIRS-1). For standardization, PCR was performed using primers specific to mouse GAPDH. The used PCR primer sequences are shown below. PCR was performed using Ex TaqTM Hot Start Version (Takara Bio Inc., Shiga, Japan) under the following conditions. BMP-4 was subjected to initial thermal denaturation at 95°C for 3 minutes, followed by 31 cycles of 95°C for 10 seconds, 58°C for 15 seconds, and 72°C for 30 seconds and an elongation reaction at 68°C for 10 minutes. CTGF was subjected to initial thermal denaturation at 95°C for 3 minutes, followed by 31 cycles of 95°C for 10 seconds, 58°C for 15 seconds, and 72°C for 30 seconds and an elongation reaction at 72°C for 10 minutes. FGF2 was subjected to initial thermal denaturation at 95°C for 3 minutes, followed by 34 cycles of 95°C for 10 seconds, 58°C for 15 seconds, and 72°C for 30 seconds and an elongation reaction at 72°C for 10 minutes. FGFR2 was subjected to 25 cycles of thermal denaturation (95°C for 10 seconds, 58°C for 15 seconds, and 72°C for 30 seconds) and an elongation reaction at 72°C for 10 minutes. IGF-2 was subjected to 31 cycles of thermal denaturation (95°C for 10 seconds, 58°C for 15 seconds, and 72°C for 30 seconds) and an elongation reaction at 72°C for 10 minutes. PDGFc peptide, PDGFRβ3, and IRS-1 were subjected to 23 cycles of thermal denaturation (95°C for 10 seconds, 58°C for 15 seconds, and 72°C for 30 seconds) and an elongation reaction at 72°C for 10 minutes. GAPDH was subjected to initial thermal denaturation at 95°C for 3 minutes, followed by 20 cycles of 94°C for 10 seconds, 58°C for 15 seconds, and 68°C for 1 minute and an elongation reaction at 68°C for 10 minutes. A portion of each reaction solution was subjected to electrophoresis with 2% agarose gel, followed by staining with an ethidium bromide solution.

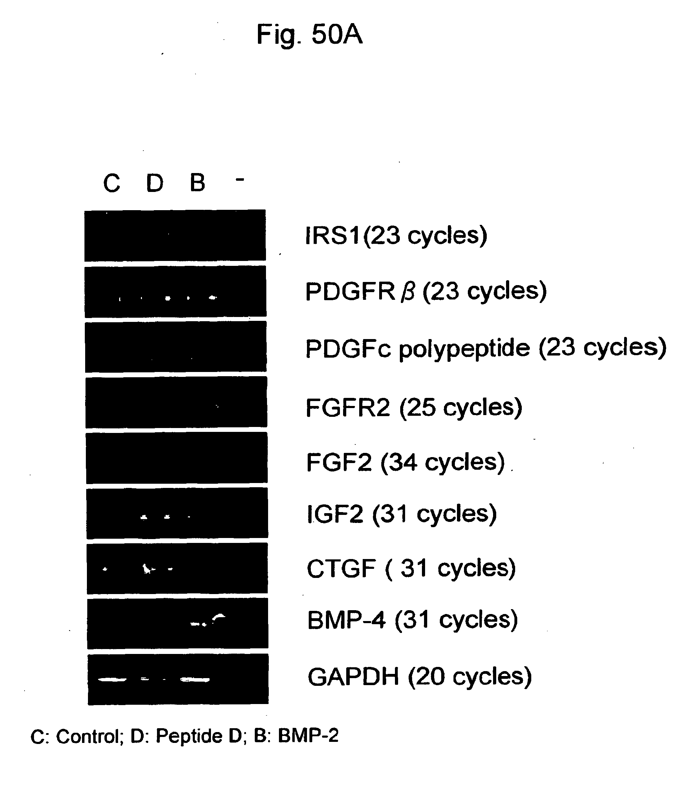

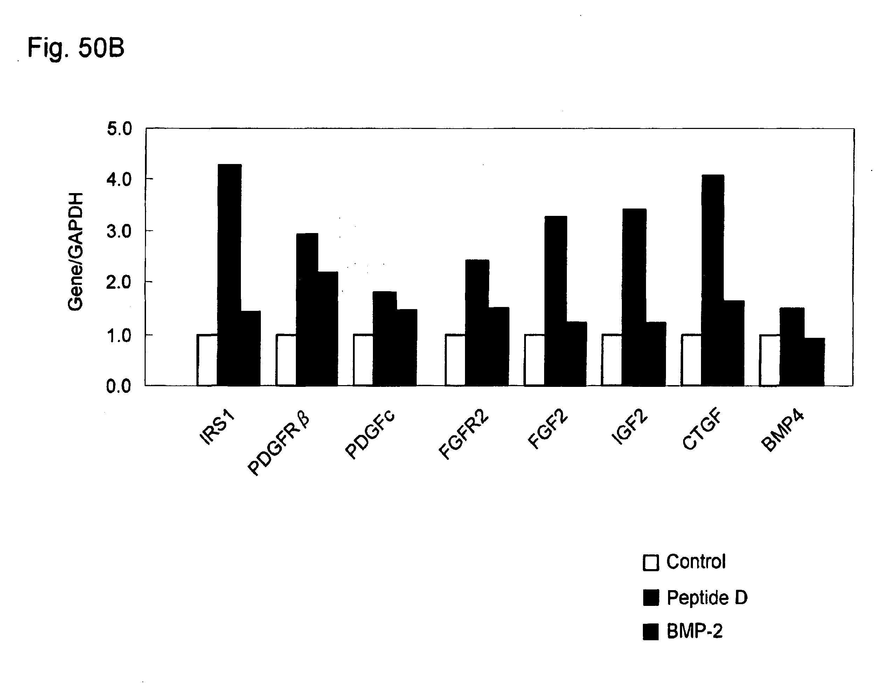

Consequently, based on the results obtained by DNA microarray analysis using MC3T3-E1 cells, it was confirmed that the addition of peptide D caused significant increases in the gene expression levels of IRS1, PDGFRβ, PDGFc, FGFR2, FGF2, CTGF, BMP-4, and IGF-2, as in the cases of OC, ALP, and Col1, (fig. 50A). The expression intensity was digitalized and a graph was created by designating each control gene expression level as 1. As a result, significant increases in the gene expression levels of IRS-1 and PDGFRβ were more obviously observed than those confirmed by DNA microarray analysis (fig. 50B). For other factors, results similar to those obtained in the case of DNA microarray analysis were obtained.

In view of the above, it is thought that peptide D acts on osteoblasts in a manner such that osteoblasts stimulated by peptide D produce, by themselves, cytokines such as PDGFRβ, PDGFc, IGF-1, IGF-2, FGF2, CTGF, and BMP-4 and a group of growth factors, and further produce a group of receptors of cytokines, such as PDGFRβ and FGFR2, and growth factors, resulting in promotion of osteoblast differentiation, proliferation, and bone formation in an autocrine manner. Physiologically, reverse signals are transmitted from RANK through RANKL to osteoblasts in contact with osteoclasts, causing a chain reaction in an autocrine/paracrine manner. This results in promotion of differentiation, proliferation, and osteogenesis not only of osteoblasts in contact with osteoclasts but also of osteoblasts existing in the proximity of osteoclasts.

### PCR primer sequences

mBMP-4-F: 5'-ATGAGGGATCTTTACCGGCT-3' (SEQ ID NO: 27)
mBMP-4-R: 5'-TTTATACGGTGGAAGCCCTG-3' (SEQ ID NO: 28)
mCTGF-F: 5'-AGTGTGCACTGCCAAAGATG-3' (SEQ ID NO: 29)
mCTGF-R: 5'-GGCCAAATGTGTCTTCCAGT-3' (SEQ ID NO: 30)
mFGF2-F: 5'-AAGCGGCTCTACTGCAAGAA-3' (SEQ ID NO: 31)
mFGF2-R: 5' -TCGTTTCAGTGCCACATACC-3' (SEQ ID NO: 32)
mFGFR2-F: 5'-CTTTGGCCTGGCCAGGGATATCAAC-3' (SEQ ID NO: 33)
mFGFR2-R: 5' -CCAACTGCTTGAATGTGGGTCTCT-3' (SEQ ID NO: 34)
mIGF2-F: 5'-CCCGCTGTTCGGTTTGCATAC-3' (SEQ ID NO: 35)
mIGF2-R: 5' -ACGGTTGGCACGGCTTGAAG-3' (SEQ ID NO: 36)
mIRS1-F: 5'-AGCGTAACTGGACATCACAGCAG-3' (SEQ ID NO: 37)
mIRS1-R: 5'-CGGTGTCACAGTGCTTTCTTGTTG-3' (SEQ ID NO: 38)
mPDGFRβ-F: 5'-GTCTGGTCTTTTGGGATCCTACTCT-3' (SEQ ID NO: 39)
mPDGFRβ-R: 5'-CTCCTCATCTACCTGCTGGTACT-3' (SEQ ID NO: 40)
mPDGFc-F: 5'-CTGATTCGGTACCTAGAGCCAGAT3' (SEQ ID NO: 41)
mPDGFc-R: 5'-CTGTCCTCTTTAGCTCTTCCCGT-3" (SEQ ID NO: 42)
mGAPDH-F: 5'-CACCATGGAGAAGGCCGGGG-3' (SEQ ID NO: 19)
mGAPDH-R: 5'-GACGGACACATTGGGGGTAG-3' (SEQ ID NO: 20)

### Example 27: Production of Fc fusion peptide

A pFUSE-hIgG1-Fc2 expression vector (Invivogen) was subjected to restriction enzyme treatment with *Eco*RV and *Bgl*II (TOYOBO). Electrophoresis was performed using 1% agarose gel (Wako). The DNA fragments are excised from the gel, followed by purification of the fragments using a Mag Extractor (TOYOBO). Meanwhile, the insertion portions were subjected to annealing at 95°C for 5 minutes and cooling to 25°C (with a temperature decrease of 1°C for a single cycle) with the use of oligonucleotides PDF1-F (SEQ ID NO: 43), PDF1-R (SEQ ID NO: 44), PAF1-F (SEQ ID NO: 45), and PAF1-R (SEQ ID NO: 46), such that two different double-strand DNAs (PDF and PAF1) were synthesized. PAF1, which is insert DNA containing peptide A comprising the amino acid sequence represented by SEQ ID NO: 47, was prepared as a negative control of PDF1, which is an insert DNA containing peptide D. The vector subjected to restriction enzyme treatment and the two different inserts were subjected to ligation at 16°C for 1 hour with the use of Ligation Mighty Mix (TAKARA). A portion (5 µL) of the resultant was transformed into DH5α (Invitrogen). Screening was carried out in an LB medium containing Zeocin (Invitrogen). The obtained colony was purified using a Mini Prep Kit (BioRad). Each plasmid was subjected to restriction enzyme treatment and sequence analysis in order to confirm the target plasmid. SEQ ID NOS: 50 and 51 represent the nucleotide sequence and the amino acid sequence of Fc fusion peptide D, which is a fusion protein of peptide D and Fc, respectively. SEQ ID NOS: 52 and 53 represent the nucleotide sequence and the amino acid sequence of Fc fusion peptide D, which is a fusion protein of peptide A and Fc, respectively.
PDF1-F: CTACTGCTGGAGCCAGTACCTGTGCTACGGTGGAGGTGGTAGCG (SEQ ID NO: 43)
PDF1-R: GATCCGCTACCACCTCCACCGTAGCACAGGTACTGGCTCCAGCAGTAG (SEQ ID NO: 44)
PAF1-F: CTACTGCGCTGCAGCTGCAGCTTGCTACGGTGGAGGTGGTAGCG (SEQ ID NO: 45)
PAF1-R: GATCCGCTACCACCTCCACCGTAGCAAGCTGCAGCTGCAGCGCAGTAG (SEQ ID NO: 46)
YCAAAAACY (SEQ ID NO: 47)

### Example 28: ALP activity enhancement capacity of Fc fusion peptide D

COS-1 cells were seeded on 10-cm dishes (2 × 10⁶ cells each). Fc fusion peptide D-expressing plasmid, Fc fusion peptide A-expressing plasmid, and the pFUSE-hIgG1-Fc2 vector that had been prepared in Example 27 (5 µg each) were subjected to transfection into COS-1 cells with the use of FuGENE HD (Roche). After 8 hours, each medium was replaced with OptiMEM (GIBCO) (10 mL), followed by culture for 72 hours. Each culture supernatant was recovered and centrifuged at 2000 rpm and 4°C for 5 minutes for removal of impurities such as dead cells. Then, Fc fusion peptide generated in each culture supernatant was concentrated using a concentration filter (Amicon). Generation of Fc fusion peptide D (SEQ ID NO: 50) and Fc fusion peptide A (SEQ ID NO: 52) in each culture supernatant and generation of Fc were confirmed by detecting bands with the relevant sizes (approximately 30 KDa) by SDS-PAGE.

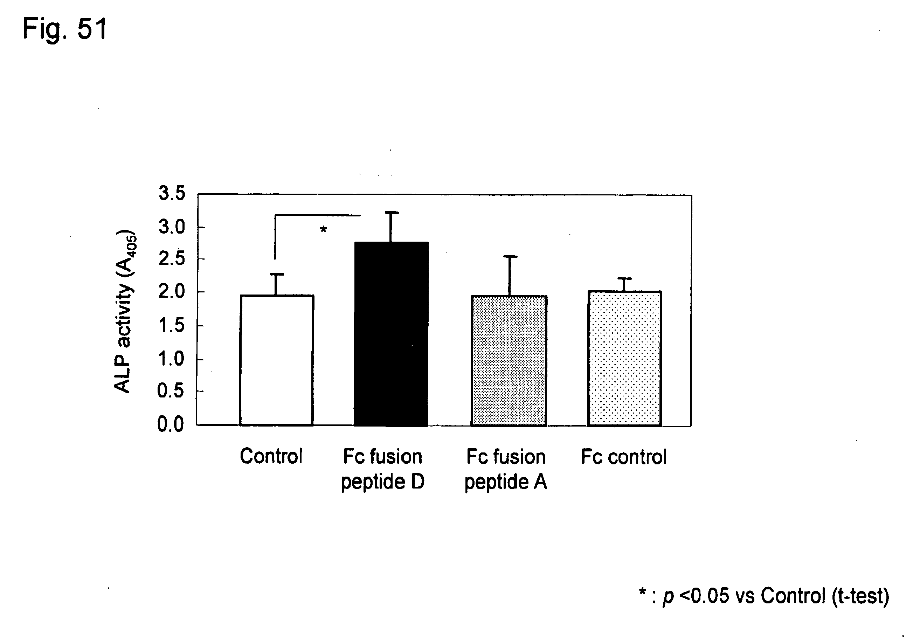

The obtained Fc fusion peptide D, Fc fusion peptide A, and Fc control were diluted with αMEM. The diluent was added to a 96-well plate (Nunc) on which MC3T3-E1 cells had been seeded (2 × 10⁴ cells/well), followed by culture. On Day 5 of culture, ALP activity determination was carried out by the method described in Example 1. As a result, it was confirmed that Fc fusion peptide D caused ALP activity enhancement to a significant degree (fig. 51). Meanwhile, such ALP activity enhancement action was not observed in the group to which Fc fusion peptide A had been added or in the group to which Fc control had been added. The above results indicate that Fc fusion peptide D obtained by fusing peptide D with Fc promoted differentiation of MC3T3-E1 cells into osteoblasts as in the case of peptide D.

### Example 29: Influence of a method of purifying synthetic peptide D on TRAP activity

The peptide D salt substitute produced in Example 18 was examined in terms of its influence on TRAP activity.

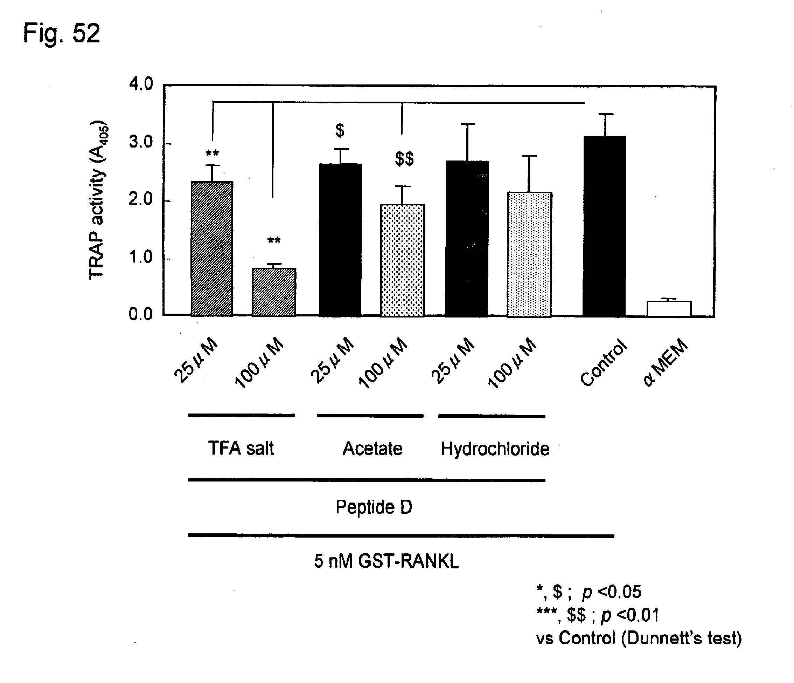

RAW264 cells mixed with 10% FBS + αMEM (SIGMA) were seeded on a 96-well plate (2 × 10³ cells/well). After cell adhesion had taken place, the medium was replaced with 10% FBS + αMEM containing 5 nM GST-RANKL (Oriental Yeast Co., Ltd.). Peptide D in the form of TFA, peptide D in the form of acetate, and peptide D in the form of hydrochloride with concentrations of 25 and 100 µM were separately added thereto, followed by culture for 4 days. After the completion of culture, acetone/ethanol (100 µL) was added to each well for cell fixation, followed by drying in a draft for 30 min. TRAP activity determination was carried out by the method described in Example 16. As a result, peptide D in the form of TFA salt with a concentration 25 µM was confirmed to have significant TRAP activity inhibition action. However, peptide D subjected to substitution with an acetate or hydrochloride was confirmed to have no such inhibition effects. In addition, peptide D in the form of TFA salt with a concentration of 100 µM had significantly high inhibition effects. Meanwhile, peptide D in the form of acetate was confirmed to have significant inhibition effects; however, such inhibition effects were weaker than those of peptide D in the form of TFA (fig. 52). In addition, peptide D in the form of hydrochloride was confirmed to have no effect on inhibition of TRAP activity, even when the concentration thereof was 100 µM. As shown in fig. 39A, it was also confirmed in terms of osteoclastogenesis inhibition activity that the use of different salts influences the activity levels even in the presence of a common amino acid sequence. Peptide D in the form of hydrochloride was confirmed to have neither osteoblast differentiation activity nor osteoclastgenesis inhibition activity. However, peptide D in the form of acetate was confirmed to have higher osteoblast differentiation activity and lower osteoclastgenesis inhibition activity compared with peptide D in the form of TFA. These results indicate that it is possible to separately control two activities of peptide D (namely, osteoblast differentiation activity and osteoclastgenesis inhibition activity) through modification such as substitution. It is also possible to produce modified peptide D having osteoblast differentiation activity alone or modified peptide D having osteoclastgenesis inhibition activity alone.

### Example 30: Examination of neutralization capacities of a variety of RANKL antibodies

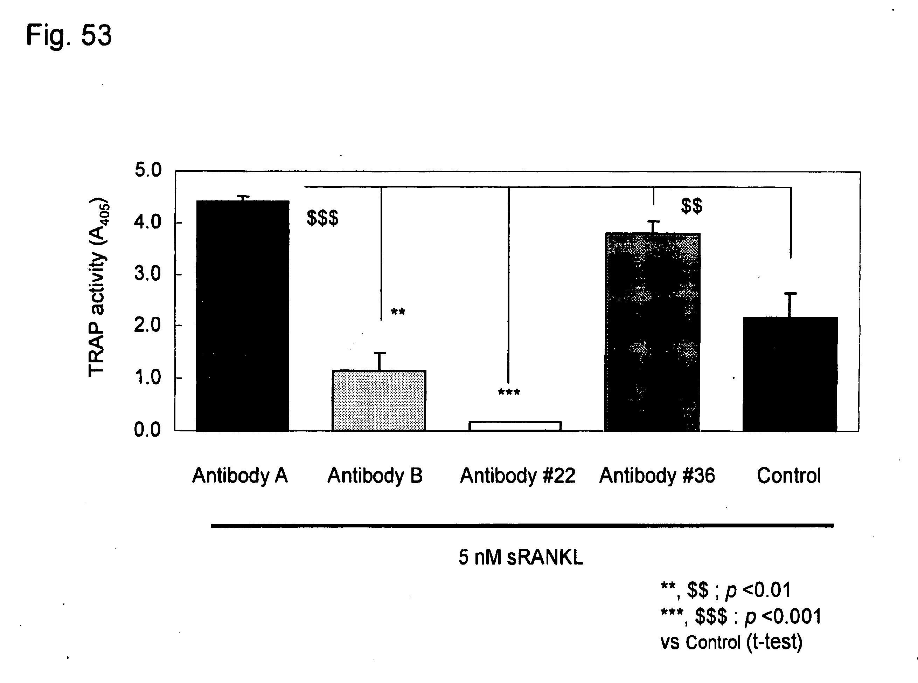

In order to examine the functions of an RANKL antibody having ALP activity enhancement capacity, a variety of RANKL antibodies were examined in terms of capacity for neutralizing RANKL osteoclastgenesis activity. Mouse monoclonal RANKL antibodies (#22, #36, A, and B) were used herein. RAW264 cells mixed with 10% FBS + αMEM (SIGMA) were seeded on a 96-well plate (2 × 10³ cells/well). After cell adhesion had taken place, the medium was replaced with 10% FBS + αMEM containing 5 nM mouse sRANKL (PeproTech EC, Ltd). A variety of RANKL antibodies (1 µg/mL) were separately added thereto, followed by culture for 4 days. After the completion of culture, acetone/ethanol (100 µL) was added to each well for cell fixation, followed by drying in a draft for 30 min. TRAP activity determination was carried out by the method described in Example 16. As a result, antibodies #22 and B were found to have TRAP activity inhibition action; while on the other hands, antibodies #36 and A were found to have no neutralization activity (fig. 53). In contrast, antibodies #36 and A significantly promoted RANKL osteoclastgenesis activity. This is probably because these antibodies caused structural changes in sRANKL when binding thereto, such that sRANKL was formed into a trimer, thereby promoting clusterization of RANK upon binding of sRANKL to RANK located on RAW264 cells, resulting in promotion of osteoclastgenesis.

### Example 31: Preparation of GST fusion peptide

As in the case of Example 27, insert DNA comprising a sequence encoding peptide D and a linker sequence and having *Eco*RI and *Bam*HI restriction enzyme sites added to both ends thereof was prepared with the use of oligonucleotides GPD1-F (SEQ ID NO: 48) and GPD1-R (SEQ ID NO: 49). The insert DNA was cloned downstream of Glutathione S-transferase of pGEX-4T-2 (GE healthcare; Genbank Accession Number U13854) with the use of the endonucleases according to a standard method. SEQ ID NO: 54 and 55 represent the nucleotide sequence and the amino acid sequence of GST fusion peptide D, which is a fusion protein of peptide D and GST, respectively. DH5α (Invitrogen) was used for transformation. The obtained positive clone was cultured by a standard method, followed by induction of protein expression with the use of IPTG (final concentration: 0.5 mM). Then, cells were suspended in an extraction buffer (50 mM Tris-HCl, pH 8.0, 100 mM NaCl, 1 mM EDTA, 1 mM DTT, and 1%(v/v) TritonX-100), followed by homogenization on ice with the use of a sonicator. The resultant was centrifuged at 18000 × g for 15 min. The supernatant was recovered and used to fill a Glutathione Sepharose column. Subsequently, the column was washed with a washing buffer (50 mM Tris-HCl, pH 8.0, 100 mM NaCl, 1 mM DTT, and 0.1%(v/v) TritonX-100), followed by elution with a glutathione solution (12 mM reduced glutathione, 50 mM Tris-HCl, and pH 8.0). After elution, dialysis was carried out with a phosphate buffer (PBS). The purified GST fusion peptide D was subjected to SDS-PAGE for confirmation of its molecular weight. The molecular weight was approximately 27 kDa. The GST fusion peptide D was sterilized by filtration using a 0.22-µm filter (Pall Corporation) and then used for the experiment described below.

### Example 32: Preparation of anti-human RANKL monoclonal antibodies

Mice were immunized with GST-RANKL (Oriental Yeast Co., Ltd.) containing an extracellular domain (aa140-317) of human RANKL such that hybridomas were prepared by a standard method. The prepared hybridomas were cultured in a DMEM cell culture medium (containing 4.5 g/L glucose and L-glutamine) + 10% FBS, followed by cloning with limiting dilution. Then, 6 different hybridomas were selected and the culture supernatant of each thereof was recovered. Each recovered culture supernatant was filtered via a 0.22-µm filter (Pall Corporation) and used to fill a protein G sepharose column (GE healthcare). Subsequently, the column was washed with PBS: Antibodies were eluted with an elution buffer (0.1 M glycine-HCl, pH 2.7). In addition, the eluted antibodies were immediately neutralized with a neutralization buffer (1M Tris-HCl, pH 9.0), followed by dialysis with PBS and then filtration sterilization with the use of a 0.22-µm filter. After confirmation of the bands for the L and H chains of the antibodies by SDS-PAGE, concentration was calculated with a spectrophotometer based on the absorbance at A280.

### Example 33: Examination of the neutralization capacity of an anti-human RANKL monoclonal antibody

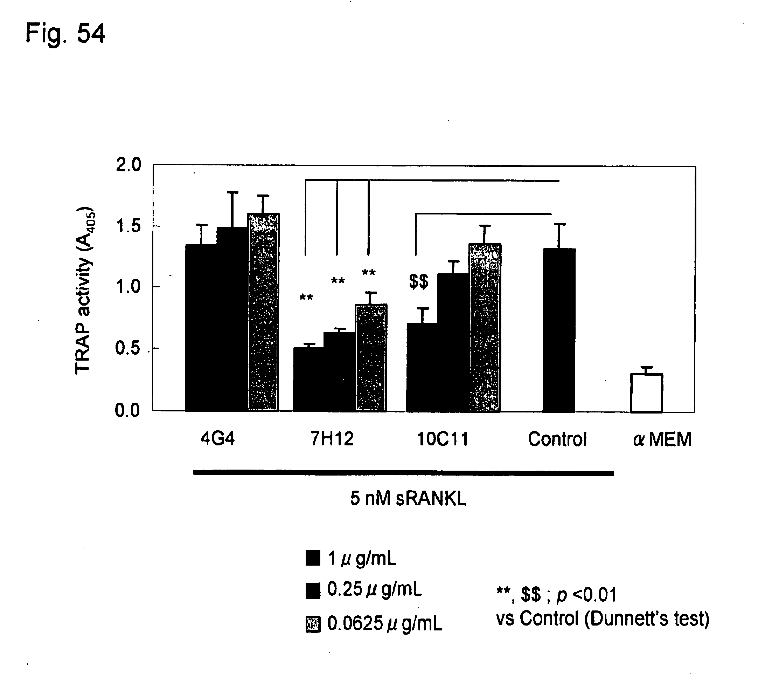

In order to review the casual relationship between the ALP activity enhancement capacity and the neutralization capacity of an RANKL antibody, the anti-human RANKL monoclonal antibodies prepared in Example 32 were examined in terms of the influence of the neutralization activity on the osteoclastgenesis capacity of RANKL. Clones 4G4, 7H12, and 10C11 were used. RAW264 cells mixed with 10% FBS + αMEM (SIGMA) were seeded on a 96-well plate (2×10³ cells/well). After cell adhesion had taken place, the medium was replaced with 10% FBS + αMEM containing 5 nM human sRANKL (PeproTech EC, Ltd). 0.0625, 0.25, and 1 µg/mL anti-human RANKL monoclonal antibodies were separately added thereto, followed by culture for 4 days. After the completion of culture, acetone/ethanol (100 µL) was added to each well for cell fixation, followed by drying in a draft for 30 min. TRAP activity determination was carried out by the method described in Example 16. As a result, 10C11 was found to have significant TRAP activity inhibition action when used at a concentration of 1 µg/mL. However, 10C11 did not exhibit such inhibition action when used at concentrations of 0.25 and 0.0625 µg/mL (fig. 54). Meanwhile, 7H12 was found to have strong neutralization capacity at each concentration; on the other hand, 4G4 was confirmed to have no neutralization action.

### Example 34: Effects of GST fusion peptide D and anti-human RANKL monoclonal antibodies upon differentiation of human mesenchymal stem cells

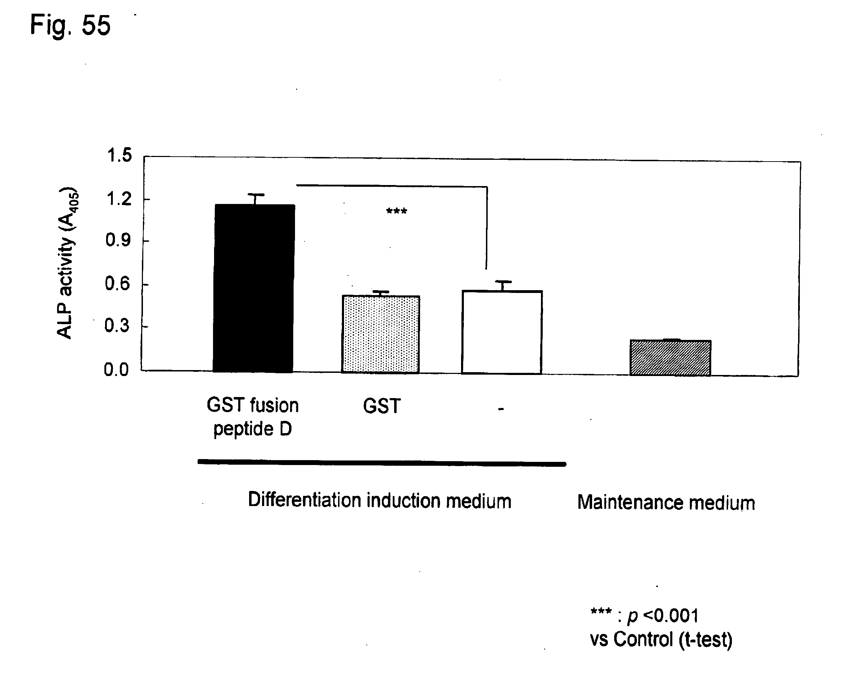

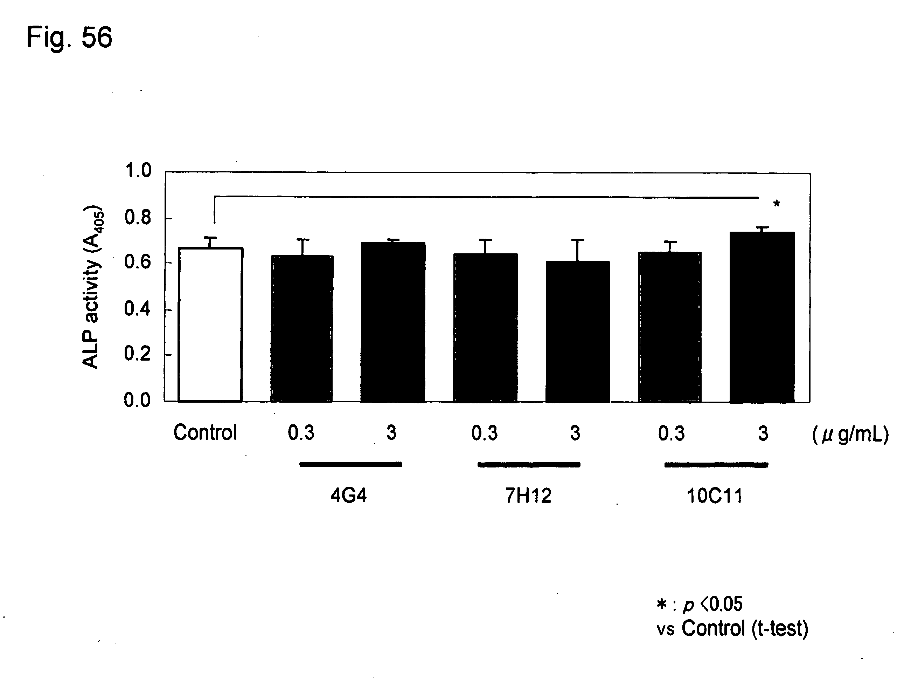

Human mesenchymal stem cells (hMSC; Lonza) were seeded on a 96-well plate (Nunc) (2 × 10³ cells/well). After cell adhesion had taken place, the medium was replaced with a differentiation medium prepared by adding 100 nM dexamethasone (SIGMA), 10 mM BGP(SIGMA), and 50 µg/mL ascorbic acid to a dedicated maintenance medium (Lonza). 10 nM GST fusion peptide D and GST or 3 types of anti-human RANKL monoclonal antibodies at concentrations of 0.3 and 3 µg/mL were separately added thereto, followed by culture. On Day 5 of culture, ALP activity determination was carried out by the method described in Example 1. As a result, it was confirmed that GST fusion peptide D had caused a significant increase in ALP activity in the case of hMSC (fig. 55). The addition of GST alone had caused no changes in ALP activity. Meanwhile, when 3 types of the anti-human RANKL monoclonal antibodies used in Example 33 were added to hMSC, the ALP activity was significantly enhanced by the 10C11 antibody (fig. 56). Meanwhile, 4G4 and 7H12 were confirmed to have no ALP activity enhancement action.

Based on the above, it was found that GST fusion peptide D obtained by fusing peptide D with GST promoted differentiation of human mesenchymal stem cells into osteoblasts, as in the case of peptide D. As a result of taking the above into account with the promotion of osteoblast differentiation caused by Fc fusion peptide D in Example 28, it has been revealed that a peptide D fused with a certain protein can exhibit actions comparable to those of peptide D. In addition, the results of the above experiments revealed that some anti-human RANKL monoclonal antibodies have action of promoting differentiation of human mesenchymal stem cells into osteoblasts. That is to say, it has been shown that an antibody capable of recognizing a specific portion of RANKL as an epitope acts on RANKL so as to transmit osteoblast differentiation signals. Based on this, it becomes possible to design, screen for, and produce an anti-RANKL monoclonal antibody that can transmit osteoblast differentiation signals in an effective manner. As in the case of the anti-mouse RANKL monoclonal antibody B described in Example 21, there is an anti-RANKL monoclonal antibody that acts on RANKL so as to transduce osteoblast proliferation signals. It has been also shown that an antibody capable of recognizing a specific portion of RANKL as an epitope acts on RANKL so as to transduce osteoblast differentiation signals. As described above, it is possible to find an antibody that can efficiently transduce osteoblast proliferation or differentiation signals by screening many anti-RANKL monoclonal antibodies so as to find an optimized antibody.

In addition, as shown in Examples 19 and 30, it has been shown that anti-mouse RANKL monoclonal antibodies (antibodies #22 and B) that neutralize the osteoclast differentiation by RANKL include one having a proliferative effect on osteoblasts (B) and one having no such effect (#22), and that anti-mouse RANKL monoclonal antibodies (#22, #36, A, and B) that stimulate osteoblast differentiation include neutralizing antibodies and non-neutralizing antibodies. Further, it has been shown that anti-human RANKL monoclonal antibodies (7H12 and 10C11) that neutralize the osteoclast differentiation by RANKL include one stimulating osteoblast differentiation (10C11) and one not having such effect (7H12). Meanwhile, there is another anti-human RANKL monoclonal antibody having none of the above effects (4G4). These facts indicate that it is possible to allow different anti-RANKL monoclonal antibodies to separately exhibit the three above effects (namely, neutralization of osteoclast differentiation, stimulation of osteoblast proliferation, and stimulation of osteoblast differentiation) that can be exhibited by anti-RANKL monoclonal antibodies. In addition, as in the case of antibody B, it is also possible to allow a single antibody to have the three above actions (osteoclast differentiation neutralization, osteoblast proliferation, and osteoblast differentiation). Further, as in the cases of antibodies #22 and 10C11, it is also possible to allow a single antibody to have any two of the above three actions.

### Industrial Applicability

According to the present invention, it has been found that transmission of reverse signals from RANK, which is an RANKL receptor, to RANKL, which is an RANK ligand, in osteoblasts or cells capable of differentiating into osteoblasts takes place, in addition to transmission of forward signals from RANKL to RANK. Also, the present inventors have found that the bidirectional signal transduction between RANKL and RANK controls coupling of bone resorption and osteogenesis. It is thought that transduction of reverse signals from membrane-bound RANK located on osteoclasts to membrane-bound RANKL located on osteoblasts controls the coupling of bone resorption and osteogenesis in physiological bone metabolism. The use of such reverse signals allows the development of an agent that can increase bone mass. Specifically, enhancement of osteoblast differentiation and maturation is caused by reverse signals that are transmitted when molecules capable of acting on RANKL such as membrane-bound RANK, an RANK analog peptide, an anti-RANKL antibody, soluble RANK, OPG, and variants and analogs thereof act on membrane-bound RANKL, resulting in an increase in bone mass.

Membrane-bound RANK, an RANK analog peptide, an anti-RANKL antibody, soluble RANK, OPG, and variants and analogs thereof, as well as a compound that promotes differentiation, proliferation, maturation, or calcification of osteoblasts or cells capable of differentiating into osteoblasts that is a natural or synthetic low-molecular compound such as a molecule capable of acting on RANKL, can be used for enhancement of osteoblast differentiation and maturation, leading to an increase in bone mass. Specifically, such compound can be used for a pharmaceutical product, an in *vitro* diagnostic agent, and the like. In addition, a novel osteogenesis promoter can be searched for or developed by screening for a compound that promotes differentiation, proliferation, maturation, or calcification of osteoblasts or cells capable of differentiating into osteoblasts, such as a molecule capable of acting on RANKL. Further, a compound that promotes differentiation, proliferation, maturation, or calcification of osteoblasts or cells capable of differentiating into osteoblasts, such as a molecule capable of acting on RANKL, can be used as a reagent that transmits signals to osteoblasts or cells capable of differentiating into osteoblasts so as to cause differentiation and maturation of osteoblasts or such cells. There are many known cells capable of expressing RANKL such as T cells, B cells, and synoviocytes, in addition to osteoblasts or cells capable of differentiating into osteoblasts. However, a compound that promotes differentiation, proliferation, maturation, or calcification of osteoblasts or cells capable of differentiating into osteoblasts, such as a molecule capable of acting on RANKL, can be used as a substance that transduces signals to the above cells in a similar manner so as to cause differentiation, maturation, and/or activation of such cells. Such compound can be used as a pharmaceutical product, an *in vitro* diagnostic agent, a research reagent, and the like for various applications.

### Free Text of Sequence Listings

SEQ ID NOS: 7 and 16: Synthetic peptides in which Cys at position 2 is bound to Cys at position 8 via a disulfide bond SEQ ID NOS: 8 to 13, 17 to 46, 48, and 49: Primers

## Claims

1. A compound for use in the treatment or prevention of bone metabolic diseases associated with osteopenia by inducing osteogenesis, which is either:
(i) a compound that acts on osteoblasts or cells capable of differentiating into osteoblasts and promotes differentiation, proliferation, maturation, or calcification of osteoblasts or cells capable of differentiating into osteoblasts, wherein the compound is selected from the group consisting of:
RANK,
a variant or fragment peptide of RANK,
OPG,
a variant or fragment peptide of OPG,
an anti-RANKL antibody or a functional fragment thereof; or
(ii) a peptide comprising the amino acid sequence represented by SEQ ID NO: 7 or SEQ ID NO: 16.

2. The compound for use in the treatment or prevention of bone metabolic diseases according to claim 1, wherein the compound of part (i) acts on RANKL located on osteoblasts or cells capable of differentiating into osteoblasts.

3. The compound for use in the treatment or prevention of bone metabolic diseases according to claim 1 or 2, wherein the compound of part (i) is a peptide consisting of the amino acid sequence represented by SEQ ID NO: 7 or SEQ ID NO: 16.

4. The compound for use in the treatment or prevention of bone metabolic diseases according to claim 1 or 2, wherein the compound of part (i) is a fusion protein of a peptide comprising the amino acid sequence represented by SEQ ID NO: 7 or SEQ ID NO: 16 and GST or the Fc region of IgG₁.

5. The compound for use in the treatment or prevention of bone metabolic diseases according to any one of claims 1 to 4, wherein the cells capable of differentiating into osteoblasts are selected from the group consisting of osteoblast precursor cells, mesenchymal stem cells, stromal cells, and myoblasts.

6. The compound for use in the treatment or prevention of bone metabolic diseases according to claim 1, part (ii) which is a peptide in the form of acetate comprising the amino acid sequence represented by SEQ ID NO: 7 or SEQ ID NO: 16.

7. The compound for use in the treatment or prevention of bone metabolic diseases according to any one of claims 1 to 6, wherein the bone metabolic diseases associated with osteopenia are selected from the group consisting of osteoporosis, juvenile osteoporosis, dysosteogenesis, hypercalcemia, hyperparathyroidism, osteomalacia, osteohalisteresis, osteolytic bone diseases, osteonecrosis, the Paget's disease, rheumatoid arthritis, bone mass reduction due to osteoarthritis, inflammatory arthritis, osteomyelitis, glucocorticoid treatment, metastatic bone diseases, periodontal bone loss, bone loss due to cancer, and bone loss due to aging.

8. The compound for use in the treatment or prevention of bone metabolic diseases according to any one of claims 1 to 7, which is for use in combination with a BMP family member.

## Patentansprüche

1. Verbindung zur Verwendung bei der Behandlung oder Vorbeugung von mit Osteopenie assoziierten Knochenstoffwechselerkrankungen durch Induktion von Osteogenese, die entweder
(i) eine Verbindung ist, die auf Osteoblasten oder Zellen, die in der Lage sind, zu Osteoblasten zu differenzieren, wirkt und die Differenzierung, Proliferation, Reifung oder Calcifizierung von Osteoblasten oder Zellen, die in der Lage sind, zu Osteoblasten zu differenzieren, fördert, wobei die Verbindung aus folgender Gruppe ausgewählt ist:
RANK,
einer Peptidvariante oder einem Peptidfragment von RANK, OPG,
einer Peptidvariante oder einem Peptidfragment von OPG,
einem Anti-RANKL-Antikörper oder einem funktionellen Fragment davon; oder
(ii) ein Peptid ist, das die durch Seq.-ID Nr. 7 oder Seq.-ID Nr. 16 dargestellte Aminosäuresequenz umfasst.

2. Verbindung zur Verwendung bei der Behandlung oder Vorbeugung von Knochenstoffwechselerkrankungen nach Anspruch 1, wobei die Verbindung aus Teil (i) auf RANKL wirkt, das sich auf Osteoblasten oder Zellen, die in der Lage sind, zu Osteoblasten zu differenzieren, befindet.

3. Verbindung zur Verwendung bei der Behandlung oder Vorbeugung von Knochenstoffwechselerkrankungen nach Anspruch 1 oder 2, wobei die Verbindung aus Teil (i) ein Peptid ist, das aus der durch Seq.-ID Nr. 7 oder Seq.-ID Nr. 16 dargestellten Aminosäuresequenz besteht.

4. Verbindung zur Verwendung bei der Behandlung oder Vorbeugung von Knochenstoffwechselerkrankungen nach Anspruch 1 oder 2, wobei die Verbindung aus Teil (i) ein Fusionsprotein aus einem Peptid, das die durch Seq.-ID Nr. 7 oder Seq.-ID Nr. 16 dargestellte Aminosäuresequenz umfasst, und GST oder der Fc-Region von IgG₁ ist.

5. Verbindung zur Verwendung bei der Behandlung oder Vorbeugung von Knochenstoffwechselerkrankungen nach einem der Ansprüche 1 bis 4, wobei die Zellen, die in der Lage sind, zu Osteoblasten zu differenzieren, aus der aus Osteoblastenvorläuferzellen, mesenchymalen Stammzellen, Stromazellen und Myoblasten bestehenden Gruppe ausgewählt sind.

6. Verbindung zur Verwendung bei der Behandlung oder Vorbeugung von Knochenstoffwechselerkrankungen nach Anspruch 1, Teil (ii), die ein Peptid in Form eines Acetats ist, das die durch Seq.-ID Nr. 7 oder Seq.-ID Nr. 16 dargestellte Aminosäuresequenz umfasst.

7. Verbindung zur Verwendung bei der Behandlung oder Vorbeugung von Knochenstoffwechselerkrankungen nach einem der Ansprüche 1 bis 6, wobei die mit Osteopenie assoziierten Knochenstoffwechselerkrankungen aus der aus Osteoporose, juveniler Osteoporose, Dysosteogenese, Hyperkalzämie, Hyperparathyroidismus, Osteomalazie, Osteohalisterese, osteolytischen Knochenerkrankungen, Osteonekrose, Paget-Syndrom, rheumatoider Arthritis, Knochenmassenverringerung aufgrund von Osteoarthritis, entzündlicher Arthritis, Osteomyelitis, Glucocorticoidbehandlung, metastatischen Knochenerkrankungen, parodontalem Knochenschwund, Knochenschwund aufgrund von Krebs und Knochenschwund aufgrund von Alterung bestehenden Gruppe ausgewählt sind.

8. Verbindung zur Verwendung bei der Behandlung oder Vorbeugung von Knochenstoffwechselerkrankungen nach einem der Ansprüche 1 bis 7, die zur Verwendung in Kombination mit einem Mitglied der BMP-Familie bestimmt ist.

## Revendications

1. Composé destiné à être utilisé dans le traitement ou la prévention de maladies métaboliques osseuses associées à une ostéopénie par l'induction d'une ostéogenèse, qui est soit :
(i) un composé qui agit sur les ostéoblastes ou des cellules capables de se différencier en ostéoblastes et favorise la différenciation, la prolifération, la maturation ou la calcification des ostéoblastes ou des cellules capables de se différencier en ostéoblastes, où le composé est sélectionné dans le groupe consistant en :
RANK,
un variant ou un fragment peptidique de RANK, l'OPG,
un variant ou un fragment peptidique de l'OPG,
un anticorps anti-RANKL ou un fragment fonctionnel de celui-ci ; ou
(ii) un peptide comprenant la séquence d'acides aminés représentée par SEQ ID NO: 7 ou SEQ ID NO: 16.

2. Composé destiné à être utilisé dans le traitement ou la prévention de maladies métaboliques osseuses selon la revendication 1, où le composé de la partie (i) agit sur RANKL localisé sur les ostéoblastes ou des cellules capables de se différencier en ostéoblastes.

3. Composé destiné à être utilisé dans le traitement ou la prévention de maladies métaboliques osseuses selon la revendication 1 ou 2, où le composé de la partie (i) est un peptide consistant en la séquence d'acides aminés représentée par SEQ ID NO: 7 ou SEQ ID NO: 16.

4. Composé destiné à être utilisé dans le traitement ou la prévention de maladies métaboliques osseuses selon la revendication 1 ou 2, où le composé de la partie (i) est une protéine de fusion entre un peptide comprenant la séquence d'acides aminés représentée par SEQ ID NO: 7 ou SEQ ID NO: 16 et la GST ou la région Fc d'une IgG₁.

5. Composé destiné à être utilisé dans le traitement ou la prévention de maladies métaboliques osseuses selon l'une quelconque des revendications 1 à 4, où les cellules capables de se différencier en ostéoblastes sont sélectionnées dans le groupe consistant en des cellules précurseurs des ostéoblastes, des cellules souches mésenchymateuses, des cellules stromales, et des myoblastes.

6. Composé destiné à être utilisé dans le traitement ou la prévention de maladies métaboliques osseuses selon la revendication 1, partie (ii), qui est un peptide sous la forme d'acétate comprenant la séquence d'acides aminés représentée par SEQ ID NO: 7 ou SEQ ID NO: 16.

7. Composé destiné à être utilisé dans le traitement ou la prévention de maladies métaboliques osseuses selon l'une quelconque des revendications 1 à 6, où les maladies métaboliques osseuses associées à une ostéopénie sont sélectionnées dans le groupe consistant en l'ostéoporose, l'ostéoporose juvénile, la dysostéogenèse, l'hypercalcémie, l'hyperparathyroïdie, l'ostéomalacie, l'halistérèse, des maladies osseuses ostéolytiques, l'ostéonécrose, la maladie de Paget, la polyarthrite rhumatoïde, une réduction de la masse osseuse due à l'arthrose, l'arthrite inflammatoire, l'ostéomyélite, un traitement par des glucocorticoïdes, des maladies osseuses métastatiques, une perte osseuse parodontale, une perte osseuse due à un cancer, et une perte osseuse due au vieillissement.

8. Composé destiné à être utilisé dans le traitement ou la prévention de maladies métaboliques osseuses selon l'une quelconque des revendications 1 à 7, qui est destiné à être utilisé en combinaison avec un membre de la famille des BMP.
